# EUROPEAN PATENT APPLICATION

(11) **EP 4 438 049 A1**
(43) Date of publication of application: **02.10.2024**
(21) Application number: 22897964.7
(22) Date of filing: 28.11.2022
(51) Int. Cl.: A61K 35/66, A61K 35/38, A61K 35/741, A61P 37/00

(54) **GENETICALLY MODIFIED MICROORGANISM AND USE THEREOF**

(30) Priority: 26.11.2021 CN 202111420763
(71) Applicant: Commbio Therapeutics Co., Ltd., Shanghai 201203 (CN)
(72) Inventor: GUO, Yuqi, Shanghai 201203 (CN); YANG, Guoxue, Shanghai 201203 (CN); XIANG, Bin, Shanghai 201203 (CN)
(74) Representative: Pestalozzi, Deborah
(86) International application number: PCT/CN2022/134672
(87) International publication number: WO 2023/093883

(57) **Abstract**

Disclosed are a genetically modified microorganism and the use thereof. The genetically modified microorganism expresses at least one, two or three exogenous genes (AMUC_1100 polypeptide, IL-10 and/or IL-22). The genetically modified microorganism can still stably survive after integrating exogenous target genes therein; and efficiently expresses and secretes proteins or polypeptides encoded by the exogenous genes in the intestinal tract, and the expressed and secreted proteins or polypeptide encoded by the exogenous genes can still have good activity. Therefore, the genetically modified microorganism is used for treating inflammatory diseases or autoimmune diseases (such as enteritis and arthritis).

## Description

This application claims the priority of Chinese Patent Application No. 202111420763.8, filed with the China National Intellectual Property Administration on November 26, 2021, which is hereby incorporated by reference in its entirety.

### FIELD

The present invention belongs to the field of biomedicine, and particularly relates to a genetically modified microorganism and use thereof.

### BACKGROUND

Inflammation is body's normal response to remove cause of disease or damaged cells from the body to help tissue repair. Immune system disorders often lead to abnormal inflammation, and inflammatory diseases involving the immune system often underlie a large class of human diseases.

Autoimmune diseases are abnormal immune response to normal body parts. At least 80 autoimmune diseases affect 24 million people in the United States alone. Current treatments include nonsteroidal anti-inflammatory drugs (NSAIDs) and immunosuppressants. However, these treatments only relieve symptoms but do not provide a complete cure. Moreover, these treatments often cause serious side effects.

Autoimmune diseases are complex diseases caused by multiple pathogenic signaling pathways. Currently, approved drugs targeting a single signaling pathway have shown limited therapeutic effects, suggesting that modulating multiple signaling pathways should lead to better therapeutic effects. However, research on the regulation of multiple signaling pathways is limited. Firstly, it is not known the modulation of which of the multiple signaling pathways is effective. Secondly, it is not known what pharmacological methodology may more effectively modulate multiple signaling pathways. Due to the lack of effective treatments, industry has made significant efforts to discover and develop new methods to treat autoimmune diseases, but has not made significant progress.

Therefore, there is an urgent need in the art to develop a more efficient method for treating inflammatory or autoimmune diseases.

### SUMMARY

In one aspect, the present disclosure provides a genetically modified microorganism comprising at least two exogenous genes encoding a polypeptide selected from the group consisting of: a) Amuc_1100 polypeptide; b) IL-10 polypeptide; and c) IL-22 polypeptide.

In some embodiments, the genetically modified microorganism comprises a) one exogenous gene encoding Amuc_1100 polypeptide and one exogenous gene encoding IL-10 polypeptide; b) one exogenous gene encoding IL-10 polypeptide and one exogenous gene encoding IL-22 polypeptide; or c) one exogenous gene encoding Amuc_1100 polypeptide and one exogenous gene encoding IL-22 polypeptide. In some embodiments, the genetically modified microorganism comprises exogenous genes encoding Amuc_1100 polypeptide, IL-10 polypeptide and IL-22 polypeptide respectively.

In another aspect, the present disclosure provides a combination of genetically modified microorganisms comprising at least two different genetically modified microorganisms, wherein each genetically modified microorganism expresses at least one of the different exogenous genes; wherein the exogenous gene respectively encodes a polypeptide selected from the group consisting of: a) Amuc_1100 polypeptide; b) IL-10 polypeptide; and c) IL-22 polypeptide.

In some embodiments, the polypeptides encoded by the exogenous genes can be expressed by the microorganism and/or can be secreted outside of the cell/microorganism after expression.

In some embodiments, the microorganism is capable of expressing and/or secreting the polypeptides encoded by the exogenous genes in the intestine of a human or animal.

In some embodiments, the secreting is achieved via a native or non-native secretion system of the microorganism.

In some embodiments, the Amuc_1100 polypeptide, the IL-10 polypeptide and/or the IL-22 polypeptide is a polypeptide without its own signal peptide.

In some embodiments, the Amuc_1100 polypeptide, the IL-10 polypeptide and/or the IL-22 polypeptide without its own signal peptide is connected to a signal peptide. For example, the Amuc_1100 polypeptide, the IL-10 polypeptide and/or the IL-22 polypeptide are respectively connected to a first signal peptide, a second signal peptide or a third signal peptide, and the first signal peptide, the second signal peptide and the third signal peptide are capable of secreting the polypeptide outside of the microorganism.

In another aspect, the present disclosure provides a polynucleotide comprising at least one recombinant expression cassette, comprising i) at least one or at least two exogenous genes encoding Amuc_1100 polypeptide, IL-10 polypeptide and/or IL-22 polypeptide respectively, and ii) one or more regulatory elements operably linked to the at least one or at least two exogenous genes.

In some embodiments, the polynucleotide comprises exogenous genes encoding the following polypeptides respectively: Amuc_1100 polypeptide and IL-10 polypeptide; IL-10 polypeptide and IL-22 polypeptide, or Amuc_1100 polypeptide and IL-22 polypeptide.

In some embodiments, the polynucleotide comprises exogenous genes encoding Amuc_1100 polypeptide, IL-10 polypeptide and IL-22 polypeptide respectively.

In another aspect, the present disclosure provides a composition, comprising: (a) the genetically modified microorganism of the present disclosure as an active ingredient; and (b) a physiologically or pharmacologically acceptable carrier.

In some embodiments, the composition is a pharmaceutical composition.

In some embodiments, the composition is an oral preparation.

In another aspect, the present disclosure provides use of the genetically modified microorganism or the composition of the present disclosure in the manufacture of a medicament for treating or preventing an inflammatory disease or autoimmune disease.

In some embodiments, the autoimmune disease is selected from the group consisting of inflammatory bowel disease, graft versus host disease (GvHD), systemic lupus erythematosus (SLE), arthritis, asthma, and a combination thereof.

In another aspect, the present disclosure provides use of the genetically modified microorganism or the composition of the present disclosure in the manufacture of a medicament for improving the therapeutic effect of a drug for treating an inflammatory disease or autoimmune disease.

In another aspect, the present disclosure provides a method for preparing the genetically modified microorganism of the present disclosure, comprising the steps of: introducing the polynucleotide of the present disclosure into a microorganism so that the exogenous gene in the polynucleotide is expressed in the microorganism.

In another aspect, the present disclosure provides a method for treating or preventing an inflammatory disease or an autoimmune disease in a subject in need thereof, comprising: administering to the subject an effective amount of the genetically modified microorganism or the composition of the present disclosure.

In another aspect, the present disclosure provides a method for improving the efficacy of a drug in a subject, comprising: administering to the subject an effective amount of the genetically modified microorganism or the composition of the present disclosure.

In another aspect, the present disclosure provides use of a genetically modified microorganism in the manufacture of a medicament for treating an inflammatory disease or an autoimmune disease, wherein the inflammatory disease or autoimmune disease is selected from the group consisting of: inflammatory bowel disease (IBD), graft-versus-host disease (GvHD), systemic lupus erythematosus (SLE), arthritis and asthma; wherein the genetically modified microorganism comprises at least one, at least two or at least three exogenous genes encoding polypeptides selected from the group consisting of: a) Amuc_1100 polypeptide; b) IL-10 polypeptide; and c) IL-22 polypeptide.

### BRIEF DESCRIPTION OF DRAWINGS

FIG. 1 shows a schematic diagram of the insertion of an expression cassette containing an exogenous gene into the EcN genome.
FIG. 2 shows the comparison results of the production of IL-10 expressed by strain CBT4078 under aerobic and anaerobic conditions.

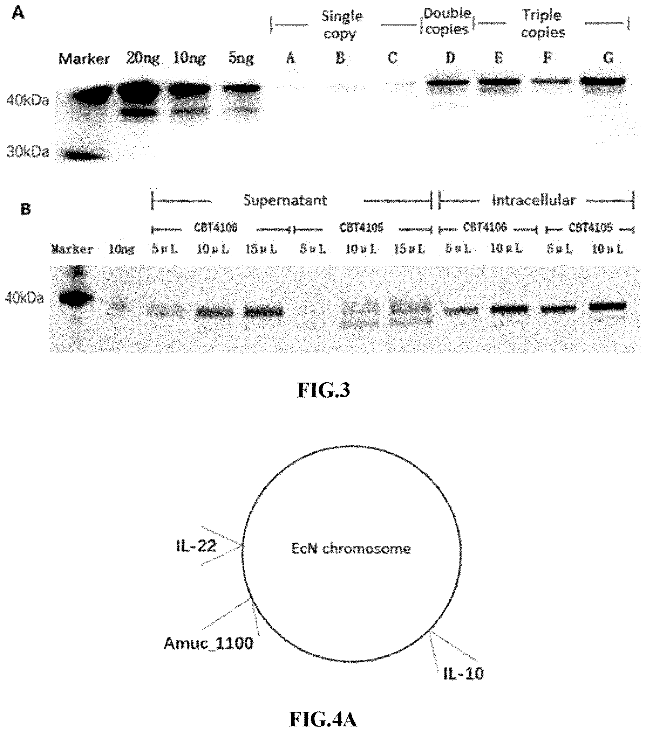

FIG. 3 shows the comparison results of the expression levels of Amuc_1100 (Y259A) with different number of copies (A), and the comparison results of improving the stability of Amuc_1100 by modifying the outer cell membrane (B); wherein, columns A and B in FIG. 3A represent strain CBT4101 with only one copy of Amuc_1100 (Y259A) under the control of promoter BBA_J23101, column C represents strain CBT4107 with only one copy of Amuc_1100 (Y259A) under the control of promoter BBA_J23110, column D represents strain CBT4102 with two copies of Amuc_1100 (Y259A) under the control of promoter BBA_J23110, column E represents strain CBT4103 with three copies of Amuc_1100 (Y259A) under the control of promoter BBA_J23101, column F represents strain CBT4108 with one copy of Amuc_1100 (Y259A) under the control of promoter BBA_J23101 and two copies of Amuc_1100 (Y259A) under the control of promoter BBA_J23110, and column G represents strain CBT4109 with three copies of Amuc_1100(Y259A) under the control of promoter BBA_J23110.
FIG. 4A shows the chromosomal profile of EcN expressing a combination of IL-10, IL-22, and Amuc_1100;
FIG. 4B shows the growth status of strains expressing a single gene, a combination of double genes, and a combination of three genes;
FIG. 4C shows the biological activity of the secreted IL-10;
FIG. 4D shows the biological activity of the secreted IL-22;
FIG. 4E shows the biological activity of the secreted Amuc_1100;
   wherein, "standard (40 µg/mL)" in the FIG. 4 represents the Amuc_1100 standard product with a concentration of 40 µg/mL, "EcN" represents the Amuc_1100 secreted by strain EcN, and "CBT4080 (28.8 µg/mL)" represents the Amuc_1100 secreted by strain CBT4080, at a concentration of 28.8 µg/mL.
FIG. 5 shows the expression amount of IL-10 in the culture supernatant after adding different concentrations of sodium salicylate and continuing to culture for 1-4 hours.
FIG. 6A shows the comparison results of the colon length at the pharmacodynamic endpoint using different strains in the mouse inflammatory bowel disease model induced by T cell transplantation;
FIG. 6B shows the HE staining results of pathological sections of colon tissues treated with different strains in the mouse inflammatory bowel disease model induced by T cell transplantation.
FIG. 7A shows the changes in body weight of DSS-induced mouse inflammatory bowel disease model;
FIG. 7B shows the DAI score of DSS-induced mouse inflammatory bowel disease model;
FIG. 7C shows the colon length at the pharmacodynamic endpoint of DSS-induced mouse inflammatory bowel disease model.
FIG. 8 shows the survival rate of mice in each experimental group of the GvHD animal model.
FIG. 9A shows the renal tubular damage scores of mice in each experimental group of the SLE animal model;
FIG. 9B shows the concentration of anti-double stranded DNA antibody IgG in the serum of mice in each experimental group of the SLE animal model;
FIG. 9C shows the albumin concentration in the urine of mice in each experimental group of the SLE animal model;
FIG. 9D shows the glomerular damage detected by PAS staining on pathological sections of renal tissue of mice in each experimental group of the SLE animal model.
FIG. 10A shows the footpad thickness of mice in each experimental group of the CIA animal model.
FIG. 10B shows the disease scores of mice in each experimental group in the CIA animal model.

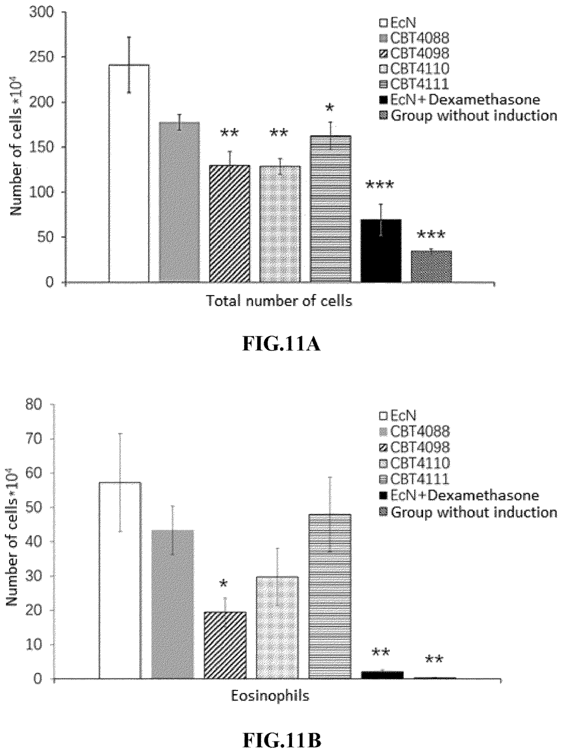

FIG. 11A shows the total cell counting results in the bronchoalveolar lavage fluid (BALF) of mice in each experimental group of the asthma animal model;
FIG. 11B shows the eosinophil counting results in the bronchoalveolar lavage fluid (BALF) of mice in each experimental group of the asthma animal model;

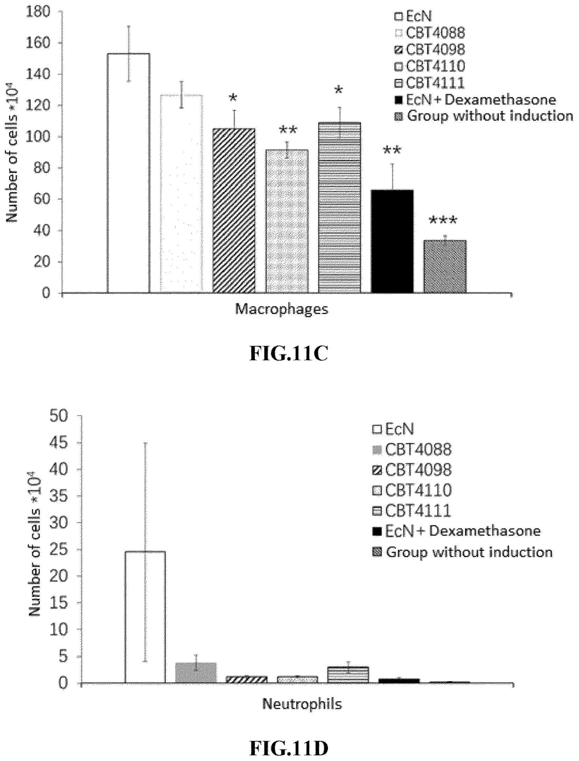

FIG. 11C shows the macrophage counting results in the bronchoalveolar lavage fluid (BALF) of mice in each experimental group of the asthma animal model;
FIG. 11D shows the neutrophil counting results in the bronchoalveolar lavage fluid (BALF) of mice in each experimental group of the asthma animal model;

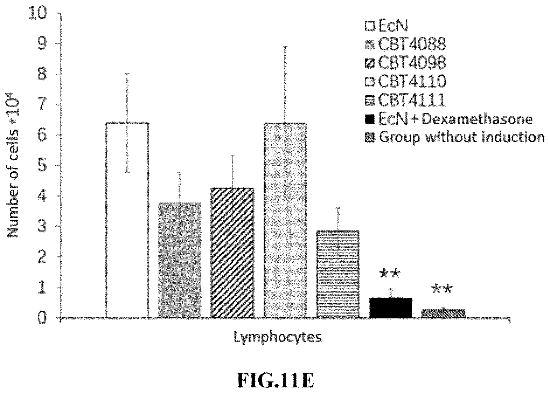

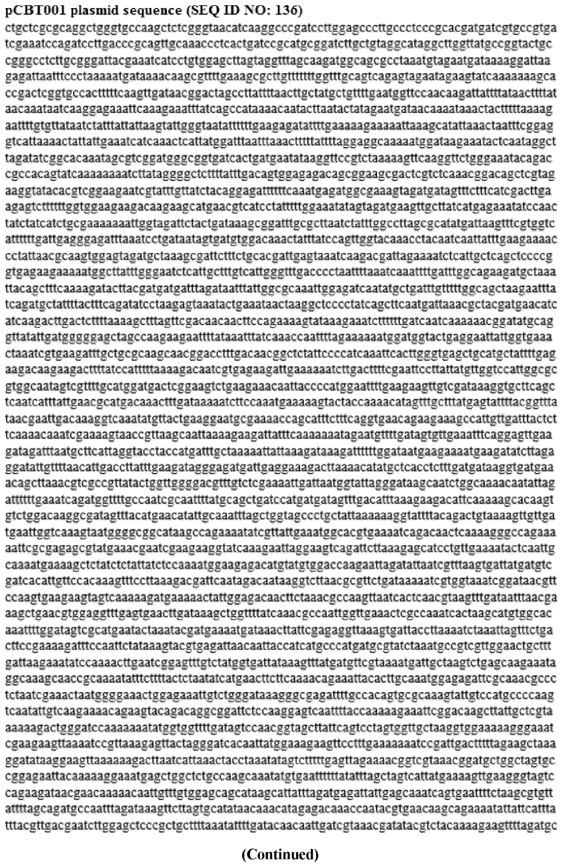

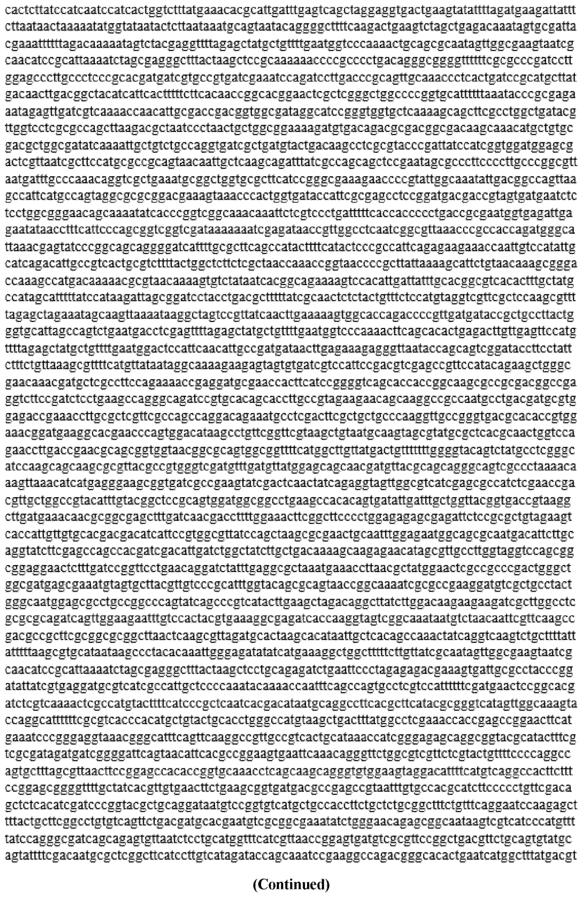

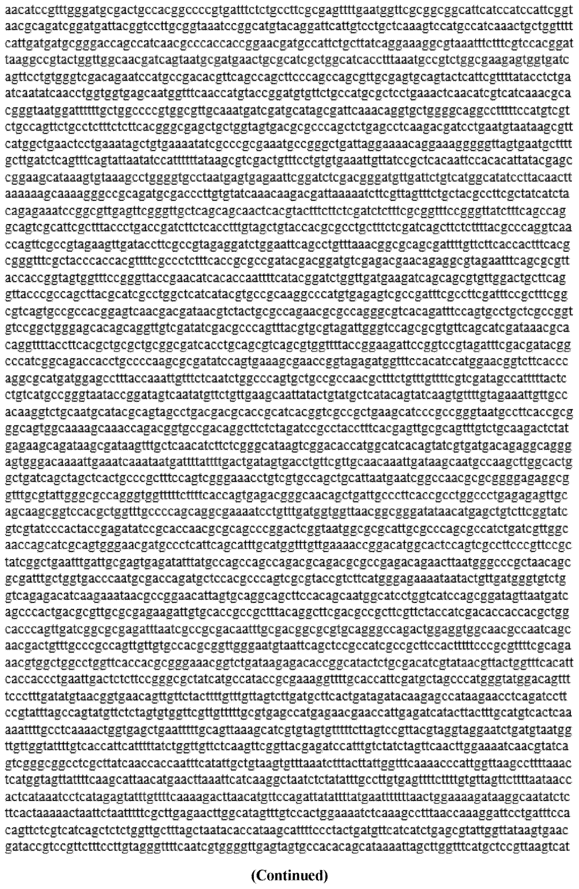

FIG. 11E shows the lymphocyte counting results in the bronchoalveolar lavage fluid (BALF) of mice in each experimental group of the asthma animal model.

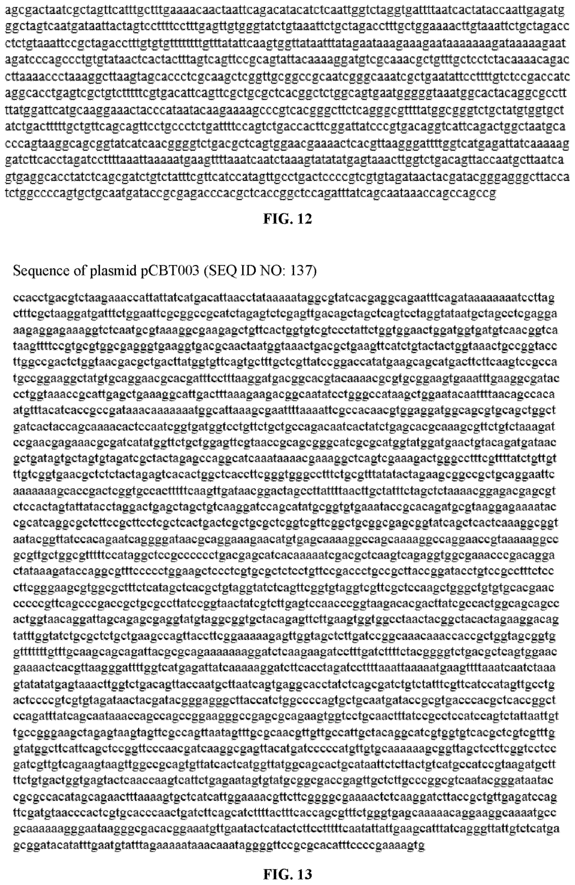

FIG. 12 shows the nucleotide sequence of the pCBT001 plasmid expressing Cas9 protein (SEQ ID NO: 136).

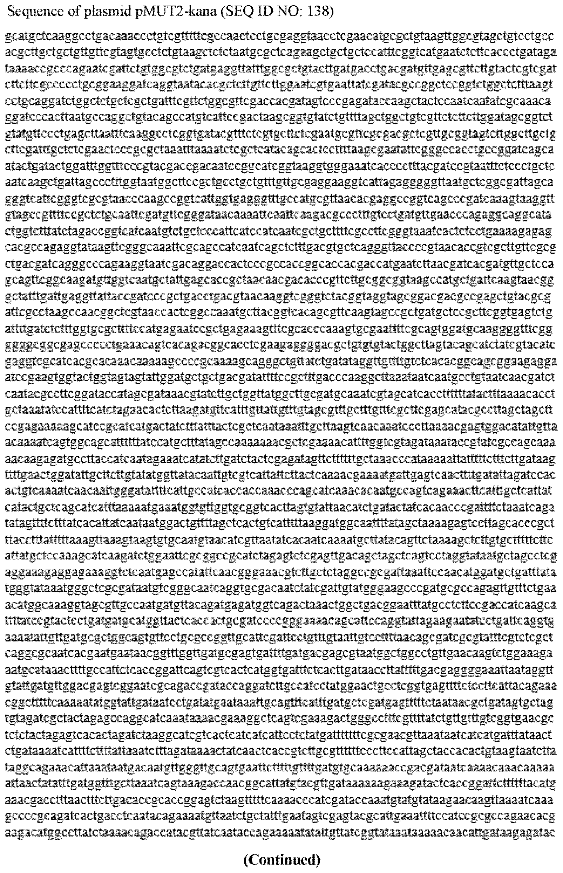

FIG. 13 shows the nucleotide sequence of pCBT003 plasmid (SEQ ID NO: 137).

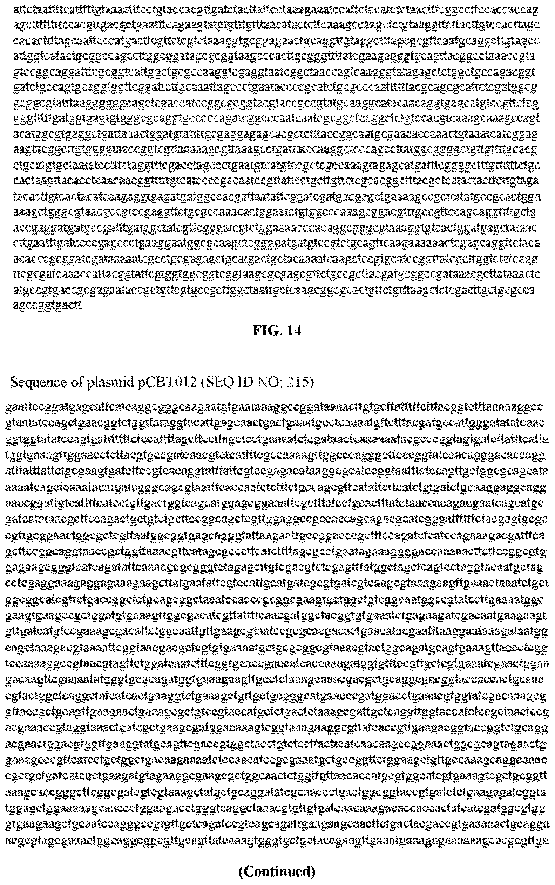

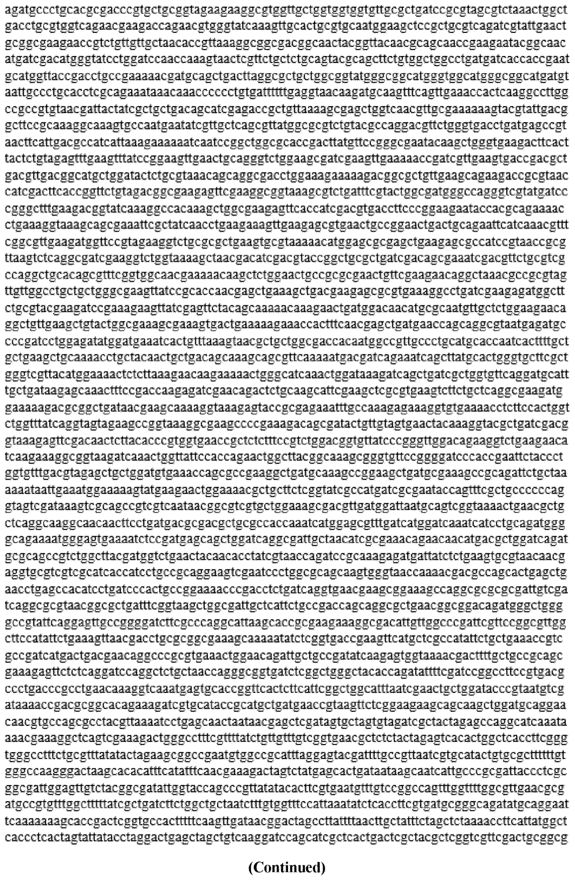

FIG. 14 shows the nucleotide sequence of pMUT2-kana plasmid (SEQ ID NO: 138).

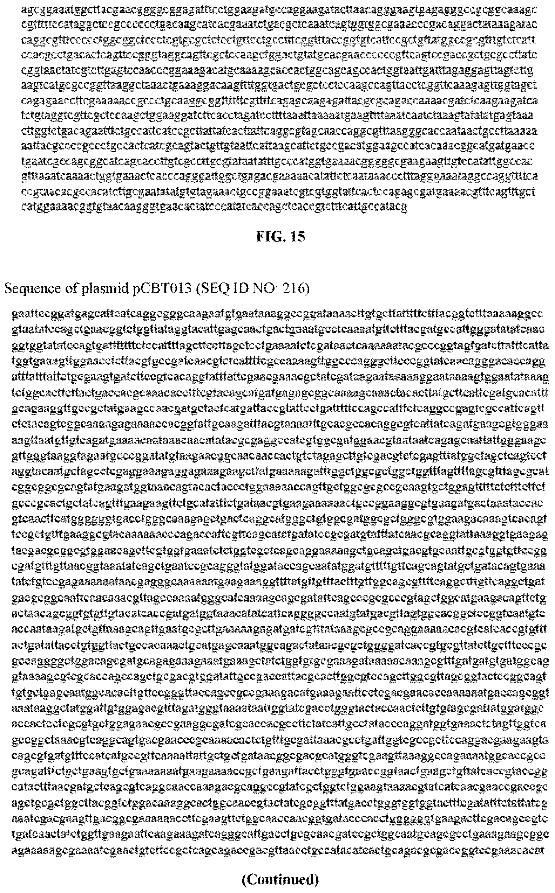

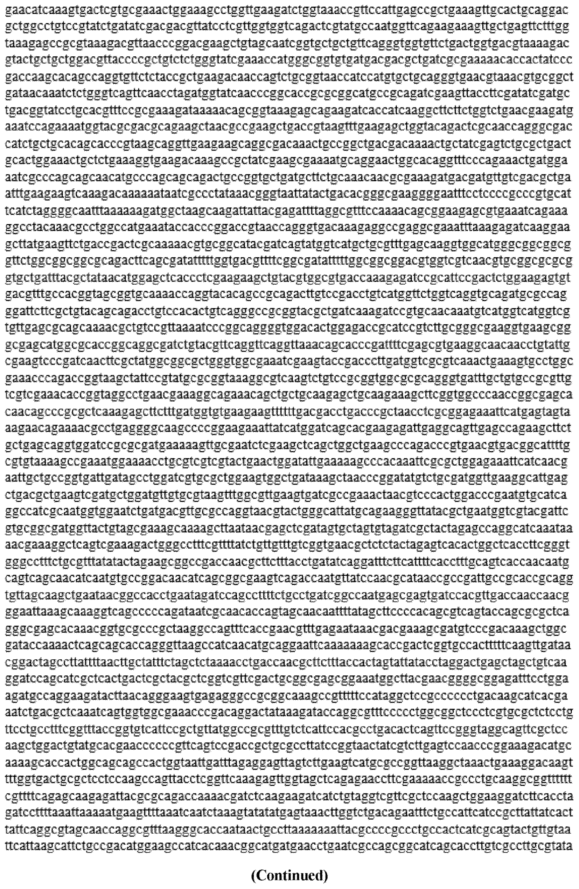

FIG. 15 shows the nucleotide sequence of pCBT012 plasmid (SEQ ID NO: 215).

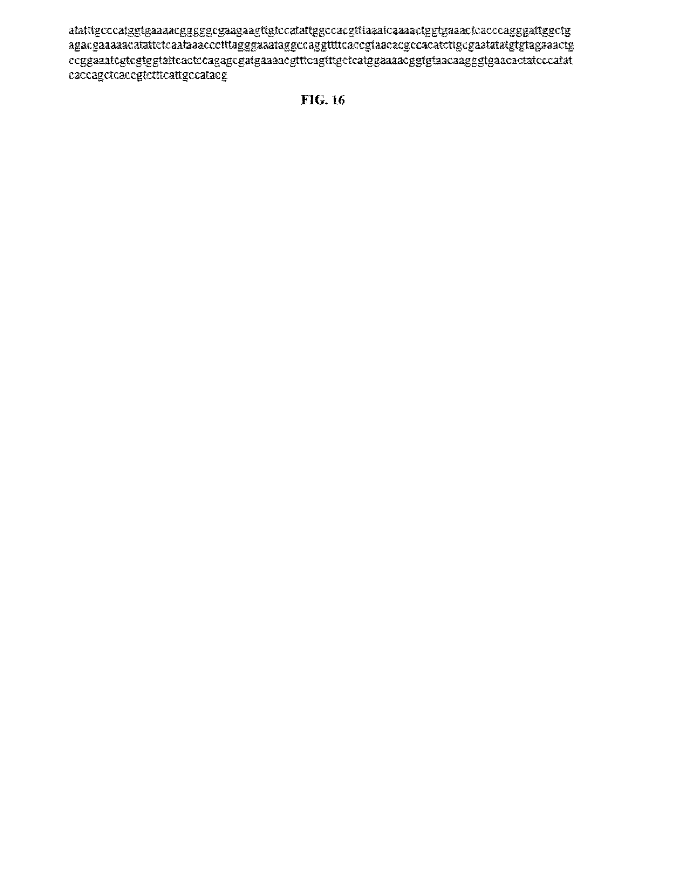

FIG. 16 shows the nucleotide sequence of pCBT013 plasmid (SEQ ID NO: 216).

### DETAILED DESCRIPTION

The following description of the present disclosure is intended only to illustrate various embodiments of the present disclosure. Therefore, the specific modifications discussed should not be construed as limitations on the scope of the present disclosure. It will be apparent to those skilled in the art that various equivalents, changes and modifications may be made without departing from the scope of the present disclosure, and it is understood that such equivalent embodiments are intended to be included herein. All references, including publications, patents, and patent applications cited herein are incorporated by reference in their entirety.

### I. Definition

As used herein, "a" and "the" are used herein to refer to one (kind) or more than one (kind) (i.e., at least one (kind)) of the grammatical object of the article. For example, "a protein" means one protein or more than one protein.

The term "about" may refer to a value or composition that is within an acceptable error range for the particular value or composition as determined by those of ordinary skill in the art, which will depend in part on how the value or composition is measured or determined.

As used herein, the term "amino acid" refers to organic compounds containing amino (-NH₂) and carboxyl (-COOH) functional groups and side chains unique to each amino acid. The names of amino acids are also represented in the present disclosure by standard one-letter or three-letter codes, an overview of which is shown in Table 1.

**Table 1 Amino acid names and codes**

| **Name** | **Three letter code** | **One letter code** |
|---|---|---|
| Alanine | Ala | A |
| Arginine | Arg | R |
| Asparagine | Asn | N |
| Aspartic acid | Asp | D |
| Cysteine | Cys | C |
| Glutamic acid | Glu | E |
| Glutamine | Gln | Q |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

The terms "polypeptide," "peptide," and "protein" are used interchangeably herein to refer to a polymer of amino acid residues. The terms also apply to amino acid polymers in which one or more amino acid residues are artificial chemical mimetics of corresponding naturally occurring amino acids, and apply to naturally occurring amino acid polymers and non-naturally occurring amino acid polymers.

A "conservative substitution" with respect to an amino acid sequence refers to the replacement of an amino acid residue with a different amino acid residue containing side chains with similar physicochemical properties. For example, a conservative substitution can occur among amino acid residues with hydrophobic side chains (e.g., Met, Ala, Val, Leu, and Ile), among amino acid residues with neutral hydrophilic side chains (e.g., Cys, Ser, Thr, Asn and Gln), among amino acid residues with acidic side chains (e.g., Asp, Glu), among amino acid residues with basic side chains (e.g., His, Lys, and Arg), or among amino acid residues with aromatic side chains (e.g., Trp, Tyr and Phe). As is known in the art, conservative substitutions generally do not cause significant changes in the conformational structure of the protein, and therefore the biological activity of the protein may be preserved.

As used herein, the term "mutant" refers to a polypeptide or polynucleotide that has at least 70% sequence identity to the parent sequence. A mutant may differ from the parent sequence by one or more amino acid residues or one or more nucleotides. For example, a mutant may have a substitution (including but not limited to conservative substitution), addition, deletion, insertion or truncation of one or more amino acid residues or one or more nucleotides of the parent sequence, or any combination thereof. For example, in certain embodiments, the mutant Amuc_1100 has at least 75%, 80%, 85%, 90%, 91%, 92%, 93%, 94%, 95%, 96%, 97 %, 98%, 99% or more sequence identity with its naturally occurring counterpart.

As used herein, the term "fragment" refers to a partial sequence of a parent polypeptide or parent polynucleotide of any length. The fragment may still at least partially retain the functionality of the parent sequence.

The term "fusion/fused" when applied to an amino acid sequence (e.g., a peptide, polypeptide, or protein) refers to the combination of two or more amino acid sequences into a non-naturally occurring single amino acid sequence, e.g., by chemical bonding or recombination. A fusion amino acid sequence can be produced by genetic recombination of two encoding polynucleotide sequences and can be expressed by introducing a construct containing the recombinant polynucleotide into a microorganism.

The term "derivative" as used herein with respect to a polypeptide or polynucleotide refers to a chemically modified polypeptide or polynucleotide in which one or more well-defined number of substituents have been covalently connected to one or more specific amino acid residues of the polypeptide, or one or more specific nucleotides of the polynucleotide. Exemplary chemical modifications to a polypeptide may be, for example, alkylation, acylation, esterification, amidation, phosphorylation, glycosylation, labeling, methylation, or conjugation to one or more moieties of one or more amino acids. Exemplary chemical modifications to a polynucleotide may be (a) terminal modification, such as 5' end modification or 3' end modification, (b) nucleobase (or "base") modification, including substitution or removal of bases, (c) sugar modifications, including modifications at the 2', 3' and/or 4' positions, and (d) main chain modifications, including modifications or substitutions of phosphodiester bonds.

Such fragments, variants, derivatives, mutants and analogs are within the scope well known by those skilled in the art in light of the teachings herein. Among them, the conservatively substituted analogs are preferably produced by amino acid substitution according to Table 2.

**Table 2 Summary table of similar amino acids**

| **Original residue** | **Representative substitution(s)** | **Preferred substitution** |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lvs (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tvr; Phe | Tyr |
| Tyr (Y) | Trp; Phe; Thr; Ser | Phe |
| Val (V) | Ile; Leu; Met; Phe; Ala | Leu |

As used herein, the term "homologous" means that when best aligned, a nucleic acid sequence (or its complement) or amino acid sequence has at least 60% (e.g., at least 65%, 70%, 75%, 80%, 85%, 88%, 90%, 91%, 92%, 93%, 94%, 95%, 96%, 97%, 98%, 99%) sequence identity with another sequence.

The term "% sequence identity" with respect to an amino acid sequence (or a nucleic acid sequence) is defined as the percentage of amino acid (or nucleic acid) residues of a candidate sequence that are identical to those in a reference sequence after aligning the sequences and introducing gaps where necessary to achieve the maximum number of identical amino acids (or nucleic acids). In other words, the sequence identity percentage (%) of an amino acid sequence (or nucleic acid sequence) can be determined by dividing the number of amino acid residues (or bases) that are identical relative to the reference sequence to which it is compared by the total number of amino acid residues (or bases) in the candidate sequence or the reference sequence, whichever is shorter. Conservative substitutions of amino acid residues may or may not be considered the same residue. Alignments for the purpose of determining amino acid (or nucleic acid) sequence identity percentage can be realized, for example, using publicly available tools such as BLASTN, BLASTp (available at the U.S. National Center for Biotechnology Information; NCBI) website, also see Altschul S.F. et al., Journal of Molecular Biology, 215:403-410 (1990); Stephen F. et al., Nucleic Acids Res., 25:3389- 3402 (1997)), ClustalW2 (available on the European Bioinformatics Institute website, see also Higgins D.G. et al., Methods in Enzymology, 266:383-402 (1996); Larkin M.A. et al., Bioinformatics (Oxford, England), 23(21): 2947-8 (2007)) and ALIGN or Megalign (DNASTAR) software. Those skilled in the art can use the default parameters provided by the tool, or customize the parameters of the alignment as needed, for example by selecting a suitable algorithm.

As used herein, the terms "nucleotide sequence," "nucleic acid," or "polynucleotide" include oligonucleotides (i.e., short polynucleotides). It also refers to synthetic and/or non-naturally occurring nucleic acid molecules (e.g., containing nucleotide analogs or modified main chain residues or bonds). The term also refers to deoxyribonucleotide or ribonucleotide oligonucleotides in single- or double-stranded form. The term encompasses nucleic acids containing analogs of natural nucleotides. The term also encompasses nucleic acid-like structures with synthetic main chains. Unless otherwise indicated, a particular polynucleotide sequence also implicitly encompasses conservatively modified variants thereof (e.g., degenerate codon substitutions), alleles, orthologs, SNPs and complementary sequences thereof, and sequences explicitly indicated. In particular, degenerate codon substitution can be achieved by generating sequences in which the third position of one or more selected (or all) codons is replaced by mixed bases and/or deoxyinosine residues (see Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

As used herein, the term "encoded/encoding" means capable of being transcribed into mRNA and/or translated into peptides or proteins. The term "coding sequence" or "gene" refers to a polynucleotide sequence encoding a peptide or protein. These two terms may be used interchangeably in the present disclosure. In some embodiments, the coding sequence is a complementary DNA (cDNA) sequence reverse-transcribed from a messenger RNA (mRNA). In some embodiments, the coding sequence is mRNA.

The term "operably linked to" refers to the joining of two or more biological sequences of interest with or without a spacer or linker such that they are in a relationship that allows them to function in the intended manner. When used in relation to a polypeptide, it means that the polypeptide sequences are linked in a manner that allows the product of the linkage to have the intended biological function. For example, an antibody variable region can be operably linked to a constant region to provide a stable product with antigen-binding activity. The term may also be used with respect to polynucleotides. For example, when a polynucleotide encoding a polypeptide is operably linked to a regulatory sequence (e.g., a promoter, enhancer, silencer sequence, etc.), it means that the polynucleotide sequence is linked in a manner that allows expression of the regulatory polypeptide from the polynucleotide.

As used herein, the term "vector" refers to a vehicle into which a genetic element is operably inserted to realize expression of the genetic element to produce a protein, RNA, or DNA encoded by the genetic element, or to replicate the genetic element. Vectors can be used to transform, transduce or transfect host cells (e.g., microorganisms), so that the genetic elements carried therein are expressed within the host cells. Different vectors may be suitable for different host cells. Examples of vectors include plasmids; phagemids; cosmids; artificial chromosomes, such as yeast artificial chromosomes (YAC), bacterial artificial chromosomes (BAC), or P1-derived artificial chromosomes (PAC); phages, such as λ phage or M13 phage; and animal viruses. A vector can contain a variety of elements for controlling expression, including a promoter sequence, transcription initiation sequence, enhancer sequence, selectable element, and reporter gene. Additionally, a vector may contain an origin of replication. A vector may further contain a material that facilitates its entry into cells, including but not limited to a virion, liposome, or protein coating. A vector may be an expression vector or a cloning vector.

### II. Genetically modified microorganisms

In one aspect, the present application provides a genetically modified microorganism comprising at least one exogenous gene selected from the group consisting of: a gene encoding Amuc_1100 polypeptide, a gene encoding IL-10 polypeptide, a gene encoding IL-22 polypeptide, and any combination thereof.

In another aspect, the present application provides a genetically modified microorganism comprising at least two exogenous genes encoding polypeptides selected from the group consisting of: (i) Amuc_1100 polypeptide, (ii) IL-10 polypeptide, and (iii) IL-22 polypeptide.

In certain embodiments, the genetically modified microorganism comprises: a) one exogenous gene encoding Amuc_1100 polypeptide and one exogenous gene encoding IL-10 polypeptide; b) one exogenous gene encoding IL-10 polypeptide and one exogenous gene encoding IL-22 polypeptide; or c) one exogenous gene encoding Amuc_1100 polypeptide and one exogenous gene encoding IL-22 polypeptide.

In certain embodiments, the genetically modified microorganism comprises exogenous genes encoding Amuc_1100 polypeptide, IL-10 polypeptide and IL-22 polypeptide respectively.

In certain embodiments, the genetically modified microorganism comprises an exogenous gene encoding Amuc_1100 polypeptide, IL-10 polypeptide, or IL-22 polypeptide.

As used herein, an "exogenous gene" is a gene introduced into the microorganism by genetic modification. An exogenous gene may include heterologous genes that are not originally expressed in the microorganism, or may include endogenous genes that are introduced through genetic modification and are also originally expressed in the microorganism (for example, for the purpose of increasing expression levels).

In the present application, the polypeptide expressed by the exogenous gene in the microorganism is also called an exogenous polypeptide. In some embodiments, the exogenous polypeptide is selected from the group consisting of: Amuc_1100 polypeptide, IL-10 polypeptide, and IL-22 polypeptide. In some embodiments, the exogenous polypeptide is a heterologous polypeptide not originally expressed in the microorganism, for example, human IL-10 polypeptide, human IL-22 polypeptide, etc. When the microorganism used does not express Amuc_1100 endogenously, Amuc_1100 is also a heterologous polypeptide.

Microorganisms may be used in the present invention include bacteria, archaea, fungi (e.g., yeast, filamentous fungi) and algae.

In some embodiments, the microorganisms provided herein include probiotic microorganisms or non-pathogenic microorganisms.

As used herein, "non-pathogenic microorganisms" refer to microorganisms that are unable to cause disease or harmful reactions in the host. In some embodiments, the non-pathogenic microorganisms do not contain lipopolysaccharide (LPS). In some embodiments, the non-pathogenic microorganisms are commensal bacteria. In some embodiments, the non-pathogenic microorganisms are attenuated pathogenic bacteria.

In some embodiments, the microorganisms used in the present invention are probiotic microorganisms. As used herein, "probiotic microorganisms" refer to microbiota that provide a beneficial effect on the health or well-being of an individual when administered in an effective amount, including, for example, health benefits associated with improving the balance of the human or animal microbiota, and/or for restoring normalcy. By definition, all probiotic microorganisms have proven nonpathogenic characteristics. In general, probiotics help the gut microbiota maintain (or back to) its balance, integrity, and diversity. The effects of probiotics might be strain dependent.

In some embodiments, the probiotic microorganism includes a preparation of probiotic microbial cells (e.g., live microbial cells).

In some embodiments, the probiotic microorganism is a probiotic bacterium or probiotic yeast.

In some embodiments, the probiotic bacterium is selected from the group consisting of: *Bacteroidetes, Bifidobacteria* (e.g., *Bifidobacterium bifidum*), *Clostridium, Escherichia, Lactobacilli* (e.g., *Lactobacillus acidophilus, Lactobacillus bulgaricus coli, Lactobacillus paracasei, Lactobacillus plantarum)* and *Lactococcus.* In some embodiments, the probiotic bacterium is a bacterium of the genus *Escherichia.* In some embodiments, the probiotic bacterium belongs to the species *Escherichia coli* strain Nissle 1917 (EcN). In some embodiments, the probiotic yeast is selected from the group consisting of: *Saccharomyces cerevisiae, Candida utilis, Kluyveromyces lactis,* and *Saccharomyces carlsbergensis.* In some embodiments, the probiotic microorganism is *Escherichia coli* strain Nissle 1917 (EcN).

As a model strain for studying microbial genetics, physiology and metabolism, *Escherichia coli* has become one of the important classic bacteria or host bacteria due to its advantages of diverse genetic manipulation tools and clear genetic background.

In some embodiments, the probiotic bacterium is a bacterium normally found in the human intestinal tract.

In some embodiments, the probiotic yeast is selected from the group consisting of: *Saccharomyces cerevisiae, Candida utilis, Kluyveromyces lactis,* and *Saccharomyces carlsbergensis.*

In the present application, the term "genetic modification" refers to introducing changes or modifications in DNA and/or RNA or introducing exogenous DNA or RNA into cells (e.g., microorganisms) by artificial intervention. Generally, the changes or modifications can be introduced through recombinant nucleic acid expression vectors, or through mutation, or through gene editing.

As used herein, the term "genetically modified microorganism" refers to a microorganism into which an exogenous gene or an exogenous expression cassette (including a vector containing an expression cassette) has been introduced. "Genetically modified microorganism" may also be used interchangeably with "genetically engineered microorganism", "engineered microorganism", "genetically modified microorganism" or "genetically engineered microorganism". In the present application, the exogenous expression cassette contains a polynucleotide encoding a polypeptide or protein of interest and can allow its expression. Introduction of an expression cassette into a microorganism can be achieved by calcium phosphate transfection, DEAE-dextran mediated transfection, or electroporation (Davis, L., Dibner, M., Battey, I., Basic Methods in Molecular Biology (1986)), and can also be achieved through gene editing technology (for example, CRISPR technology).

Genetically modified microorganisms also include any descendants of the microorganisms provided herein or derivatives thereof. It is understood that all descendants may differ from the parent cell due to possible mutations that occurred during replication.

The present application further provides a nucleotide sequence comprising at least one recombinant expression cassette, which comprises i) at least one or at least two exogenous genes encoding Amuc_1100 polypeptide, IL-10 polypeptide and/or IL-22 polypeptide respectively, and ii) one or more regulatory elements operably linked to the at least one or at least two exogenous genes.

In some embodiments, the present disclosure further provides a combination of genetically modified microorganisms, which comprises a combination of a genetically modified microorganism comprising at least one (e.g., at least one, at least two, or at least three) exogenous gene described in the present disclosure.

In some embodiments, the combination of genetically modified microorganisms comprises two different genetically modified microorganisms, wherein each genetically modified microorganism expresses one different exogenous gene respectively; wherein, the exogenous genes encode polypeptides selected from the group consisting of a) Amuc_1100 polypeptide and IL-10 polypeptide; b) IL-10 polypeptide and IL-22 polypeptide; and c) Amuc_1100 polypeptide and IL-22 polypeptide.

In some embodiments, the combination of genetically modified microorganisms comprises three different genetically modified microorganisms, wherein each genetically modified microorganism expresses one different exogenous gene respectively; or the combination of genetically modified microorganisms comprises two different genetically modified microorganisms, wherein the two genetically modified microorganisms respectively express one exogenous gene and two other exogenous genes; wherein the exogenous gene encode polypeptide selected from the group consisting of Amuc_1100 polypeptide, IL-10 polypeptide and IL-22 polypeptide.

### A. Amuc_1100

Amuc_1100 is known as an outer membrane protein present in the bacterial species *Akkermansia muciniphilα* ("*A. muciniphila*"). Along with two other members, Amuc_1099 and Amuc_1101, Amuc_1100 is located within a gene cluster involved in type IV pilus-like formation (Ottman et al., PLoS One, 2017). An exemplary amino acid sequence of Amuc_1100 is disclosed in GenBank: ACD04926.1. In the present disclosure, the terms "Amuc_1100" and "AMUC_1100" are used interchangeably.

As used herein, the term "Amuc_1100" broadly encompasses Amuc_1100 polypeptides and Amuc_1100 polynucleotides, such as DNA or RNA sequences encoding Amuc_1100 polypeptides. As used herein, the term "Amuc_1100" further encompasses wild-type Amuc_1100 and functional equivalents that are functionally equivalent to the wild-type Amuc_1100 polypeptide.

In the present application, when the term "wild-type" is used to describe a polypeptide or polynucleotide, it means that the sequence of the polypeptide or polynucleotide is identical to those found in nature. A wild-type polypeptide or polynucleotide may be a native or naturally occurring polypeptide or polynucleotide sequence, and broadly includes fragments thereof, even though the fragments themselves may not be found in nature.

In the present application, when the term "functional equivalent" is used to describe a polypeptide or polynucleotide, it refers to any variant that at least partially retains one or more biological functions of the reference sequence despite differences in its amino acid sequence or polynucleotide sequence or chemical structure compared with the reference sequence.

In the present application, when the term "variant" is used to describe a polypeptide or polynucleotide, it encompasses all different forms of the polypeptide or polynucleotide, including, but not limited to, naturally occurring fragments, mutants, fusions, derivatives, mimetics of the polypeptide or polynucleotide, or any combination thereof.

In the present application, the term "wild-type Amuc_1100" means that the polypeptide or polynucleotide sequence of the Amuc_1100 is identical to the sequence or sequences found in nature. Wild-type Amuc_1100 may be the native or naturally occurring Amuc_1100 sequence and fragments thereof, even though the fragments themselves may not be found in nature. Wild-type Amuc_1100 may further include naturally occurring variants, such as mutants or isomers with same functions found in different bacterial strains or different native sequences. The wild-type, full-length Amuc_1100 polypeptide is 317 amino acid residues in length. Exemplary amino acid sequences of wild-type Amuc_1100 include, but are not limited to, amino acid sequences such as Amuc_1100 (1-317) set forth in SEQ ID NO: 1, Amuc_1100 (31-317) shown at positions 31-317 in the amino acid sequence set forth in SEQ ID NO: 1 and Amuc_1100 (81-317) shown at positions 81-317 in the amino acid sequence set forth in SEQ ID NO: 1.

Amuc 1100 (1-317) amino acid sequence (SEQ ID NO: 1:

MSNWITDNKPAAMVAGVGLLLFLGLSATGYIVNSKRSELDKKISIAAKEIKSANAAEITPSRSSNEELEK
ELNRYAKAVGSLETAYKPFLASSALVPTTPTAFQNELKTFRDSLISSCKKKNILITDTSSWLGFQVYSTQA
PSVQAASTLGFELKAINSLVNKLAECGLSKFIKVYRPQLPIETPANNPEESDEADQAPWTPMPLEIAFQG
DRESVLKAMNAITGMQDYLFTVNSIRIRNERMMPPPIANPAAAKPAAAQPATGAASLTPADEAAAPAAP
AIQQVIKPYMGKEQVFVQVSLNLVHFNQPKAQEPSED.

In some embodiments, the Amuc_1100 polypeptide includes a functional equivalent of the wild-type Amuc_1100 polypeptide.

As used herein, a functional equivalent of the wild-type Amuc_1100 refers to any Amuc_1100 variant that at least partially retains one or more biological functions of wild-type Amuc_1100 despite differences in their amino acid sequences or polynucleotide sequences or chemical structures. The biological functions of wild-type Amuc_1100 include but are not limited to a) regulating and/or promoting intestinal immune system function of mammals, b) maintaining, restoring and/or increasing physical integrity of the intestinal mucosal barrier of mammals, c) activating TLR2, d) enhancing the immune response to cancer immunotherapy (e.g., immune checkpoint modulators) in mammals, and e) reducing, delaying, and/or preventing resistance to one or more immune checkpoint modulators in mammals.

In certain embodiments, the functional equivalent of the Amuc_1100 substantially retains biological activity of the parent molecule. In certain embodiments, the functional equivalent of the Amuc_1100 described herein substantially retains similar functions as wild-type Amuc_1100, e.g., the functional equivalent of the Amuc_1100 may retain at least a portion (e.g., at least 80%, 85%, 90%, 91%, 92%, 93%, 94%, 95%, 96%, 97%, 98%, 99% or 100%) of activity of wild-type Amuc_1100, such as the activity of modulating intestinal immunity and/or activating TLR2.

The functional equivalent of the Amuc_1100 polypeptide may include a mutant, a fragment, a fusion, a derivative of wild-type Amuc_1100, or any combination thereof. The functional equivalent of the Amuc_1100 may include a variant produced by artificial modification of wild-type Amuc_1100, such as an artificial polypeptide sequence obtained by a recombinant method or chemical synthesis. The functional equivalent of the Amuc_1100 may contain non-naturally occurring amino acid residues without affecting activity. Suitable non-naturally occurring amino acids include, for example, β-fluoroalanine, 1-methylhistidine, γ-methyleneglutamic acid, α-methylleucine, 4,5-dehydrolysine, hydroxylproline, 3-fluorophenylalanine, 3-aminotyrosine, 4-methyltryptophan, and so on.

In the present application, an Amuc_1100 variant may encompass all different forms of Amuc_1100, including but not limited to a fragment, a mutant, a fusion, a derivative, or a mimetic of wild-type Amuc_1100, or any combination thereof.

While not wishing to be bound by any theory, certain fragments of wild-type Amuc_1100, such as Amuc_1100 (31-317), can bind and activate TLR2 with higher affinity than their full-length counterpart.

In some embodiments, the amino acid sequence of a fragment of Amuc_1100 is set forth in SEQ ID NO: 2.

AINSLVNKLAECGLSKFIKVYRPQL (SEQ ID NO: 2).

In some embodiments, the amino acid sequence of a fragment of Amuc_1100 is set forth in SEQ ID NO: 3.

SLETAYKPFLASSALVPTTPTAFQNELKTFRDSLISSCKKKNILITDTSSWLGFQVYSTQAPSV
QAASTLGFELKAINSLVNKLAECGLSKFIKVYRPQLPIETPANNPEESDEADQAPWTPMPLEIAFQGDRE
SVLKAMNAITGMQDYLFTVNSIRIRNERMMPPPIANPAAAKPAAAQPATGAASLTPADEAAAPAAPAIQ
QVIKPYMGKEQVFVQVSLNLVHFNQ PKAQEPSED (SEQ ID NO: 3).

In some embodiments, the Amuc_1100 polypeptide comprises the sequence set forth in SEQ ID NO: 5, or comprises an amino acid sequence having at least 80% sequence identity with the sequence set forth in SEQ ID NO: 5 and still maintaining activity of modulating intestinal immunity and/or or activating toll-like receptor 2 (TLR2). In some embodiments, the Amuc_1100 polypeptide comprises an amino acid sequence that has at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99 % sequence identity with the sequence set forth in SEQ ID NO: 5 and still maintains activity of modulating intestinal immunity and/or activating toll-like receptor 2 (TLR2). In some embodiments, the nucleotide sequence encoding the amino acid sequence set forth in SEQ ID NO: 5 is set forth in SEQ ID NO: 4.

In certain embodiments, the Amuc_1100 polypeptide has a Y259A or Y259S mutation at position 259, and the position 259 is numbered based on the numbering of the sequence set forth in SEQ ID NO: 5. In some embodiments, the Amuc_1100 polypeptide having Y259A mutation comprises an amino acid sequence set forth in SEQ ID NO: 7. In some embodiments, the nucleotide sequence encoding the amino acid sequence set forth in SEQ ID NO: 7 is set forth in SEQ ID NO: 6. In some embodiments, the Amuc_1100 polypeptide having Y259S mutation comprises an amino acid sequence set forth in SEQ ID NO: 9. In some embodiments, the nucleotide sequence encoding the amino acid sequence set forth in SEQ ID NO: 9 is set forth in SEQ ID NO: 8.

In certain embodiments, the Amuc_1100 polypeptide further comprises a tag or amino acid extension at the N-terminus or C-terminus. The tag can be used for purification of the Amuc_1100 polypeptide. The amino acid extension can be used to improve stability or reduce clearance of the polypeptide. Any suitable tag or extension can be used, such as His-tag (e.g. 6×His tag), protein A, lacZ (β-gal), maltose-binding protein (MBP), calmodulin-binding peptide (CBP), intein-chitin binding domain (intein-CBD) tag, streptavidin/biotin-based tag, tandem affinity purification (TAP) tag, epitope tag, reporter gene tag, and the like.

In certain embodiments, the Amuc_1100 polypeptide further comprises an enzyme digestion site between the tag and the rest of the Amuc_1100 polypeptide. The enzyme digestion site is available for tag removal when required. Examples of suitable enzyme digestion site include enterokinase recognition site (e.g., DDDDK), factor Xa recognition site, genenase I recognition site, furin recognition site, and the like.
DNA sequence of Amuc_1100 (SEQ ID NO: 4): 
attgttaacagcaagcgtagcgagctggataagaaaatcagcattgcggcgaaagagatcaagagcgcgaacgcggcggaaattaccccgag
ccgtagcagcaacgaggaactggagaaagaactgaaccgttacgcgaaggcggttggtagcctggaaaccgcgtataaaccgtttctggcgagcagcgcgct
ggtgccgaccaccccgaccgcgttccaaaacgagctgaaaacctttcgtgacagcctgatcagcagctgcaagaaaaagaacatcctgattaccgataccagca
gctggctgggcttccaggtttacagcacccaagcgccgagcgtgcaggcggcgagcaccctgggttttgagctgaaagcgattaacagcctggttaacaagctg
gcggaatgcggcctgagcaaattcatcaaggtgtatcgtccgcagctgccgattgaaaccccggcgaacaacccggaggaaagcgacgaagcggatcaggc
gccgtggaccccgatgccgctggagatcgcgttccagggtgaccgtgaaagcgttctgaaagcgatgaacgcgattaccggcatgcaagattacctgtttaccgt
gaacagcatccgtattcgtaacgaacgtatgatgccgccgccaatcgcgaacccggctgcggcgaagccggctgcggcgcagccggcgaccggtgcggcg
agcctgaccccggcggacgaggctgcggcgccggctgcgccggcgatccagcaagttattaaaccgtatatgggcaaggaacaggtgttcgttcaagtgagc
ctgaacctggtgcactttaaccagccgaaagcgcaagagccgagcgaagattga.
Amino acid sequence of Amuc_1100 (SEQ ID NO: 5): 
IVNSKRSELDKKISIAAKEIKSANAAEITPSRSSNEELEKELNRYAKAVGSLETAYKPFLASSAL
VPTTPTAFQNELKTFRDSLISSCKKKNILITDTSSWLGFQVYSTQAPSVQAASTLGFELKAINSLVNKLA
 
ECGLSKFIKVYRPQLPIETPANNPEESDEADQAPWTPMPLEIAFQGDRESVLKAMNAITGMQDYLFTV
NSIRIRNERMMPPPIANPAAAKPAAAQPATGAASLTPADEAAAPAAPAIQQVIKPYMGKEQVFVQVSLN
LVHFNQPKAQEPSED*.
DNA sequence of Amuc_1100 (Y259A) (SEQ ID NO: 6): 
attgttaacagcaagcgtagcgagctggataagaaaatcagcattgcggcgaaagagatcaagagcgcgaacgcggcggaaattaccccgag
ccgtagcagcaacgaggaactggagaaagaactgaaccgttacgcgaaggcggttggtagcctggaaaccgcgtataaaccgtttctggcgagcagcgcgct
ggtgccgaccaccccgaccgcgttccaaaacgagctgaaaacctttcgtgacagcctgatcagcagctgcaagaaaaagaacatcctgattaccgataccagca
gctggctgggcttccaggtttacagcacccaagcgccgagcgtgcaggcggcgagcaccctgggttttgagctgaaagcgattaacagcctggttaacaagctg
gcggaatgcggcctgagcaaattcatcaaggtgtatcgtccgcagctgccgattgaaaccccggcgaacaacccggaggaaagcgacgaagcggatcaggc
gccgtggaccccgatgccgctggagatcgcgttccagggtgaccgtgaaagcgttctgaaagcgatgaacgcgattaccggcatgcaagattacctgtttaccgt
gaacagcatccgtattcgtaacgaacgtatgatgccgccgccaatcgcgaacccggctgcggcgaagccggctgcggcgcagccggcgaccggtgcggcg
agcctgaccccggcggacgaggctgcggcgccggctgcgccggcgatccagcaagttattaaaccggctatgggcaaggaacaggtgttcgttcaagtgagc
ctgaacctggtgcactttaaccagccgaaagcgcaagagccgagcgaagat.
Amino acid sequence of Amuc_1100 (Y259A) (SEQ ID NO: 7): 
IVNSKRSELDKKISIAAKEIKSANAAEITPSRSSNEELEKELNRYAKAVGSLETAYKPFLASSAL
VPTTPTAFQNELKTFRDSLISSCKKKNILITDTSSWLGFQVYSTQAPSVQAASTLGFELKAINSLVNKLA
ECGLSKFIKVYRPQLPIETPANNPEESDEADQAPWTPMPLEIAFQGDRESVLKAMNAITGMQDYLFTV
NSIRIRNERMMPPPIANPAAAKPAAAQPATGAASLTPADEAAAPAAPAIQQVIKPAMGKEQVFVQVSLN
LVHFNQPKAQEPSED*.
DNA sequence of Amuc_1100 (Y259S) (SEQ ID NO: 8): 
attgttaacagcaagcgtagcgagctggataagaaaatcagcattgcggcgaaagagatcaagagcgcgaacgcggcggaaattaccccgag
ccgtagcagcaacgaggaactggagaaagaactgaaccgttacgcgaaggcggttggtagcctggaaaccgcgtataaaccgtttctggcgagcagcgcgct
ggtgccgaccaccccgaccgcgttccaaaacgagctgaaaacctttcgtgacagcctgatcagcagctgcaagaaaaagaacatcctgattaccgataccagca
gctggctgggcttccaggtttacagcacccaagcgccgagcgtgcaggcggcgagcaccctgggttttgagctgaaagcgattaacagcctggttaacaagctg
gcggaatgcggcctgagcaaattcatcaaggtgtatcgtccgcagctgccgattgaaaccccggcgaacaacccggaggaaagcgacgaagcggatcaggc
gccgtggaccccgatgccgctggagatcgcgttccagggtgaccgtgaaagcgttctgaaagcgatgaacgcgattaccggcatgcaagattacctgtttaccgt
gaacagcatccgtattcgtaacgaacgtatgatgccgccgccaatcgcgaacccggctgcggcgaagccggctgcggcgcagccggcgaccggtgcggcg
agcctgaccccggcggacgaggctgcggcgccggctgcgccggcgatccagcaagttattaaaccgagcatgggcaaggaacaggtgttcgttcaagtgag
cctgaacctggtgcactttaaccagccgaaagcgcaagagccgagcgaagat.
Amino acid sequence of Amuc_1100 (Y259S) (SEQ ID NO: 9):

IVNSKRSELDKKISIAAKEIKSANAAEITPSRSSNEELEKELNRYAKAVGSLETAYKPFLASSAL
VPTTPTAFQNELKTFRDSLISSCKKKNILITDTSSWLGFQVYSTQAPSVQAASTLGFELKAINSLVNKLA
ECGLSKFIKVYRPQLPIETPANNPEESDEADQAPWTPMPLEIAFQGDRESVLKAMNAITGMQDYLFTV
NSIRIRNERMMPPPIANPAAAKPAAAQPATGAASLTPADEAAAPAAPAIQQVIKPSMGKEQVFVQVSLNL
VHFNQPKAQEPSED*.

In addition, since Amuc_1100 is a membrane protein, its N-terminal signal peptide sequence will promote membrane localization of the Amuc_1100 polypeptide. Therefore, in order to achieve the purpose of the present disclosure, that is, to achieve the secretion of Amuc_1100 polypeptide, it is necessary to replace the signal peptide of Amuc_1100 its own with other signal peptides of the present disclosure, which are capable of secreting Amuc_1100 polypeptide outside the body of microorganism, such as USP45 signal peptide.

### B. Cytokines

The genetically modified microorganism provided by the present disclosure can further express and secrete one or more cytokines.

In some embodiments, the cytokine is selected from the group consisting of IL-10 and IL-22.

In some embodiments, the cytokine may be IL-17A, IL-19, IL-23, IL-35, IL-37 or TGF-beta.

### Interleukin-10 (IL-10)

IL-10 is an anti-inflammatory cytokine that maintains the balance of immune responses and is synthesized by multiple types of cells, including B cells, monocytes, dendritic cells, natural killer cells, and T cells. IL-10 is recognized by specific receptors expressed by hematopoietic cells and belongs to class II cytokine. IL-10 transmits signals through two receptors, IL-10R1 and IL-10R2, and their downstream JAK/STAT pathway, ultimately activating the expression of anti-inflammatory response genes. IL-10 can inhibit the activity of macrophages and dendritic cells, and indirectly inhibit the activation of T cells and effector functions. IL-10 has a protective effect on inflammatory bowel disease, allergic reactions, etc. Defects in IL-10 and/or its receptors are associated with IBD and intestinal sensitivity (Nielsen, 2014).

Interleukin-10 (IL-10) is a pleiotropic cytokine produced by several types of cells, such as macrophages, monocytes, Th2-type and regulatory T cells, and B cells. IL-10 is a cytokine with immunosuppressive and anti-inflammatory properties, which regulates the activity of many myeloid and lymphoid cells and directly inhibits the production of several inflammatory cytokines by T cells and natural killer (NK) cells.

IL-10 was originally described as a cytokine synthesis inhibitory factor (CSIF) produced by Th2 cells. Th2 cells inhibit the production of pro-inflammatory cytokines by Th1 cells, such as γ-interferon (IFN-γ), interleukin-1-α (IL-1α), interleukin-1-β (IL-1β), interleukin-2 (IL-2), and tumor necrosis factor α (TNF-α). It has been shown that, in addition to inhibiting the production of pro-inflammatory cytokines, IL-10 can inhibit the proliferation of antigen-specific Th1 cells, thereby reducing the antigen presentation ability of monocytes by deregulating the expression of major histocompatibility complex (MHC)-II in these cells.

The finding that IL-10 has a strong inhibitory effect on the activation of Th1 cells and on the production of pro-inflammatory cytokines led to the hypothesis that IL-10 is a strong immunosuppressant of cell-mediated immune responses. Other authors have proposed the use of this cytokine in the treatment of acute and chronic inflammation and in the treatment of autoimmune diseases. For these reasons, this cytokine has been used in several autoimmune diseases, such as psoriasis, rheumatoid arthritis, and Crohn's disease. However, in other diseases, such as infectious processes or cancer, it has side effects because it prevents the induction of therapeutically beneficial Th1 responses. Examples of these processes include leprosy, tuberculosis, leishmaniasis, and viral infections. Therefore, IL-10 has been documented to be abundantly expressed in chronic infection caused by hepatitis C virus. This cytokine can be produced by Th2 cells as a result of stimulation with HCV antigens. It can also be produced by regulatory T cells (D4 and CD8) that inhibit the formation of Th1-type antiviral effector cells. Finally, infected dendritic cells (DCs) or monocytes in contact with HCV proteins produce greater amounts of IL-10 than non-infected cells, which favors the development of Th2 responses while hindering the elimination of virus.

In some embodiments, the genetically modified microorganism provided by the present disclosure expresses and secretes IL-10.

As used herein, the term "IL-10" broadly encompasses IL-10 and the polynucleotide of IL-10, such as DNA or RNA sequences encoding IL-10. As used herein, the term "IL-10" further encompasses wild-type IL-10, and functional equivalents that are functionally equivalent to wild-type IL-10. In the present application, a functional equivalent of the wild-type IL-10 refers to any IL-10 variant that at least partially retains one or more biological functions of wild-type IL-10 despite differences in their amino acid sequences or polynucleotide sequences or chemical structures. The one or more biological functions of wild-type IL-10 include, but are not limited to, the ability of binding IL-10R1/IL-10R2 and exerting functions through the receptor-JAK-STAT signaling pathway.

In some embodiments, the IL-10 comprises an amino acid sequence set forth in SEQ ID NO: 11, or an amino acid sequence having at least 80%, at least 85%, at least 90%, at least 95%, or at least 99% sequence identity with the sequence set forth in SEQ ID NO: 11 and still maintaining the activity of modulating immune cells (e.g., macrophages, dendritic cells). In some embodiments, the IL-10 comprises an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity with the sequence set forth in SEQ ID NO: 11 and still maintaining the activity of modulating immune cells (e.g., macrophages, dendritic cells).

In some embodiments, the IL-10 comprises a mutation of P2A, wherein the numbering is relative to SEQ ID NO: 13; that is, the IL-10 comprises an amino acid sequence set forth in SEQ ID NO: 13.

In the present application, the variant of IL-10 encompasses all different forms of IL-10, including but not limited to a fragment, a mutant, a fusion, a derivative, a mimetic, or any combination thereof of IL-10.

In some embodiments, the genetically modified microorganism provided by the present disclosure may comprise any suitable gene encoding IL-10 (e.g., human IL-10). In some embodiments, the gene encoding IL-10 comprises a nucleotide sequence set forth in SEQ ID NO: 10, or a nucleotide sequence having at least 80%, at least 85%, at least 90%, at least 95% or at least 99% sequence identity with the sequence set forth in SEQ ID NO: 10 and whose encoded protein still maintains the activity of modulating immune cells (e.g., macrophages, dendritic cells). In some embodiments, the gene encoding IL-10 comprises a nucleotide sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% sequence identity with the sequence set forth in SEQ ID NO: 10 and whose encoded protein still maintains the activity of modulating immune cells (e.g., macrophages, dendritic cells).

The gene encoding IL-10 may comprise modifications and/or mutations, for example, to enhance stability under inducing conditions, increase expression of IL-10, and/or increase anti-inflammatory efficacy. In some embodiments, the gene encoding IL-10 comprises a nucleotide sequence set forth in SEQ ID NO: 12.

In some embodiments, the genetically modified microorganism provided by the present disclosure is capable of producing IL-10 under inducing conditions (e.g., under conditions induced by micro environmental factors in inflammatory tissue). In some embodiments, the genetically modified microorganism is capable of producing IL-10 under hypoxic or anaerobic conditions. In some embodiments, the genetically modified microorganism is capable of secreting at least 300 ng, at least 350 ng, at least 400 ng, at least 450 ng, at least 500 ng, at least 550 ng, at least 600 ng, or at least 650 ng of IL-10 from 1 × 10⁹ CFU of the microorganism.
DNA sequence of IL-10 (SEQ ID NO: 10): 
agcccgggtcagggtacccaaagcgaaaacagctgcacccacttcccgggcaacctgccgaacatgctgcgtgacctgcgtgatgcgttcagc
cgtgtgaaaaccttcttccagatgaaagaccaactggataacctgctgctgaaggagagcctgctggaggacttcaaaggttacctgggctgccaggcgctgag
cgaaatgatccaattttatctggaggaagtgatgccgcaggcggagaaccaagacccggatattaaggcgcacgttaacagcctgggcgagaacctgaaaacc
ctgcgtctgcgtctgcgtcgttgccaccgtttcctgccgtgcgagaacaaaagcaaggcggtggaacaggttaagaacgcgtttaacaaactgcaagagaaggg
catctataaagcgatgagcgaattcgatatctttattaactacatcgaggcgtatatgaccatgaaaattcgtaactag.
Amino acid sequence of IL-10 (SEQ ID NO: 11): 
SPGQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENKSKAVEQVKNAF
NKLQEKGIYKAMSEFDIFINYIEAYMTMKIRN*.
DNA sequence of IL-10 (P2A) (SEQ ID NO: 12): 
agcgcgggtcagggtacccaaagcgaaaacagctgcacccacttcccgggcaacctgccgaacatgctgcgtgacctgcgtgatgcgttcagc
cgtgtgaaaaccttcttccagatgaaagaccaactggataacctgctgctgaaggagagcctgctggaggacttcaaaggttacctgggctgccaggcgctgag
cgaaatgatccaattttatctggaggaagtgatgccgcaggcggagaaccaagacccggatattaaggcgcacgttaacagcctgggcgagaacctgaaaacc
ctgcgtctgcgtctgcgtcgttgccaccgtttcctgccgtgcgagaacaaaagcaaggcggtggaacaggttaagaacgcgtttaacaaactgcaagagaaggg
 catctataaagcgatgagcgaattcgatatctttattaactacatcgaggcgtatatgaccatgaaaattcgtaact.
Amino acid sequence of IL-10 (P2A) (SEQ ID NO: 13):

SAGQGTQSENSCTHFPGNLPNMLRDLRDAFSRVKTFFQMKDQLDNLLLKESLLEDFKGYLG
CQALSEMIQFYLEEVMPQAENQDPDIKAHVNSLGENLKTLRLRLRRCHRFLPCENKSKAVEQVKNAF
NKLQEKGIYKAMSEFDIFINYIEAYMTMKIRN*.

### Interleukin-22 (IL-22)

As used herein, "interleukin-22" or "IL-22" is a T cell-secreted glycoprotein that upon discovery was named IL-10-related T cell-derived inducible factor (IL-TIF), and has become one of the most intensively studied IL-10 family members due to its unique functions.

IL-22 is mainly secreted by adaptive immune cells (CD4-positive T cells, CD8-positive T cells, etc.) and innate immune cells (LTi cells, NK cells, etc.). It has been found that numerous transcription factors, such as signal transduction and activator of transcription 3 (STAT3), retinoic acid-related orphan nuclear receptor γτ (PORγτ) and aryl hydrocarbon receptor (AhR), and cytokines such as IL-23, IL-6, IL-17, TNF-α and TNF-β, can all affect the expression of IL-22.

IL-22 exerts biological functions by binding to IL-22RI receptor and IL-10R2 receptor. Unlike other immune cytokines that mainly act on hematopoietic cells, IL-22 mainly exerts functions on non-blood-derived cells in tissues, such as endothelial cells, stromal cells and fibroblasts. In addition, IL-22 is widely distributed in many tissues, such as lung, liver, kidney, thymus, breast, intestine, skin and synovial membrane.

IL-22 participates in mucosal barrier maintenance and repair functions through inducing downstream proliferative and anti-apoptotic effects by activating the STAT signal transduction pathway. Although IL-22 can enhance the post-injury repair of epithelial cells and improve the survival of epithelial cells through anti-apoptosis and pro-proliferation, the sustained high expression of IL-22 can actually cause pathogenic effects such as tissue damage and chronic inflammation in certain diseases such as tumors and autoimmune diseases. In addition, IL-22 can also induce the production of antimicrobial peptides to resist microbial and parasitic invasion. It is precisely because of the extensive and complex protective and pathogenic functions of IL-22 that many studies in recent years have confirmed that IL-22 plays a unique role in diseases such as infections, autoimmune diseases and tumors.

In some embodiments, the genetically modified microorganism provided by the present disclosure expresses and secretes IL-22.

As used herein, the IL-22 includes the polypeptide of SEQ ID NO: 15 and functional equivalents thereof. As used herein, the term "IL-22" broadly encompasses IL-22 and IL-22 polynucleotides, such as DNA or RNA sequences encoding IL-22. As used herein, the term "IL-22" further encompasses wild-type IL-22 and functional equivalents that are functionally equivalent to wild-type IL-22. In the present application, the functional equivalent of the wild-type IL-22 refers to any IL-22 variant that at least partially retains one or more biological functions of wild-type IL-22 despite differences in their amino acid sequences or polynucleotide sequences or chemical structures. The one or more biological functions of wild-type IL-22 include, but are not limited to, the essential activity of binding IL-22R1/IL-10R2, and signaling through Janus kinase (associated with IL-22R subunit) and STAT molecules.

In some embodiments, wherein the IL-22 comprises a sequence set forth in SEQ ID NO: 15, or an amino acid sequence having at least 80% sequence identity with the sequence set forth in SEQ ID NO: 15 and still retaining the activity of binding to IL-22 receptor and regulating IL-22 receptor-expressing cells. In some embodiments, the IL-22 comprises an amino acid sequence having at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99 % sequence identity with the sequence set forth in SEQ ID NO: 15 and still retaining the activity of binding to IL-22 receptor and regulating IL-22 receptor-expressing cells.

In the present application, the variant of IL-22 encompasses all different forms of IL-22, including but not limited to a fragment, a mutant, a fusion, a derivative, a mimetic, or any combination thereof of IL-22.
DNA sequence of IL-22 (SEQ ID NO: 14): 
gcgccgatcagcagccactgccgtctggacaaaagcaacttccagcaaccgtatattaccaaccgtacctttatgctggcgaaagaggcgagcc
tggcggacaacaacaccgatgtgcgtctgatcggcgagaaactgttccacggcgttagcatgagcgaacgttgctacctgatgaagcaggtgctgaactttaccc
tggaggaagttctgttcccgcaaagcgatcgttttcagccgtatatgcaagaggtggttccgttcctggcgcgtctgagcaaccgtctgagcacctgccacatcga
aggtgacgatctgcacattcagcgtaacgtgcaaaaactgaaggacaccgttaaaaagctgggcgagagcggcgaaatcaaagcgattggcgaactggatctg
ctgtttatgagcctgcgtaacgcgtgcatttag.
Amino acid sequence of IL-22 (SEQ ID NO: 15):

APISSHCRLDKSNFQQPYITNRTFMLAKEASLADNNTDVRLIGEKLFHGVSMSERCYLMKQ
VLNFTLEEVLFPQSDRFQPYMQEVVPFLARLSNRLSTCHIEGDDLHIQRNVQKLKDTVKKLGESGEIKA
IGELDLLFMSLRNACI*.

### Interleukin-17A (IL-17A)

As used herein, "interleukin-17" or "IL-17A" is produced by activated T cells and mediates inflammatory responses in conjunction with IL-17R, which is ubiquitously expressed in all types of cells. However, the exact mechanism of IL-17A signaling remains incompletely elucidated. In chronic inflammation including RA, IL-17A can directly degrade matrix, or indirectly increase the number of activated inflammatory cells and induce other pro-inflammatory cytokines, including IL-1b and TNF-α, to enter the inflammatory tissue and cause tissue damage. IL-17A activates all three members of mitogen-activated protein kinases (MAPKs), namely extracellular signal-regulated kinases (ERK1 and ERK2) p44 and p42, Jun protein N-terminal kinase (JNK), and p38. IL-17A can induce the activation of NF-kb in human fibroblasts, intestinal epithelial cells and cultured chondrocytes, and this effect may be related to TNF receptor-associated factor (TRAF)-6. In the human monocytic leukemia cell line U937, IL-17A induces tyrosine phosphorylation of some members of the Janus kinase (JAN) and signal transducer and activator of transcription (STAT) pathways, including Tyk2 and JAK1, 2 and 3, STAT 1, 2, 3 and 4, suggesting that the JAK/STAT pathway may be involved in regulating the biological effects of IL-17A.
DNA sequence of IL-17A (SEQ ID NO: 217): 
ggtattacaatcccgcgtaatccgggttgtccgaatagtgaagataaaaattttccgcgtacagttatggttaatctgaatattcataaccgtaacaca
aacaccaatccgaaacgtagtagcgattattataatcgtagcacaagcccgtggaatctgcatcgtaacgaagatccggaacgttatccaagcgttatctgggaag
caaaatgccgccatctgggttgtatcaatgcagatggtaacgttgattatcatatgaatagcgtgccgattcagcaggaaattctggttctgcgtcgtgaaccgccgc
attgtccgaatagctttcgtctggaaaaaattctggttagcgttggttgtacctgtgttaccccgattgttcatcatgttgca.
Amino acid sequence of IL-17A (SEQ ID NO: 218):

GITIPRNPGCPNSEDKNFPRTVMVNLNIHNRNTNTNPKRSSDYYNRSTSPWNLHRNEDPERY
PSVIWEAKCRHLGCINADGNVDYHMNSVPIQQEILVLRREPPHCPNSFRLEKILVSVGCTCVTPIVHHVA

### Interleukin-19 (IL-19)

As used herein, "interleukin-19" or "IL-19" mainly plays a biological role by transmitting biological signals through the pair of receptor complexes IL-20R1/R2. IL-19 is a member of the cytokine IL-10 family, but the role of IL-19 is not yet clear. There is still controversy and uncertainty as to whether IL-19 is a pro-inflammatory or anti-inflammatory cytokine. Some studies believe that IL-19 may have the same anti-inflammatory effect as IL-10, and may induce the phenotype of lymphocytes to shift from Th1 to Th2. Some studies also believe that IL-19 upregulates the production of IL-6 and TNF-α in monocytes, showing that IL-19 has pro-inflammatory characteristics. Although the expression of IL-20R1 in immune cell populations was not detected, there are still many studies that clearly show the effects of IL-19 on these cells. Most studies indicate that IL-19 is part of the Th2 system. Studies have shown that IL-19 exerts anti-inflammatory effects in myocarditis. Endogenous IL-19 exhibits a protective effect in the inflammatory bowel. Some scholars established an IL-19 mouse DSS colitis model, which showed that these mice were more susceptible to colitis than those with intact immunity. In active Crohn's disease, defective IL-19 expression and lack of response to IL-19 favor the development of disease inflammation. In addition, adenovirus-mediated IL-19 fusion gene can effectively reduce damage and activate MAPK after being transferred into rats. It is further found that some cellular inflammatory factors can induce the production of IL-19. IL-19 can reduce the inflammatory response of vascular smooth muscle by reducing the stability of mRNA species encoding inflammatory proteins. Some *in vitro* experimental studies have shown that IL-19 can induce CD4+ T cells to produce Th2 cytokines and increase the production of IL-10 in peripheral blood mononuclear cells, and T cells exposed to IL-19 for a long time will downregulate IFN- γ and upregulate IL-4 and IL-13. However, some controversial studies found that IL-19 in monocytes upregulates the production of pro-inflammatory factors IL-6 and TNF-α, and the expression of IL-19 and its two receptor chains IL-20R1/IL-20R2 was found in the skin of patients with psoriasis. Further studies found that treatment of reducing IL-19 levels was effective.
DNA sequence of IL-19 (SEQ ID NO: 219): 
ctgcgtcgttgtctgattagcaccgatatgcatcatattgaagaaagctttcaggaaattaaacgtgccattcaggcaaaagatacctttccgaatgtt
accattctgagcaccctggaaaccctgcagattatcaaaccactggatgtttgttgtgttaccaaaaatctgctggccttttatgttgatcgtgtttttaaagatcatcag
gaaccgaatccgaaaattctgcgtaaaattagtagcattgccaatagctttctgtatatgcagaaaaccctgcgccagtgtcaggaacagcgtcagtgtcattgtcgt
caggaagcaaccaatgcaacccgtgtgattcatgataactatgatcagctggaagttcatgcagcagcgattaaaagcctgggtgaactggatgtgtttctggcat
ggattaataaaaatcatgaagtgatgtttagcgca.
Amino acid sequence of IL-19 (SEQ ID NO: 220):

LRRCLISTDMHHIEESFQEIKRAIQAKDTFPNVTILSTLETLQIIKPLDVCCVTKNLLAFYVDR
VFKDHQEPNPKILRKISSIANSFLYMQKTLRQCQEQRQCHCRQEATNATRVIHDNYDQLEVHAAAIKSL
GELDVFLAWINKNHEVMFSA.

### Interleukin-23 (IL-23)

As used herein, "interleukin-23" or "IL-23" is a heterodimeric cytokine composed of IL-12 p40 subunit and IL-23 p19 subunit, wherein the p40 subunit is shared between IL-23 and IL-12. The functional receptor for IL-23 has been identified consisting of IL-12R β1 and IL-23R. IL-23 plays a role in the immune response of type 1 polarized T cells. Although IL-12 can potently activate naive T cells, initial reports suggested that IL-23 can act preferentially on memory T cells, promoting their secretion of IFN-γ and proliferation, suggesting that IL-23 plays an important role in controlling bacterial infections. IL-23 is further described as a key cytokine in controlling inflammation in peripheral tissues. Overexpression of p19 is associated with inflammation in multiple organs and epithelial tissues including skin. In addition, IL-23 is also involved in inflammation in the central nervous system and various autoimmune diseases.
DNA sequence of IL-23 (IL-12 p40 subunit) (SEQ ID NO: 221): 
atctgggaactgaaaaaggatgtttatgtggttgaactggattggtatccggatgcaccgggcgaaatggtggttctgacgtgtgatacgccggaa
gaagatggtattacctggacactggatcagagcagcgaagttctgggcagtggtaaaaccctgacgattcaggttaaagaatttggtgatgcaggtcagtatacct
gtcataaaggtggtgaagttctgagccattccctgctgctgctgcataaaaaggaagatggtatctggagcaccgatattctgaaagatcagaaagaaccgaaaaa
caaaacctttctgcgttgcgaagcgaaaaattattcaggccgttttacctgttggtggctgacgaccattagcacagatctgacctttagcgtgaaaagcagccgtg
gtagcagcgatccgcagggtgttacttgtggtgcagcaacgctgtctgcagaacgtgttcgtggtgataataaagaatatgaatatagcgtggaatgccaggaag
attcagcatgtccggcagcagaagaaagcctgccgattgaagtgatggttgatgcagttcataaactgaaatatgaaaactataccagcagcttttttatccgtgatat
tattaaaccggaccctccgaaaaatctgcagctgaaacctctgaaaaatagtcgtcaggtggaagtgtcctgggaatatccggatacctggagcaccccacatag
ctattttagtctgaccttttgtgttcaggttcagggcaaatcaaaacgcgaaaaaaaagatcgtgtttttaccgataaaaccagcgcaaccgttatttgtcgtaaaaatg
caagcattagcgttcgtgcacaggatcgttattatagcagcagctggagcgaatgggcaagcgttccgtgtagc.
Amino acid sequence of IL-23 (IL-12 p40 subunit) (SEQ ID NO: 222): 
IWELKKDVYVVELDWYPDAPGEMVVLTCDTPEEDGITWTLDQSSEVLGSGKTLTIQVKEFG
DAGQYTCHKGGEVLSHSLLLLHKKEDGIWSTDILKDQKEPKNKTFLRCEAKNYSGRFTCWWLTTIST
 
DLTFSVKSSRGSSDPQGVTCGAATLSAERVRGDNKEYEYSVECQEDSACPAAEESLPIEVMVDAVHKL
KYENYTSSFFIRDIIKPDPPKNLQLKPLKNSRQVEVSWEYPDTWSTPHSYFSLTFCVQVQGKSKREKKD
RVFTDKTSATVICRKNASISVRAQDRYYSSSWSEWASVPCS.
DNA sequence of IL-23 (IL-23 p19 subunit) (SEQ ID NO: 223): 
cgtgcagttccgggtggtagcagcccggcatggacccagtgtcagcagctgagccagaaactgtgtaccctggcatggagcgcacatccgctg
gttggtcatatggatctgcgtgaagaaggtgatgaagaaaccaccaatgatgttccgcatattcagtgtggtgatggttgtgatccgcagggtctgcgtgataatag
ccagttttgtctgcagcgtattcatcagggtctgattttttatgaaaaactgctgggtagcgatatttttaccggtgaaccgagcctgctgccggatagcccggttggtc
agctgcatgcaagcctgctgggtctgagccagctgctgcagccggaaggtcatcattgggaaacccagcagattccgagcctgagcccgagccagccgtggc
agcgtctgctgctgcgttttaaaattctgcgtagcctgcaggcatttgttgcagttgcagcacgtgtttttgcacatggtgcagcaaccctgagcccg.
Amino acid sequence of IL-23 (IL-23 p19 subunit) (SEQ ID NO: 224):

RAVPGGSSPAWTQCQQLSQKLCTLAWSAHPLVGHMDLREEGDEETTNDVPHIQCGDGCDP
QGLRDNSQFCLQRIHQGLIFYEKLLGSDIFTGEPSLLPDSPVGQLHASLLGLSQLLQPEGHHWETQQIPS
LSPSQPWQRLLLRFKILRSLQAFVAVAARVFAHGAATLSP.

### Interleukin-35 (IL-35)

As used herein, "interleukin-35" or "IL-35" is a heterologous dimer composed of Epstein-Barr virus-induced gene 3 (EBI3) protein and IL-12 p35 (IL-12A) subunit, which together with IL-12, IL-23 and IL-27, constitutes the IL-12 cytokine family, and plays an important regulatory role in the proliferation and activation of T cells and the production of cytokines. Studies have confirmed that IL-35 is mainly secreted by regulatory T cells (Treg) and is one of the main cytokines on which Treg exerts negative immune regulation. IL-35 is involved in the immune regulation of inflammation in various diseases such as experimental colitis, collagen-induced arthritis, autoimmune demyelinitis, chronic hepatitis, diabetes and tumors, and is closely related to the occurrence and development of diseases.
DNA sequence of IL-35 (EBI3 protein) (SEQ ID NO: 225): 
cgcaaaggtccgccggcagcactgaccctgccgcgtgttcagtgtcgtgcatcccgctatccgattgcagttgattgtagttggaccctgccgcc
ggcaccgaattccacctcaccggttagctttattgctacctatcgtctgggtatggccgcacgtggtcattcatggccttgtctgcagcagaccccgaccagcacga
gctgtaccatcaccgatgttcagctgttttctatggcaccgtatgttctgaatgttacggcagttcatccgtggggttcaagcagctcttttgttccatttattaccgaaca
tatcatcaaaccggaccctccggaaggtgttcgtctgagcccgctggcagaacgtcagctgcaggttcagtgggaaccgccaggttcatggccgtttcctgaaatt
tttagcctgaaatattggattcgttataaacgtcagggtgcggcccgttttcatcgtgttggtcctattgaagcaacctcatttattctgcgtgcggtccgtcctcgtgcc
cgttattatgttcaggtcgccgcacaggatctgaccgattatggtgaactgagcgattggtcactgccggcaaccgcgaccatgtccctgggtaaa.
Amino acid sequence of IL-35 (EBI3 protein) (SEQ ID NO: 226): 
RKGPPAALTLPRVQCRASRYPIAVDCSWTLPPAPNSTSPVSFIATYRLGMAARGHSWPCLQQT
PTSTSCTITDVQLFSMAPYVLNVTAVHPWGSSSSFVPFITEHIIKPDPPEGVRLSPLAERQLQVQWEPPGS
WPFPEIFSLKYWIRYKRQGAARFHRVGPIEATSFILRAVRPRARYYVQVAAQDLTDYGELSDWSLPATAT
MSLGK.
DNA sequence of IL-35 (IL-12 p35 subunit) (SEQ ID NO: 227): 
cgtaacctgccggttgcgaccccggaccctggcatgtttccgtgtctgcatcattctcagaatctgctgcgtgcagtctctaatatgctgcagaaag
cacgtcagaccctggaattttatccgtgtaccagtgaagaaatcgatcatgaagatattaccaaagataaaaccagcaccgtggaagcatgtctgccgctggaact
gaccaaaaacgaaagctgtctgaacagccgcgaaaccagttttattacaaatggtagttgtctggcaagccgcaaaaccagctttatgatggcgctgtgtctgagc
tcaatttatgaagatctgaaaatgtatcaggtggaatttaaaaccatgaatgccaaactgctgatggaccctaaacgtcagatttttctggatcagaatatgctggcag
ttatcgatgaactgatgcaggcactgaactttaatagcgaaaccgttccgcagaaaagctccctggaagaaccggatttttataaaaccaaaatcaaactgtgcatc
ctgctgcatgcatttcgtattcgtgcagttaccattgatcgtgtgatgagctatctgaatgcaagc.
Amino acid sequence of IL-35 (IL-12 p35 subunit) (SEQ ID NO: 228):

RNLPVATPDPGMFPCLHHSQNLLRAVSNMLQKARQTLEFYPCTSEEIDHEDITKDKTSTVEA
CLPLELTKNESCLNSRETSFITNGSCLASRKTSFMMALCLSSIYEDLKMYQVEFKTMNAKLLMDPKRQI

FLDQNMLAVIDELMQALNFNSETVPQKSSLEEPDFYKTKIKLCILLHAFRIRAVTIDRVMSYLNAS.

### Interleukin-37 (IL-37)

As used herein, "interleukin-37" or "IL-37" has five different subtypes (IL-37a-e). Current research shows that only IL-37b has biological functions and has anti-inflammatory effects in a variety of diseases. IL-37 can be secreted extracellularly as a cytokine that binds to receptors to regulate cell activity, or can enter the nucleus and serve as a transcriptional regulator. IL-37 has high homology with IL-18, and can bind to IL-18 binding protein (IL-18BP) to have a certain inhibitory effect on the IL-18 signaling pathway.
DNA sequence of IL-37 (SEQ ID NO: 229): 
gttcataccagtccgaaagtgaaaaatctgaatccgaaaaagtttagcatccatgatcaggatcataaagttctggtactggatagcggtaatctgat
tgcagtgccggataaaaattatattcgtccggaaattttttttgccctggcaagcagcctgagttcagcctcagcggaaaaaggtagcccgattctgctgggtgttag
taaaggtgaattttgtctgtattgtgataaagataaaggccagagccatccgagtctgcagctgaaaaaggaaaaactgatgaaactggcagcacagaaagaaag
cgctcgtcgtccgtttattttttatcgtgcacaggtgggtagctggaatatgctggaaagcgcagcccatccaggttggtttatttgcaccagctgcaattgtaatgaa
ccggtgggtgttaccgataaatttgaaaatcgtaaacatatcgaatttagctttcagccggtttgtaaagcagaaatgagcccgagcgaagttagtgat.
Amino acid sequence of IL-37 (SEQ ID NO: 230):

VHTSPKVKNLNPKKFSIHDQDHKVLVLDSGNLIAVPDKNYIRPEIFFALASSLSSASAEKGSPI
LLGVSKGEFCLYCDKDKGQSHPSLQLKKEKLMKLAAQKESARRPFIFYRAQVGSWNMLESAAHPGW
FICTSCNCNEPVGVTDKFENRKHIEFSFQPVCKAEMSPSEVSD.

### TcF-β

As used herein, "transforming growth factor β" or "TGF-β" is a multifunctional cytokine that can be produced and secreted by numerous types of cells in the human body. TGF-β is involved in the mechanisms of many diseases, playing dual roles in inhibiting or promoting diseases, including wound healing, tissue fibrosis, atherosclerosis, cancer occurrence and metastasis, autoimmune diseases, diabetic complications, and nerve damage caused by Alzheimer's disease, which are all closely related to TGF-β. There are two immune responses in the human body, namely type 1 helper T cell (TH1)-mediated normal immune defense response, and type 2 helper T cell (TH2)-mediated allergic immune defense response. The TH1-mediated normal immune defense response is responsible for the immune defense mechanism of infectious microorganisms. If the function of TH1 cells is excessively strong, it will cause the secretion of cytokines that cause inflammatory reactions, such as interleukin-2 (IL-2) and interferon-y (IFN-γ), and increase the cellular immune response, attacking specific tissues or special cells of the human body, causing long-term damage to certain tissues or organs of the human body, especially autoimmune diseases and organ transplant rejection. The TH2-mediated allergic immune defense response is responsible for the immune defense mechanism of parasites, biting insects, allergens and irritants to barrier organs. If the function of TH2 cells is excessively strong, the secretion of cytokines of TH2 cell will be too high, prompting B cells to produce a large amount of allergic antibodies IgE. IgE will induce mast cells or basophils to release inflammatory substances, such as histamine, interleukins, cytokines and platelet activating factors, which act on cells or blood vessels, causing vasodilation and smooth muscle contraction, leading to allergic symptoms such as allergic asthma, allergic rhinitis and atopic dermatitis. These two types of immunity are balanced in a scale-like manner in the human body. TH1 and TH2 balance with each other and are jointly regulated by regulatory cells (Treg) and immune regulatory factors (TGF-β). Maintaining a balance between systemic TH1 and TH2 in the body's immune defense system can prevent autoimmune diseases and allergy-related diseases. In common hereditary allergic diseases, such as atopic dermatitis, asthma and allergic rhinitis, the TGF-β immune regulatory factors are all lacked in the body. Studies have shown that long-term supplementation of TGF-β can increase the concentration thereof in the body, improve physical fitness, reduce inflammation, reduce allergy index, repair tissues, extend the protection of breast milk for infants and young children, improve the body's food tolerance, and maintain the function of digestive tract.
DNA sequence of TGF-β (SEQ ID NO: 231): 
gcactggataccaattattgtttttcaagcaccgaaaaaaattgttgtgttcgtcagctgtatattgattttcgtaaagatctgggttggaaatggattcat
gaaccgaaaggttatcatgcaaatttttgtctgggtccgtgtccgtatatttggagcctggatacccagtatagcaaagttctggcactgtataatcagcataatccgg
gtgcaagcgcagcaccgtgttgtgttccgcaggcactggaaccgctgccgattgtttattatgttggtcgtaaaccgaaagttgaacagctgagcaatatgattgttc
gtagctgtaaatgtagc.
Amino acid sequence of TGF-β (SEQ ID NO: 232):

ALDTNYCFSSTEKNCCVRQLYIDFRKDLGWKWIHEPKGYHANFCLGPCPYIWSLDTQYSKV
LALYNQHNPGASAAPCCVPQALEPLPIVYYVGRKPKVEQLSNMIVRSCKCS.

### C. Expression cassette

As used herein, the term "expression cassette" refers to a DNA sequence capable of directing the expression of a specific nucleotide sequence in an appropriate microorganism, comprising a promoter operably linked to the nucleotide sequence of interest, and the nucleotide sequence is operably linked to a termination signal. It usually further comprises sequences required for proper translation of the nucleotide sequence. The coding region typically encodes the protein of interest, but may also encode the functional RNA of interest in the antisense orientation, such as antisense RNA or non-translating RNA. An expression cassette comprising the nucleotide sequence of interest may be chimeric, meaning that at least one of its components is heterologous with respect to at least one of its other components.

As used herein, the term "recombinant" refers to a polynucleotide synthesized or otherwise manipulated *in vitro* (e.g., a "recombinant expression cassette"), or refers to a method of producing a product in a cell or other biological system using a recombinant polynucleotide or a recombinant expression cassette, or refers to a polypeptide encoded by a recombinant polynucleotide. A "recombinant expression cassette" encompasses nucleic acid molecules from different sources that are joined into an expression cassette or vector to express, for example, a fusion protein; or a protein produced by inducible or constitutive expression of polypeptides. A recombinant expression cassette encompasses a recombinant polynucleotide operably linked to one or more regulatory elements.

In some embodiments, in the genetically modified microorganism described herein, the at least two exogenous genes are included in (at least one) exogenous expression cassette.

In some embodiments, the genetically modified microorganism provided by the present application comprises at least one, at least two, or all three exogenous expression cassettes selected from the group consisting of: a first exogenous expression cassette, a second exogenous expression cassette and a third exogenous expression cassette. The first exogenous expression cassette, also referred to as "Amuc_1100 expression cassette", "exogenous expression cassette A" or "expression cassette A" which can be used interchangeably in the present disclosure and have the same meaning, comprises a nucleotide sequence encoding Amuc_1100 polypeptide. The second exogenous expression cassette, also referred to as "IL-10 expression cassette", "exogenous expression cassette B" or "expression cassette B" which may be used interchangeably in the present disclosure and have the same meaning, comprises a nucleotide sequence encoding IL-10. The third exogenous expression cassette, also referred to as an "IL-22 expression cassette", "exogenous expression cassette C" or "expression cassette C" which are used interchangeably in the present disclosure and have the same meaning, comprises a nucleotide sequence encoding IL-22.

In some embodiments, the at least one exogenous expression cassette comprises one or more regulatory elements (or "expression regulatory elements", which have the same meaning herein) operably linked to the exogenous gene.

In some embodiments, the regulatory element comprises one or more elements selected from the group consisting of a promoter, a ribosome binding site (RBS), a cistron, a terminator, and any combination thereof.

### i. Promoter

As used herein, the term "promoter" refers to a non-translating DNA sequence containing a binding site for RNA polymerase and initiating DNA transcription, usually upstream of a coding region. The promoter region may also contain other elements that act as regulators of gene expression. In some embodiments, the promoter is suitable for initiating a polynucleotide encoding Amuc_1100 polypeptide, IL-10, and/or IL-22 in the genetically modified microorganism. In some embodiments, the promoter is a constitutive promoter or an inducible promoter.

The term "constitutive promoter" refers to a promoter capable of promoting continuous transcription of a coding sequence or gene under its control and/or operably linked thereto. Constitutive promoters and variants thereof are well known in the art and include, but are not limited to, BBa_J23119, BBa_J23101, BBa_J23102, BBa_J23103, BBa_J23109, BBa_J23110, BBa_J23114, BBa_J23117, USP45_promoter, Amuc_1102_promoter, OmpA_ promoter, BBa_J23100, BBa_J23104, BBa_J23105, BBa_I14018, BBa_J45992, BBa_J23118, BBa_J23116, BBa_J23115, BBa_J23113, BBa_J23112, BBa_J23111, BBa_J23108, BBa_J23107, BBa_J23106, BBa_I14033, BBa_K256002, BBa_K1330002, BBa_J44002, BBa_J23150, BBa_I14034, Oxb19, oxb20, BBa_K088007, Ptet, Ptrc, PlacUV5, BBa_K292001, BBa_K292000, BBa_K137031 and BBa_K137029. An exemplary constitutive promoter comprises a nucleotide sequence selected from the group consisting of: SEQ ID NOs: 16-56 as shown in Table 3, and homologous sequences having at least 80% (e.g., at least 85 %, 90%, 91%, 92%, 93%, 94%, 95%, 96%, 97%, 98%, 99%) sequence identity with SEQ ID NOs: 16-56. In some embodiments, the constitutive promoter comprises a nucleotide sequence set forth in any of SEQ ID NOs: 16-56. In some embodiments, the promoter is active *in vitro,* e.g., under culture, amplification and/or manufacturing conditions. In some embodiments, the promoter is active *in vivo,* e.g., under conditions in an *in vivo* environment, such as the intestinal and/or inflammatory microenvironment. Exemplary nucleotide sequences of constitutive promoters are shown in Table 3.

**Table 3 Constitutive promoters**

| Constitutive promoter | Sequence | SEQ ID NO: |
|---|---|---|
| BBa_J23119 | ttgacagctagctcagtcctaggtataatgctagc | 16 |
| BBa_J23101 | tttacagctagctcagtcctaggtattatgctagc | 17 |
| BBa_J23102 | ttgacagctagctcagtcctaggtactgtgctagc | 18 |
| BBa_J23103 | ctgatagctagctcagtcctagggattatgctagc | 19 |
| BBa_J23109 | tttacagctagctcagtcctagggactgtgctagc | 20 |
| BBa_J23110 | tttacggctagctcagtcctaggtacaatgctagc | 21 |
| BBa_J23114 | tttatggctagctcagtcctaggtacaatgctagc | 22 |
| BBa_J23117 | ttgacagctagctcagtcctagggattgtgctagc | 23 |
| USP45_promoter | aaagtgttttgtaatcataaagaaatattaaggtggggtaggaatagtataatatgtttatt caaccgaacttaatg | 24 |
| Amuc_1102_prom oter | gggccgagcatgtggaaacacctctgccaggtcaagcccgcagccatgccgcatgtt tttttcttgaccgcccgggcagaaccgtcgggcggagccgtgtcatgctccacacac gctacatggaaagaaacaagagatttttgctagccacagttcggtttttggtgtaactaa tc | 25 |
| OmpA_promoter | gtaaatttaggattaatcctggaactttttttgtcgcccagccaatgctttcagtcgtgact aattttccttgcggaggcttgtctgaagcggtttccgcgattttcttctgtaaattgtcgct gacaaaaaagattaaacgtaccttatacaagacttttttttcatatgcctgacggagttca cacttgtaagttttcaactacgttgtagactttacatcgccaggggtgctcggcataagc cgaagatatcggtagagttaatattgagcagatccccggtgaaggatttaaccgtgtta tctcgttggagatattcatggcgtattttggatga | 26 |
| BBa_J23100 | ttgacggctagctcagtcctaggtacagtgctagc | 27 |
| BBa_J23104 | ttgacagctagctcagtcctaggtattgtgctagc | 28 |
| BBa_J23105 | tttacggctagctcagtcctaggtactatgctagc | 29 |
| BBa_J23106 | tttacggctagctcagtcctaggtatagtgctagc | 30 |
| BBa_J23107 | tttacggctagctcagccctaggtattatgctagc | 31 |
| BBa J23108 | ctgacagctagctcagtcctaggtataatgctagc | 32 |
| BBa_J23111 | ttgacggctagctcagtcctaggtatagtgctagc | 33 |
| BBa_J23112 | ctgatagctagctcagtcctagggattatgctagc | 34 |
| BBa_J23113 | ctgatggctagctcagtcctagggattatgctagc | 35 |
| BBa_J23115 | tttatagctagctcagcccttggtacaatgctagc | 36 |
| BBa_J23116 | ttgacagctagctcagtcctagggactatgctagc | 37 |
| BBa_J23118 | ttgacggctagctcagtcctaggtattgtgctagc | 38 |
| BBa_J45992 | ggtttcaaaattgtgatctatatttaacaa | 39 |
| BBa_I14018 | gtttatacataggcgagtactctgttatgg | 40 |
| BBa_I14033 | agaggttccaactttcaccataatgaaaca | 41 |
| BBa_I14034 | taaacaactaacggacaattctacctaaca | 42 |
| BBa_J23150 | ggctagctcagtcctaggtattatgctagc | 43 |
| BBa_J44002 | aaagtgtgacgccgtgcaaataatcaatgt | 44 |
| BBa_K1330002 | ggctagctcagtcctaggtactatgctagc | 45 |
| BBa_K256002 | caccttcgggtgggcctttctgcgtttata | 46 |
| Oxb19 | aagctgttgtgaccgcttgctctagccagctatcgagttgtgaaccgatccatctagca attggtctcgatctagcgataggcttcgatctagctatgtatcactcattaggcacccca ggctttacactttatgcttccggctcgtataatgtgtggtgctggttagcgcttgctat | 47 |
| oxb20 | aagctgttgtgaccgcttgctctagccagctatcgagttgtgaaccgatccatctagca attggtctcgatctagcgataggcttcgatctagctatgtagaaacgccgtgtgctcga tcgcttgataaggtccacgtagctgctataattgcttcaacagaacatattgactatccg gtattacccggc | 48 |
| BBa_K088007 | catacgccgttatacgttgtttacgctttg | 49 |
| BBa_K137029 | atatatatatatatataatggaagcgtttt | 50 |
| BBa_K137031 | ccccgaaagcttaagaatataattgtaagc | 51 |
| BBa_K292000 | ggctagctcagtcctaggtacagtgctagc | 52 |
| BBa_K292001 | tgctagctactagagattaaagaggagaaa | 53 |
| PlacUV5 | cccaggctttacactttatgcttccggctcgtataatgtgtggaattgtgag | 54 |
| Ptrc | ttgacaattaatcatccggctcgtataatgtgtggaattgtgag | 55 |
| Ptet | taattcctaatttttgttgacactctatcgttgatagagttattttaccactccctatcagtga tagagaaaa | 56 |

As used herein, the term "inducible promoter" refers to a regulated promoter that can be activated in one or more types of cells by external stimuli, such as chemicals, light, hormones, stress, or pathogens. Inducible promoters and variants thereof are well known in the art and include, but are not limited to, PLteto1, galP1, PLlacO1, Pfnrs, Psal and Pvan. In some embodiments, an exemplary inducible promoter comprising a nucleotide sequence selected from the group consisting of: SEQ ID NOs: 57-61 as shown in Table 4, and homologous sequences having at least with 80% (e.g., at least 85%, 90%, 91%, 92%, 93%, 94%, 95%, 96%, 97%, 98%, 99%) sequence identity with SEQ ID NOs: 57-61.

**Table 4 Inducible promoters**

| Inducible promoter | Sequence | SEQ ID NO: |
|---|---|---|
| PLteto1 | tccctatcagtgatagagattgacatccctatcagtgatagagatactgagcacatcagcag gacgcactgacc | 57 |
| galP1 | attccactaatttattccatgtcacacttttcgcatctttgttatgctatggttatttcataccataa | 58 |
| PLlacO1 | ataaatgtgagcggataacaattgacattgtgagcggataacaagatactgagcacatcag caggacgcactgacc | 59 |
| Pfnrs | aaaaacgccgcaaagtttgagcgaagtcaataaactctctacccattcagggcaatatctct ctt | 60 |
| PfdhF | tgaataaatagtgcccgtaatatcagggaatgaccccacataaaatgtggcataaaagatg | 61 |
| | catactgtagtcgagagcgcgtatgcgtgatttgatta | |
| Psal | ggggcctcgcttgggttattgctggtgcccggccgggcgcaatattcatgttgatgatttatt atatatcgagtggtgtatttatttatattgtttgctccgttaccgttattaacag | 233 |
| Pvan | attggatccaattgacagctagctcagtcctaggtaccattggatccaatag | 234 |

In some embodiments, the promoter is an endogenous promoter or an exogenous promoter. An exogenous promoter refers to a promoter that is operably combined with a coding region, wherein the promoter is not a promoter naturally associated with the coding region in the genome of the organism. A promoter in the genome that is naturally associated or linked to a coding region is called an endogenous promoter of the coding region. In some embodiments, the promoter is operably connected upstream or downstream of the transcription start site of the gene of interest.

In some embodiments, the promoter is selected from the group consisting of BBa_J23101, BBa_J23108, BBa_J23110, PfnrS, Psal, Pvan, BBa_J23119, BBa_J23102 and BBa_J23114.

### ii. Ribosome binding site

As used herein, the term "ribosome binding site" ("RBS") refers to a sequence on an mRNA to which ribosomes bind to initiate protein translation. A RBS is approximately 35 nucleotides in length and contains three discrete domains: (1) a Shine-Dalgarno (SD) sequence, (2) a spacer, and (3) the first five to six codons of the coding sequence (CDS). RBS and variants thereof are well known in the art and include, but are not limited to, USP45, synthesized RBS, Amuc_1102 and OmpA. An exemplary RBS comprises a nucleotide sequence selected from the group consisting of: SEQ ID NOs: 62-65 as shown in Table 5, and homologous sequences having at least 80% (e.g., at least 85%, 90 %, 91%, 92%, 93%, 94%, 95%, 96%, 97%, 98%, 99%) sequence identity with SEQ ID NOs: 62-65. In certain embodiments, the ribosome binding site is operably linked to 8-13 nucleotides upstream of the coding region of the gene of interest.

**Table 5 Ribosome binding site (RBS)**

| RBS | Sequence | SEQ ID NO: |
|---|---|---|
| USP45 | ggaggaaaaattanaaaagaac | 62 |
| Synthesized | aaagaggagaaa | 63 |
| Amuc 1102 | agggaa | 64 |
| OmpA | taacgagg | 65 |

### iii. Cistronic

As used herein, the term "cistron" refers to a fragment of a nucleic acid sequence that is transcribed and encodes a polypeptide. Cistrons and variants thereof are well known in the art and include, but are not limited to, GFP, BCD2, luciferase and MBP. An exemplary cistron comprises a nucleotide sequence selected from the group consisting of: SEQ ID NOs: 66-75 as shown in Table 6 and homologous sequences having at least 80% (e.g., at least 85% , 90%, 91%, 92%, 93%, 94%, 95%, 96%, 97%, 98%, 99%) sequence identity with SEQ ID NOs: 66-75. In certain embodiments, the cistron is operably linked to the N-terminus of the coding region of the gene of interest.

**Table 6 Cistrons**

| Cistron | | Sequence | SEQ ID NO: |
|---|---|---|---|
| T7g10 | Amino acid sequence | LSL | 66 |
| | DNA sequence | ttaagttta | 67 |
| GFP | Amino acid sequence | MRKGEEKEVN | 68 |
| | DNA sequence | atgcgtaaaggcgaagagaaggaggttaactga | 69 |
| BCD2 | Amino acid sequence | MKAIFVLKHLNHAKEVF | 70 |
| | DNA sequence | atgaaagcaattttcgtactgaaacatcttaatcatgctaagga ggttttctaa | 71 |
| Luciferase | Amino acid sequence | MIMSGYKEVN | 72 |
| | DNA sequence | atgattatgtccggttataaggagttaactga | 73 |
| MBP | Amino acid sequence | MKIEAGKLVQKEVN | 74 |
| | DNA sequence | atgaaaatcgaagcaggtaaactggtacagaaggaggttaa ctga | 75 |

### iv. Terminator

As used herein, the term "terminator" refers to a nucleotide sequence that provides a termination signal of RNA polymerase transcription, which prevents subsequent incorporation of a nucleotide into the resulting polynucleotide chain, and thereby interrupts polymerase-mediated elongation. In some embodiments, the terminator used in the present disclosure is T7 terminator. In some embodiments, the terminator comprises a nucleotide sequence selected from the group consisting of: SEQ ID NOs: 76-79 as shown in Table 7 and homologous sequences having at least 80% (e.g., at least 85%, 90 %, 91%, 92%, 93%, 94%, 95%, 96%, 97%, 98%, 99%) sequence identity with SEQ ID NOs: 76-79. In certain embodiments, the terminator is operably linked to the 3' end of the coding gene. In some embodiments, the terminator used in the present disclosure is rrnB_T1_T7Te_terminator, which comprises a sequence obtained by concatenating rrnB_T1_terminator and T7Te_terminator, and the specific sequence is as follows: caaataaaacgaaaggctcagtcgaaagactgggcctttcgttttatctgttgtttgtcggtgaacgctctctactagagtcacactggctcaccttcgggtgggcctt tctgcg (SEQ ID NO: 242).

**Table 7 Transcription terminator**

| Transcription terminator | Sequence | SEQ ID NO: |
|---|---|---|
| rrnB_T1_terminator | caaataaaacgaaaggctcagtcgaaagactgggcctttcgttttatctgttgttt gtcggtgaacgctctc | 76 |
| rrnB_T1(mutant)_ terminator | caaataaaacgaaagactgggcctttcgttttatctgttgtttgtcggtgaacgct ctc | 77 |
| T7Te_terminator | ggctcaccttcgggtgggcctttctgcg | 78 |
| LPP_terminator | tagtacctgtgaagtgaaaaatggcgcacattgtgcgccattttttttgtctgccg tttaccgcta | 79 |

### D. Secretion systems and/or export pathways in microorganisms

In some embodiments, in the exogenous expression cassette provided herein, the polynucleotide sequence encoding the Amuc_1100 polypeptide, IL-10, and/or IL-22 is operably linked to a signal peptide.

As used herein, the term "signal peptide" or "signal sequence" refers to a peptide that can be used to secrete a heterologous polypeptide into the periplasm or culture medium of cultured bacteria or to secrete an Amuc_1100 polypeptide, IL-10 and/or IL-22 into the periplasm. The signal peptide of a heterologous polypeptide may be homologous or heterologous to the bacteria, comprising a signal native to the polypeptide produced in the bacteria. For Amuc_1100, IL-10 and/or IL-22, the signal sequence is typically the endogenous signal sequence of the bacterial cell, but it does not have to be an endogenous signal sequence as long as it is effective for its purpose. Non-limiting examples of secretion peptides include USP45, OppA (ECOLIN_07295), OmpA, OmpF, cvaC, TorA, fdnG, dmsA, PelB, HlyA, adhesin (ECOLIN_19880), DsbA (ECOLIN 21525), Gltl (ECOLIN 03430), GspD ( ECOLIN 16495), HdeB (ECOLIN_19410), MalE (ECOLIN_22540), PhoA (ECOLIN 02255), PpiA (ECOLIN_18620), TolB, tort, mglB and lamB. In some embodiments, the secretion peptide is USP45.

In some embodiments, the signal peptide for secreting Amuc_1100, IL-10 and/or IL-22 provided herein is selected from the group consisting of USP45, OmpA, DsbA, pelB, Cel-CD, sat and endogenous signal peptides (or "own signal peptide", "endogenous signal peptide" and "own signal peptide" have the same meaning herein, both representing the original or naturally occurring signal peptide sequence in the wild-type polypeptide sequence) of Amuc_1100, IL-10 and/or IL-22. Exemplary nucleotide sequences of signal peptides include a nucleotide sequence selected from the group consisting of: SEQ ID NOs: 123-135 as shown in Table 9 and homologous sequences having at least 80% (e.g., at least 85%, 90%, 91%, 92%, 93%, 94%, 95%, 96%, 97%, 98%, 99%) sequence identity therewith. In some embodiments, the signal peptide comprises an amino acid sequence selected from the group consisting of: SEQ ID NOs: 80-122 as shown in Table 8 and homologous sequences having at least 80% (e.g., at least 85%, 90%, 91%, 92%, 93%, 94%, 95%, 96%, 97%, 98%, 99%) sequence identity therewith. In some embodiments, the signal peptide is operably linked to the N-terminus of the expressed target protein. In some embodiments, the own signal peptide sequence of the target protein (e.g., Amuc_1100, IL-10, and/or IL-22) is removed, and the N-terminus of the target protein is connected to a selected signal peptide (such as the first signal peptide, the second signal peptide or the third signal peptide as described herein, such as the USP45 signal peptide), i.e., the own signal peptide is replaced with the selected signal peptide, for protein secretion.

In some embodiments, the expression cassette comprises a nucleotide sequence encoding Amuc_1100, IL-10, and/or IL-22 operably linked to a signal peptide and an autotransporter domain, wherein the signal peptide and autotransporter domain are operably linked to the opposite ends of Amuc_1100, IL-10 and/or IL-22, for example, the signal peptide is operably linked to the N-terminus of Amuc_1100, IL-10 and/or IL-22, and the autotransporter domain is operably linked to the C-terminus of Amuc_1100, IL-10 and/or IL-22. The constructs can be used for type V autocrine-mediated secretion, wherein the signal peptide at N-terminus is removed upon translocation of the precursor protein from the cytoplasm into the periplasmic compartment by a native secretion system, such as the Sec system. In addition, once the autocrine is translocated across the outer membrane, the autotransporter domain at C-terminus can be removed by autocatalytic or protease-catalyzed, for example, cleavage of OmpT, thereby releasing the mature protein (e.g., Amuc_1100 polypeptide, IL-10, and/or IL-22) into the extracellular environment.

**Table 8 Amino acid sequences of signal peptides**

| Signal peptide | Amino acid sequence | SEQ ID NO: |
|---|---|---|
| Amuc_1100 | MSNWITDNKPAAMVAGVGLLLFLGLSATGY | 80 |
| AnsB | MEFFKKTALAALVMGFSGAALA | 81 |
| Bla | MSIQHFRVALIPFFAAFCLPVFA | 82 |
| Caf1M | MILNRLSTLGIITFGMLSFAPGPPPGPPRVS | 83 |
| Cel-CD | EGNTREDNFKHLLGNDNVKR | 84 |
| Cex | MKKAKAIFLFILIVSGFLLVA | 85 |
| DsbA | MKKIWLALAGLVLAFSASA | 86 |
| EltB | MNKVKFYVLFTALLSPLCAHG | 87 |
| FkpA | MKSLFKVTLLATTMAVALHAPITFA | 88 |
| LamB | MMITLRKLPLAVAVAAGVMSAQAMA | 89 |
| Lpp | MKATKLVLGAVILGSTLLAG | 90 |
| LTB | MNKVKCYVLFTALLSSLYAHG | 91 |
| MalE | MKIKTGARILALSALTTMMFSASALA | 92 |
| MglB | MNKKVLTLSAVMASMLFGAAAHA | 93 |
| Npr | MGLGKKLSVAVAASFMSLTISLPGVQA | 94 |
| OmpA | MKKTAIAIAVALAGFATVAQA | 95 |
| OmpC | MKVKVLSLLVPALLVAGAANA | 96 |
| OmpF | MMKRNILAVIVPALLVAGTANA | 97 |
| OmpT | MRAKLLGIVLTTPIAISPFA | 98 |
| OsmY | MTMTRLKISKTLLAVMLTSAVATGSAYA | 99 |
| PelB | MKYLLPTAAAGLLLLAAQPAMA | 100 |
| PgaA | MYSSSRKRCPKTKWALKLLTAAFLAA | 101 |
| PgaB | MLRNGNKYLLMLVSIIMLTA | 102 |
| PhoA | MKQSTIALALLPLLFTPVTKA | 103 |
| PhoE | MKKSTLALVVMGIVASASVQA | 104 |
| Sat | MNKIYSLKYSAATGGLIAVSELAKRVSGKTNRKLVATMLSLAVA GTVNA | 105 |
| SfmC | MMTKIKLLMLIIFYLIISASAHA | 106 |
| Skp | MKKWLLAAGLGLALATSAQA | 107 |
| Sta1 | MKKLMLAIFISVLSFPSFS | 108 |
| StiI | MKKNIAFLLASMFVFSIATNAYA | 109 |
| SurA | MKNWKTLLLGIAMIANTSFA | 110 |
| TolB | MKQALRVAFGFLILWASVLHA | 111 |
| TorA | MNNNDLFQASRRRFLAQLGGLTVAGMLGPSLLTPRRATA | 112 |
| TorT | MRVLLFLLLSLFMLPAFS | 113 |
| USP45 | MKKKIISAILMSTVILSAAAPLSGVYA | 114 |
| XynS | MFKFKKKFLVGLTAAFMSISMFSATASA | 115 |
| YebF | MKKRGAFLGLLLVSACASVFA | 116 |
| csgA_N22 | MKLLKVAAIAAIVFSGSALAGVVPQYGGGGNHGGGGNNSGP N | 117 |
| FlgI_N22 | VIKFLSALILLLVTTAAQAGVVPQYGGGGGNHGGGNNSGPN | 118 |
| sfmC_N22 | MMTKIKLLMLIIFYLIISASAHAGVVPQYGGGGNHGGGGNNS GPN | 119 |
| USP45_N22 | MKKIISAILMSTVILSAAAPLSGVYAGVVPQYGGGGGNHGGG GNNSGPN | 120 |
| YraI_N22 | MSKRTFAVILTLLCSFCIGQALA GVVPQYGGGGNHGGGGNNSGPN | 121 |
| csgA | MKLLKVAAIAAIVFSGSALAGVVPQYGGGGNHGGGGNNSGP NSELNIYQYGGGNSALALQADARNSDLTITQHGGGNGADVGQ GSDDSSIDLTQRGFGNSATLDQWNGKDSTMTVKQFGGGNGAA VDQTASNSSVNVTQVGFGNNATAHQY | 122 |

**Table 9 DNA sequences of some signal peptides**

| Signal peptide | DNA sequence | SEQ ID NO: |
|---|---|---|
| Amuc_1100 | atgagcaactggatcaccgacaacaaaccggctgcgatggttgcgggtgtggg cctgctgctgttcctgggtctgagcgcgaccggctac | 123 |
| Cel-CD | gaaggaaacactcgtgaagacaattttaaacatttattaggtaatgacaatgttaaa cgc | 124 |
| DsbA | atgaagaaaatctggctggcgctggcgggtctggtgctggcgttcagcgcgag cgcg | 125 |
| OmpA | atgaagaaaaccgcgattgcgattgcggtggcgctggcgggtttcgcgaccgtt gcgcaggcg | 126 |
| PelB | atgaagtacctgctgccgaccgcggcggcgggtctgctgctgctggcggcgca gccggcgatggcg | 127 |
| Sat | atgaataaaatatactcccttaaatatagtgctgccactggcggactcattgctgttt ctgaattagcgaaaagagtttctggtaaaacaaaccgaaaacttgtagcaacaat gttgtctctggctgttgccggtacagtaaatgca | 128 |
| USP45 | aagaaaaagatcattagcgcgatcctgatgagcaccgtgattctgagcgcggcg gcgccgctgagcggtgtttatgcg | 129 |
| csgA_N22 | atgaaacttttaaaagtagcagcaattgcagcaatcgtattctccggtagcgctct ggcaggtgttgttcctcagtacggcggcggcggtggcaaccacggtggtggcg gtaataacagcggtccgaat | 130 |
| FlgI_N22 | gtgataaagtttctgtcagcactgatattacttcttgttaccaccgccgttcaggccg gtgttgttcctcagtacggcggcggcggtggcaaccacggtggtggcggtaata acagcggtccgaat | 131 |
| sfmC_N22 | atgatgactaaaataaagttattgatgctcattatattttatttaatcatttcggccagc gcccatgctggtgttgttcctcagtacggcggcggcggtggcaaccacggtggt ggcggtaataacagcggtccgaat | 132 |
| USP45_N22 | atgaagaaaaagatcattagcgcgatcctgatgagcaccgtgattctgagcgcg gcggcgccgctgagcggtgtttatgcgggtgttgttcctcagtacggcggcggc ggtggcaaccacggtggtggcggtaataacagcggtccgaat | 133 |
| YraI_N22 | atgtcaaaacgaacattcgcggtgatattaaccttgttgtgtagcttctgtattggcc aggcgcttgcaggtgttgttcctcagtacggcggcggcggtggcaaccacggt ggtggcggtaataacagcggtccgaat | 134 |
| csgA | atgaaacttttaaaagtagcagcaattgcagcaatcgtattctccggtagcgctct ggcaggtgttgttcctcagtacggcggcggcggtggcaaccacggtggtggcg gtaataacagcggtccgaattcagagctgaatatttaccagtacggtggcggtaa ctctgctcttgctctgcaagctgacgcccgtaactctgatctgaccattacccagc acggcggcggtaatggcgcagatgtgggccaaggttctgatgacagctcaatc gatctgactcagcgtggtttcggcaacagcgctactcttgatcagtggaatggtaa agattctactatgactgttaaacagttcggtggcggtaacggtgctgctgttgacc agactgcatctaactccagcgttaacgtcactcaggttggctttggtaacaacgcg accgctcatcagtac | 135 |

In some embodiments, the signal peptide can be processed through export pathways and/or secretion systems present in genetically modified microorganisms (e.g., genetically modified bacteria). The signal peptide is usually at the N-terminus of the precursor protein and directs the precursor protein into the export pathway in the plasma membrane of the bacteria. The secretion system can remove the signal peptide from the precursor protein prior to secreting the mature protein from an engineered microorganism such as a bacterium.

As used herein, the term "secretion system" is used interchangeably herein with "export system" and "export pathway", and refers to a native or non-native secretion mechanism that is capable of secreting or exporting an expressed polypeptide product (e.g., Amuc_1100 polypeptide) from a microorganism (e.g., a bacterium). In Gram-negative bacteria such as *Escherichia coli,* from outside to inside, there are outer membrane (OM), peptidoglycan cell wall, periplasm, inner membrane (IM) and cytoplasmic compartments. OM is an extremely effective and selective permeability barrier. OM is a lipid bilayer composed of phospholipids in the inner layer, glycolipids in the outer layer, and lipoproteins and β-barrel proteins. OM is anchored to the underlying peptidoglycan via a lipoprotein called Lpp. The periplasm is densely packed with proteins, which is more viscous than the cytoplasm. IM is a phospholipid bilayer and primarily accommodates membrane proteins that play a role in energy production, lipid biosynthesis, and protein secretion and transport.

In bacteria, there are two common protein export pathways, including a general secretion (or Sec) pathway that is commonly existing and a twin-arginine translocation (or Tat) pathway. Typically, the Sec pathway processes precursor proteins with high molecular weight in an unfolded state, wherein a signal peptide directs the substrate protein to the membrane-bound Sec translocase. The precursor target protein is delivered to the translocase and passes through the SecYEG pore via SecA, SecD and SecF. The chaperone proteins SecB, GroEL-GroES, and DnaK-DnaJ-GrpE assist in the transport of the target protein. The Tat pathway usually transports proteins in a fully folded or even oligomeric state, and consists of the components TatA, TatB, and TatC in both Gram-negative and Gram-positive bacteria. Following membrane translocation, the signal peptide is removed by signal peptidase, and the mature protein is secreted.

Gram-negative bacteria have a dedicated single-step secretion system. Non-limiting examples of a secretion system include Sat secretion system, type I secretion system (T1SS), type II secretion system (T2SS), type III secretion system (T3SS ), type IV secretion system (T4SS), type V secretion system (T5SS), type VI secretion system (T6SS), the resistance-nodulation-division (RND) family of multidrug efflux pumps, and various single membrane secretion systems.

In some embodiments, the secretion system is native or non-native secretion system of the genetically modified microorganism. "Native" to the microorganism means that the secretion system is normally present in the microorganism, while "non-native" to the microorganism means that the secretion system is not usually present in the microorganism, e.g., an additional secretion system, such as secretion systems of different species, strains or subtypes of bacteria or viruses, or a secretion system that is modified and/or mutated compared to the unmodified secretion system of a microorganism from the same subtype.

In some embodiments, the genetically modified microorganism of the present disclosure further comprises one or more genetic modifications in the microorganism's genome. In some embodiments, the one or more genetic modifications include engineering and/or optimizing the secretion system such that at least one outer membrane protein encoding gene is deleted, inactivated, or inhibited.

In some embodiments, the secretion system is engineered and/or optimized such that at least one gene encoding outer membrane protein is deleted, inactivated, or inhibited in the genetically modified microorganism. In some embodiments, the outer membrane protein is selected from the group consisting of OmpC, OmpA, OmpF, OmpT, pldA, pagP, tolA, Pal, To1B, degS, mrcA, and lpp.

To produce Gram-negative bacteria (e.g., EcN) capable of secreting a desired therapeutic protein or peptide, the bacterial host needs to be genetically modified and given a "leaky" or destabilized outer membrane that does not affect the chemical physical activity of the host bacterial. Genes such as lpp, ompC, ompA, ompF, ompT, pldA, pagP, tolA, to 1B, pal, degS, degP and nlpI can be deleted or mutated to produce a "leaky" outer membrane. Lpp is the most abundant polypeptide in bacterial cells and serves as the main "staple" between the bacterial cell wall and peptidoglycan. Deletion of lpp has minimal effect on bacterial growth but increases the secretion of Amuc_1100, e.g., by at least double. Inducible promoters can be used to replace the endogenous promoters of one or more selected genes to minimize negative effects on cell viability.

"Deletion/missing", "inactivation" or "inhibition" of a gene or coding region means that the enzyme or protein encoded by the gene or coding region would not be produced, or produced in an inactive form in the microorganism, or produced in the microorganism at a lower rate than that found in the wild-type microorganism under the same or similar growth conditions. This can be achieved, for example, by one or more of the following methods: (1) homologous recombination, (2) RNA interference-based technologies, (3) ZFNs and TALENs, (4) CRISPR/Cas systems.

In some embodiments, the secretion system is engineered such that at least one chaperone encoding gene is amplified, overexpressed, or activated.

Chaperones are involved in many important biological processes, such as protein folding and aggregation of oligomeric protein complexes, maintaining protein precursors in an unfolded state to facilitate protein transport across membranes, and enabling denatured proteins to be depolymerized and repaired. Chaperones mainly assist other peptides to maintain their normal conformation to form the correct oligomeric structure and thereby exert normal physiological functions. Various chaperones are well known in the art. In some embodiments, the chaperone protein is selected from the group consisting of: dsbA, dsbC, dnaK, dnaJ, grpE, groES, groEL, tig, fkpA, surA, skp, PpiD and DegP. In some embodiments, the chaperone protein is selected from the group consisting of: Ssa1p, Ssa2p, Ssa3p and Ssa4p of the cytoplasmic SSA subfamily of 70 kDa heat shock proteins (Hsp70), BiP, Kar2, Lhs1, Sil1, Sec63 and protein disulfide isomerase Pdi1p. In some embodiments, the chaperones consist of dsbA, dsbC, dnaK, dnaJ, grpE, groES, groEL, tig, fkpAand surA.

"Overexpressed/overexpression" of a gene or coding region means that the enzyme or protein encoded by the gene or coding region is produced in the microorganism at a greater rate than that found in the wild-type microorganism under the same or similar growth conditions. This can be achieved by, for example, one or more of the following methods: (1) providing a stronger promoter, (2) providing a stronger ribosome binding site, such as the DNA sequence 5'-AGGAGG, located at approximately four to ten bases upstream of the start codon of translation, (3) providing a terminator or a stronger terminator, (4) improving selection of codon at one or more sites in the coding region, (5) increasing stability of mRNA, and (6) increasing the number of copies of genes by introducing multiple copies into the chromosome or placing the cassette on a multi-copy plasmid. Enzymes or proteins produced by overexpressed genes are said to be "overproduced". An "overexpressed" gene or an "overproduced" protein may be native to the microorganism, or it may be transplanted into the microorganism from a different organism by genetic modification methods. In the latter case, the enzyme or protein and the gene or coding region encoding the enzyme or protein are considered to be "exogenous" or "heterologous." Exogenous or heterologous genes and proteins are overexpressed and overproduced because they are not present in the unengineered microorganism.

The secretion system of yeast mainly consists of nascent protein translocation, protein folding in the endoplasmic reticulum (ER), glycosylation, and protein sorting and transport. As soon as the signal peptide emerges from the ribosome during translation, newly synthesized membrane or secreted proteins are recognized by signal recognition particles (SRPs) and subsequently targeted for post-translational secretion through the Sec61 or Ssh1 translocon pore. Various methods have been implemented to improve heterologous protein production in yeast as follows: (1) engineering signal peptides, and increasing strength of promoters and number of copies of plasmids; (2) engineering post-translational pathways of the host strain, such as overexpressing ER folding chaperones and vesicle transport components, and reducing intercellular and extracellular proteolysis. Exemplary secretion signal peptides of yeast for secretion of heterologous proteins include *Saccharomyces cerevisiae* α-mating factor (α-MF) pre pro-peptide, a signal peptide of inulinase of *Kluyveromyces marxianus,* a signal peptide of PHAE of *Phaseolus vulgaris agglutinin,* a signal peptide of viral prepro toxin, a signal peptide of *Rhizopus oryzae* amylase or a signal peptide of the hydrophobin of *Trichoderma reesei.*

### E. Genomic integration site

In some embodiments, an exogenous expression cassette is integrated into the genome of the genetically modified microorganism.

In some embodiments, the exogenous expression cassette is carried by a plasmid, and the plasmid is introduced into the microorganism and is suitable for expressing in the microorganism.

In some embodiments, the genetically modified microorganism described herein is capable of growing stably, expressing non-natural genetic material stably, such as genes encoding Amuc_1100, IL-10 and/or IL-22, or expressing exogenous expression cassettes of these polypeptides/proteins. In some embodiments, the non-natural genetic material is introduced into the genome of the microorganism or replicated with an extrachromosomal plasmid, such that the non-natural genetic material is retained, transcribed and expressed.

In some embodiments, a stable microorganism (e.g., a bacterium) may be a genetically modified microorganism that expresses and secretes Amuc_1100, IL-10, and/or IL-22, wherein a plasmid or chromosome containing an exogenous expression cassette encoding Amuc_1100, IL-10, and/or IL-22 is stably maintained in the genetically modified microorganism, so that the exogenous expression cassette can be expressed in the genetically modified microorganism, and the genetically modified microorganism is capable of surviving and/or growing *in vitro* and/or *in vivo.* Stable microorganisms are capable of surviving and growing *in vitro* (e.g., in culture media) or *in vivo* (e.g., in the digestive tract).

In some embodiments, copy number of the unnatural genetic material affects the expression stability of the unnatural genetic material. In some embodiments, copy number affects the expression level of the unnatural genetic material. In an embodiment where the genetically modified microorganism comprises one or more gene sequences and one or more gene cassettes, the gene sequences may be present in one or more plasmids, and the gene cassettes may be present in the chromosomes of the microorganism and vice versa. In addition, multiple copies (e.g., one, two, three, four, five, six, or more) of any gene, gene cassette, or regulatory region may be present in the microorganism, wherein one or more copies of the gene, gene cassette, or regulatory region may be mutated or otherwise altered as described herein. In some embodiments, the genetically modified microorganism is engineered to contain multiple copies of the same gene, gene cassette, or regulatory region to increase the number of copies. In some embodiments, the genetically modified microorganism is engineered to contain a variety of different components of a gene cassette that performs a variety of different functions. In some embodiments, the genetically modified microorganism is engineered to contain one or more copies of different genes, gene cassettes, or regulatory regions to produce a genetically modified microorganism that expresses more than one therapeutic molecules and/or performs more than one functions. In some embodiments, number of copies affects the stability of expression of the unnatural genetic material. In some embodiments, number of copies affects the expression level of the unnatural genetic material.

In some embodiments, two or more gene sequences are multiple copies of the same gene. In some embodiments, two or more gene sequences are sequences encoding different genes. In some embodiments, two or more gene sequences are sequences encoding multiple copies of one or more different genes. In some embodiments, the genetically modified microorganism comprises one or more gene cassettes of molecules used for treating or preventing inflammatory or autoimmune diseases. For example, the genetically modified microorganism may comprise two or more gene cassettes of the molecules used for treating or preventing inflammatory or autoimmune diseases. In some embodiments, two or more gene cassettes are multiple copies of the same gene cassette. In some embodiments, two or more gene cassettes are different gene cassettes for producing the same or different molecules that treat or prevent inflammatory diseases or autoimmune diseases. In some embodiments, two or more gene cassettes are gene cassettes for multiple copies of a molecule.

In addition, multiple copies of any regulatory region, promoter, gene, and/or gene cassette may be present in a genetically modified microorganism, wherein one or more copies of the regulatory region, promoter, gene, and/or gene cassette may be mutated or otherwise altered as described herein. In some embodiments, the genetically modified microorganism is engineered to contain multiple copies of the same regulatory region, promoter, gene, and/or gene cassette to increase the number of copies, or to contain a variety of different components of a gene cassette that performs a variety of different functions, or to contain one or more copies of different regulatory regions, promoters, genes, and/or gene cassettes, to produce a genetically modified microorganism that expresses more than one therapeutic molecules and/or performs more than one functions.

In some embodiments, the exogenous expression cassette is integrated into the genome of the genetically modified microorganism via the CRISPR-Cas gene editing system. Any suitable microorganism provided herein can be engineered such that exogenous expression cassettes are integrated into the genome.

In some embodiments, the genetically modified microorganism is *Escherichia coli* strain *Nissle 1917* (*EcN*), and the exogenous expression cassette is integrated into a site of the EcN genome. In some embodiments, suitable integration sites in the EcN genome are integration sites agal/rsmI, lacZ, kefB, malP/T, yicS/nepl, rhtB/C, maeB, malE/K, yieN, lldD, maeA, pflB or araB/C. The Amuc_1100, IL-10 and/or IL-22 of the present invention are integrated into sites of the EcN genome. Without wishing to be bound by any theory, it is believed that the site in the EcN genome of the present disclosure is advantageous for inserting the expression cassette of Amuc_1100, IL-10 and/or IL-22 through at least one of the following characteristics: (1) one or more bacterial genes affected by engineering the site are not necessary for EcN growth and do not alter the biochemical and physiological activity of the host bacterium, (2) the site can be readily edited, and (3) the Amuc_1100, IL-10 and/or IL-22 gene cassettes in the site can be transcribed.

### F. Auxotrophs

In some embodiments, the microorganism of the present disclosure further comprises inactivation or deletion of at least one of the auxotrophy-associated genes.

In order to produce environmentally friendly bacteria, some essential genes necessary for bacterial cell survival can be deleted or inactivated through gene editing, rendering the engineered bacteria to be auxotrophic. As used herein, the term "auxotroph" refers to a microorganism (e.g., a strain of microorganism) that requires an external source of a specific metabolite for growth that is unable to be synthesized due to an acquired genetic defect.

As used herein, the term "auxotrophy-associated gene" refers to a gene required for the survival of a microorganism (e.g., a microorganism, such as a bacterium). An auxotrophy-associated gene may be required for the microorganism to produce nutrients necessary for survival or growth, or may be required for detecting signals in the environment that regulate the activity of transcription factors, wherein the absence of the signals would cause cell death.

In some embodiments, an auxotrophic modification is intended to cause the death of the microorganism in the absence of exogenously added nutrients necessary for survival or growth because the microorganism lacks one or more genes necessary to produce the necessary nutrients. In some embodiments, any of the genetically modified bacteria described herein further comprises a deletion or mutation of a gene required for cell survival and/or growth.

Various auxotrophy-associated genes in bacteria are well known in the art. Exemplary auxotrophy-associated genes include, but are not limited to: thy A, cysE, glnA, ilvD, leuB, lysA, serA, metA, glyA, hisB, ilvA, pheA, proA, thrC, trpC, tyrA, uraA, dapF, flhD, metB, metC, proAB, yhbV, yagG, hemB, secD, secF, ribD, ribE, thiL, dxs, ispA, dnaX, adk, hemH, IpxH, cysS, fold, rplT, infC, thrS, nadE, gapA, yeaZ, aspS, argS, pgsA, yeflA, metG, folE, yejM, gyrA, nrdA, nrdB, folC, accD, fabB, gltX, ligA, zipA, dapE, dapA, der, hisS, ispG, suhB, tadA, acpS, era, rnc, fisB, eno, pyrG, chpR, Igt, fbaA, pgk, yqgD, metK, yqgF, plsC, ygiT, pare, ribB, cca, ygjD, tdcF, yraL, yihA, ftsN, murl, murB, birA, secE, nusG, rplJ, rplL, rpoB, rpoC, ubiA, plsB, lexA, dnaB, ssb, alsK, groS, psd, orn, yjeE, rpsR, chpS, ppa, valS, yjgP, yjgQ, dnaC, ribF, IspA, ispH, dapB, folA, imp, yabQ, flsL, flsl, murE, murF, mraY, murD, ftsW, murG, murC, ftsQ, ftsA, ftsZ, IpxC, secM, secA, can, folK, hemL, yadR, dapD, map, rpsB, infB, nusA, ftsH, obgE, rpmA, rplU, ispB, murA, yrbB, yrbK, yhbN, rpsl, rplM, degS, mreD, mreC, mreB, accB, accC, yrdC, def, fint, rplQ, rpoA, rpsD, rpsK, rpsM, entD, mrdB, mrdA, nadD, hlepB, rpoE, pssA, yfiO, rplS, trmD, rpsP, ffh, grpE, yfjB, csrA, ispF, ispD, rplW, rplD, rplC, rpsJ, fusA, rpsG, rpsL, trpS, yrfF, asd, rpoH, ftsX, ftsE, ftsY, frr, dxr, ispU, rfaK, kdtA, coaD, rpmB, djp, dut, gmk, spot, gyrB, dnaN, dnaA, rpmH, rnpA, yidC, tnaB, glmS, glmU, wzyE, hemD, hemC, yigP, ubiB, ubiD, hemG, secY, rplO, rpmD, rpsE, rplR, rplF, rpsH, rpsN, rplE, rplX, rplN, rpsQ, rpmC, rplP, rpsC, rplV, rpsS, rplB, cdsA, yaeL, yaeT, lpxD, fabZ, IpxA, IpxB, dnaE, accA, tilS, proS, yafF, tsf, pyrH, olA, rlpB, leuS, Int, glnS, fldA, cydA, infA, cydC, ftsK, lolA, serS, rpsA, msbA, IpxK, kdsB, mukF, mukE, mukB, asnS, fabA, mviN, rne, yceQ, fabD, fabG, acpP, tmk, holB, lolC, lolD, lolE, purB, ymflC, minE, mind, pth, rsA, ispE, lolB, hemA, prfA, prmC, kdsA, topA, ribA, fabi, racR, dicA, yd B, tyrS, ribC, ydiL, pheT, pheS, yhhQ, bcsB, glyQ, yibJ and gpsA.

In a modification, the essential gene thyA is deleted or replaced by another gene, rendering the genetically modified bacterium dependent on exogenous thymine for growth or survival. The addition of thymine to the growth medium or the high thymine content naturally existing in the human intestine can support the growth and survival of thyA auxotrophic bacteria. Such modification is to ensure that the genetically modified bacterium cannot grow and survive outside the intestinal tract or in an environment lacking the product of the auxotrophic gene.

In some embodiments, the microorganism is an auxotroph of the substance selected from the group consisting of: uracil, thymine, diaminopimelic acid, leucine, histidine, tryptophan, lysine, methionine, adenine, a non-naturally occurring amino acid and a combination thereof. In some embodiments, the non-naturally occurring amino acid is selected from the group consisting of: l-4,4'-biphenylalanine, p-acetyl-l-phenylalanine, p-iodo-l-phenylalanine and p-azido-l-phenylalanine.

In some embodiments, the microorganism comprises an allosteric transcription factor capable of detecting a signal in an environment that modulates the activity of the transcription factor, wherein the absence of the signal would cause the death of cell. The "signal molecule-transcription factor" pair is selected from the group consisting of: tryptophan-TrpR, IPTG-LacI, benzoate derivative-XylS, ATc-TetR, galactose-GalR, estradiol-estrogen receptor hybrid protein, cellobiose-CelR, homoserine lactone-luxR and a combination thereof.

### G. Deletion of endogenous plasmid

In some embodiments, one or more endogenous plasmids are deleted from the genetically modified bacterium.

Some chassis bacterial hosts comprise one or more endogenous plasmids that consume considerable resources for their transcription. Without being bound by any theory, it is believed that the deletion of these endogenous plasmids frees up resources that can be better utilized for the expression of heterologous genes.

One or more endogenous plasmids may be removed from the target microorganism by methods known in the art. For example, EcN comprises two endogenous plasmids, pMUT1 and pMUT2, which can be removed from EcN by any appropriate method. An exemplary method is through CRISPR-Cas9-mediated DNA double-strand cleavage. In short, a guide RNA (gRNA or single-stranded guide RNA, sgRNA) that specifically binds pMUT1 or pMUT2 and a nucleic acid sequence (such as a plasmid) expressing the Cas9 protein can be introduced into EcN, thereby destroying pMUT1 or pMUT2 through DNA double-strand cleavage mediated by Cas9 protein in EcN. As another embodiment, pMUT2 in EcN can also be replaced with a recombinant pMUT2 plasmid expressing a specific resistance gene (such as a kanamycin resistance gene), for example, by screening for resistant recombinant EcN. Then, gRNA or sgRNA that specifically binds to the resistance gene and a nucleic acid sequence (such as a plasmid) expressing the Cas9 protein is introduced, so that the double-stranded DNA cleavage mediated by the Cas9 protein in EcN destroys the replaced recombinant pMUT2 plasmid.

The inventors of the present application surprisingly found that the genetically modified microorganism (e.g., a bacterium) provided by the present invention can not only grow well and maintain viability, but can also allow integration of at least two exogenous genes into their genome simultaneously, and remain stable and efficient expression and secretion of the polypeptides/proteins encoded by the at least two exogenous genes. More importantly, the microorganism expressing and secreting a novel combination of exogenous polypeptides (at least two combinations of Amuc_1100/IL-10/IL-22) is administered through the intestinal tract and shows therapeutic effect, even unexpected synergistic effect, on a variety of inflammatory diseases or autoimmune diseases (such as inflammatory bowel disease) models in animal. Therefore, the engineered microorganisms of the present invention have broad clinical application prospects as a living drug.

The present invention further provides a method for producing the genetically modified microorganism, comprising the steps of: introducing into a microorganism a nucleotide sequence that can exogenously express Amuc_1100, IL-10 and/or IL-22 of the present invention, so that the exogenous gene in the nucleotide sequence can be expressed in the microorganism, thereby obtaining the genetically modified microorganism.

In some embodiments, an exemplary optimized Amuc_1100 expression cassette of the present invention has a structure set forth in any one of the sequences SEQ ID NO: 139 to SEQ ID NO: 143. The IL-10 expression cassette has a structure set forth in any one of the sequences SEQ ID NO: 144 to SEQ ID NO: 156 and 235. The IL-22 expression cassette has a structure set forth in any one of the sequences SEQ ID NO: 157 to SEQ ID NO: 163.

### Examples of Amuc_1100 sequences comprising different regulatory elements

In some embodiments, the present application provides a variety of specific Amuc_1100 expression cassette sequences comprising different regulatory elements (such as different promoters, different signal peptides, different cistrons, etc.), as shown below. In some embodiments, the present application further provides a genetically modified microorganism (e.g., EcN) comprising any of the specific Amuc_1100 expression cassette sequences. Examples of the specific Amuc_1100 expression cassette sequence provided in the present application are as follows:

BBa_J23101_USP45_Amuc_1100(Y259A)_rmB_T1_T7Te (corresponding to the expression cassette comprised in strains CBT4101, CBT4103 and CBT4108 in the embodiments of the present disclosure, SEQ ID NO: 139)

tctagagcttgtcgacgtctcgagtttacagctagctcagtcctaggtattatgctagcctcgaggaaagaggagaaagaagcttaagaaaaagat
cattagcgcgatcctgatgagcaccgtgattctgagcgcggcggcgccgctgagcggtgtttatgcgattgttaacagcaagcgtagcgagctggataagaaaat

cagcattgcggcgaaagagatcaagagcgcgaacgcggcggaaattaccccgagccgtagcagcaacgaggaactggagaaagaactgaaccgttacgcg
aaggcggttggtagcctggaaaccgcgtataaaccgtttctggcgagcagcgcgctggtgccgaccaccccgaccgcgttccaaaacgagctgaaaacctttc
gtgacagcctgatcagcagctgcaagaaaaagaacatcctgattaccgataccagcagctggctgggcttccaggtttacagcacccaagcgccgagcgtgca
ggcggcgagcaccctgggttttgagctgaaagcgattaacagcctggttaacaagctggcggaatgcggcctgagcaaattcatcaaggtgtatcgtccgcagc
tgccgattgaaaccccggcgaacaacccggaggaaagcgacgaagcggatcaggcgccgtggaccccgatgccgctggagatcgcgttccagggtgaccg
tgaaagcgttctgaaagcgatgaacgcgattaccggcatgcaagattacctgtttaccgtgaacagcatccgtattcgtaacgaacgtatgatgccgccgccaatc
gcgaacccggctgcggcgaagccggctgcggcgcagccggcgaccggtgcggcgagcctgaccccggcggacgaggctgcggcgccggctgcgccgg
cgatccagcaagttattaaaccggctatgggcaaggaacaggtgttcgttcaagtgagcctgaacctggtgcactttaaccagccgaaagcgcaagagccgagc
gaagatcatcatcaccaccaccactaacgagctcgatagtgctagtgtagatcgctactagagccaggcatcaaataaaacgaaaggctcagtcgaaagactgg
gcctttcgttttatctgttgtttgtcggtgaacgctctctactagagtcacactggctcaccttcgggtgggcctttctgcgtttatatactagaagcggccgctgcag.

BBa_J23110_USP45_Amuc_1100(Y259A)_rrnB_T1_T7Te (corresponding to the expression cassettes comprised in strains CBT4102, CBT4107, CBT4108 and CBT4109 in the embodiments of the present disclosure, SEQ ID NO: 140)

tctagagcttgtcgacgtctcgagtttacggctagctcagtcctaggtacaatgctagcctcgaggaaagaggagaaagaagcttaagaaaaaga
tcattagcgcgatcctgatgagcaccgtgattctgagcgcggcggcgccgctgagcggtgtttatgcgattgttaacagcaagcgtagcgagctggataagaaaa
tcagcattgcggcgaaagagatcaagagcgcgaacgcggcggaaattaccccgagccgtagcagcaacgaggaactggagaaagaactgaaccgttacgc
gaaggcggttggtagcctggaaaccgcgtataaaccgtttctggcgagcagcgcgctggtgccgaccaccccgaccgcgttccaaaacgagctgaaaaccttt
cgtgacagcctgatcagcagctgcaagaaaaagaacatcctgattaccgataccagcagctggctgggcttccaggtttacagcacccaagcgccgagcgtgc
aggcggcgagcaccctgggttttgagctgaaagcgattaacagcctggttaacaagctggcggaatgcggcctgagcaaattcatcaaggtgtatcgtccgcag
ctgccgattgaaaccccggcgaacaacccggaggaaagcgacgaagcggatcaggcgccgtggaccccgatgccgctggagatcgcgttccagggtgacc
gtgaaagcgttctgaaagcgatgaacgcgattaccggcatgcaagattacctgtttaccgtgaacagcatccgtattcgtaacgaacgtatgatgccgccgccaat
cgcgaacccggctgcggcgaagccggctgcggcgcagccggcgaccggtgcggcgagcctgaccccggcggacgaggctgcggcgccggctgcgccg
gcgatccagcaagttattaaaccggctatgggcaaggaacaggtgttcgttcaagtgagcctgaacctggtgcactttaaccagccgaaagcgcaagagccgag
cgaagatcatcatcaccaccaccactaacgagctcgatagtgctagtgtagatcgctactagagccaggcatcaaataaaacgaaaggctcagtcgaaagactg
ggcctttcgttttatctgttgtttgtcggtgaacgctctctactagagtcacactggctcaccttcgggtgggcctttctgcgtttatatactagaagcggccgctgcag.

BBa_J23101_USP45_Amuc_1100(WT)_rrnB_T1(mutant)_T7Te (corresponding to the expression cassettes comprised in strains CBT4068, CBT4069, CBT4067 and CBT4070 in the embodiments of the present disclosure, SEQ ID NO: 141)

tctagagcttgtcgacgtctcgagtttacagctagctcagtcctaggtattatgctagcctcgaggaaagaggagaaagaagcttatgaagaaaaa
gatcattagcgcgatcctgatgagcaccgtgattctgagcgcggcggcgccgctgagcggtgtttatgcgggtagcagccatcatcaccaccaccacgacgac
gacgacaaaattgttaacagcaagcgtagcgagctggataagaaaatcagcattgcggcgaaagagatcaagagcgcgaacgcggcggaaattaccccgag
ccgtagcagcaacgaggaactggagaaagaactgaaccgttacgcgaaggcggttggtagcctggaaaccgcgtataaaccgtttctggcgagcagcgcgct
ggtgccgaccaccccgaccgcgttccaaaacgagctgaaaacctttcgtgacagcctgatcagcagctgcaagaaaaagaacatcctgattaccgataccagca
gctggctgggcttccaggtttacagcacccaagcgccgagcgtgcaggcggcgagcaccctgggttttgagctgaaagcgattaacagcctggttaacaagctg
gcggaatgcggcctgagcaaattcatcaaggtgtatcgtccgcagctgccgattgaaaccccggcgaacaacccggaggaaagcgacgaagcggatcaggc
gccgtggaccccgatgccgctggagatcgcgttccagggtgaccgtgaaagcgttctgaaagcgatgaacgcgattaccggcatgcaagattacctgtttaccgt
gaacagcatccgtattcgtaacgaacgtatgatgccgccgccaatcgcgaacccggctgcggcgaagccggctgcggcgcagccggcgaccggtgcggcg
agcctgaccccggcggacgaggctgcggcgccggctgcgccggcgatccagcaagttattaaaccgtatatgggcaaggaacaggtgttcgttcaagtgagc
ctgaacctggtgcactttaaccagccgaaagcgcaagagccgagcgaagattgacgagctcgatagtgctagtgtagatcgctactagagccaggcatcaaata
aaacgaaagactgggcctttcgttttatctgttgtttgtcggtgaacgctctctactagagtcacactggctcaccttcgggtgggcctttctgcgtttatatactagaag
cggccgctgcag.

BBa_J23110_USP45_Amuc_1100(WT)_rrnB_T1_T7Te (corresponding to the expression cassettes comprised in strains CBT4105, CBT4106, CBT4068, CBT4069, CBT4067 and CBT4070 in the embodiments of the present disclosure, SEQ ID NO: 142)

tctagagcttgtcgacgtctcgagtttacggctagctcagtcctaggtacaatgctagcctcgaggaaagaggagaaagaagcttatgaagaaaa
agatcattagcgcgatcctgatgagcaccgtgattctgagcgcggcggcgccgctgagcggtgtttatgcgggtagcagccatcatcaccaccaccacgacgac
gacgacaaaattgttaacagcaagcgtagcgagctggataagaaaatcagcattgcggcgaaagagatcaagagcgcgaacgcggcggaaattaccccgag
ccgtagcagcaacgaggaactggagaaagaactgaaccgttacgcgaaggcggttggtagcctggaaaccgcgtataaaccgtttctggcgagcagcgcgct
ggtgccgaccaccccgaccgcgttccaaaacgagctgaaaacctttcgtgacagcctgatcagcagctgcaagaaaaagaacatcctgattaccgataccagca

gctggctgggcttccaggtttacagcacccaagcgccgagcgtgcaggcggcgagcaccctgggttttgagctgaaagcgattaacagcctggttaacaagctg
gcggaatgcggcctgagcaaattcatcaaggtgtatcgtccgcagctgccgattgaaaccccggcgaacaacccggaggaaagcgacgaagcggatcaggc
gccgtggaccccgatgccgctggagatcgcgttccagggtgaccgtgaaagcgttctgaaagcgatgaacgcgattaccggcatgcaagattacctgtttaccgt
gaacagcatccgtattcgtaacgaacgtatgatgccgccgccaatcgcgaacccggctgcggcgaagccggctgcggcgcagccggcgaccggtgcggcg
agcctgaccccggcggacgaggctgcggcgccggctgcgccggcgatccagcaagttattaaaccgtatatgggcaaggaacaggtgttcgttcaagtgagc
ctgaacctggtgcactttaaccagccgaaagcgcaagagccgagcgaagattgacgagctcgatagtgctagtgtagatcgctactagagccaggcatcaaata
aaacgaaaggctcagtcgaaagactgggcctttcgttttatctgttgtttgtcggtgaacgctctctactagagtcacactggctcaccttcgggtgggcctttctgcg
tttatatactagaagcggccgctgcag.

PfnrS_USP45_Amuc_1100(WT)_rrnB_T1_T7Te (corresponding to the expression cassette comprised in strains CBT4080, CBT4096, CBT4088, CBT4098 and CBT4111 in the embodiments of the present disclosure, SEQ ID NO: 143)

tctagagcttgtcgacgtctcgagaaaaacgccgcaaagtttgagcgaagtcaataaactctctacccattcagggcaatatctctcttctcgagga
aagaggagaaagaagcttatgaagaaaaagatcattagcgcgatcctgatgagcaccgtgattctgagcgcggcggcgccgctgagcggtgtttatgcgggta
gcagccatcatcaccaccaccacgacgacgacgacaaaattgttaacagcaagcgtagcgagctggataagaaaatcagcattgcggcgaaagagatcaaga
gcgcgaacgcggcggaaattaccccgagccgtagcagcaacgaggaactggagaaagaactgaaccgttacgcgaaggcggttggtagcctggaaaccgc
gtataaaccgtttctggcgagcagcgcgctggtgccgaccaccccgaccgcgttccaaaacgagctgaaaacctttcgtgacagcctgatcagcagctgcaaga
aaaagaacatcctgattaccgataccagcagctggctgggcttccaggtttacagcacccaagcgccgagcgtgcaggcggcgagcaccctgggttttgagctg
aaagcgattaacagcctggttaacaagctggcggaatgcggcctgagcaaattcatcaaggtgtatcgtccgcagctgccgattgaaaccccggcgaacaaccc
ggaggaaagcgacgaagcggatcaggcgccgtggaccccgatgccgctggagatcgcgttccagggtgaccgtgaaagcgttctgaaagcgatgaacgcg
attaccggcatgcaagattacctgtttaccgtgaacagcatccgtattcgtaacgaacgtatgatgccgccgccaatcgcgaacccggctgcggcgaagccggct
gcggcgcagccggcgaccggtgcggcgagcctgaccccggcggacgaggctgcggcgccggctgcgccggcgatccagcaagttattaaaccgtatatgg
gcaaggaacaggtgttcgttcaagtgagcctgaacctggtgcactttaaccagccgaaagcgcaagagccgagcgaagattgacgagctcgatagtgctagtgt
agatcgctactagagccaggcatcaaataaaacgaaaggctcagtcgaaagactgggcctttcgttttatctgttgtttgtcggtgaacgctctctactagagtcaca
ctggctcaccttcgggtgggcctttctgcgtttatatactagaagcggccgctgcag.

### Examples of IL-10 sequences comprising regulatory elements

In some embodiments, the present application provides a variety of specific Amuc_1100 expression cassette sequences comprising different regulatory elements (such as different promoters, different signal peptides, and different cistrons), as shown below. In some embodiments, the present application further provides a genetically modified microorganism (e.g., EcN) comprising any of the specific IL-10 expression cassette sequences. Examples of the specific Amuc_1100 expression cassette sequence provided in the present application are as follows:

BBa_J23101_USP45_IL-10_rrnB_T1_T7Te (corresponding to the expression cassette comprised in strain CBT4003 in the embodiments of the present disclosure, SEQ ID NO: 144)

tctagagcttgtcgacgtctcgagtttacagctagctcagtcctaggtattatgctagcctcgaggaaagaggagaaagaagcttatgaagaaaaa
gatcattagcgcgatcctgatgagcaccgtgattctgagcgcggcggcgccgctgagcggtgtttatgcgagcccgggtcagggtacccaaagcgaaaacagc
tgcacccacttcccgggcaacctgccgaacatgctgcgtgacctgcgtgatgcgttcagccgtgtgaaaaccttcttccagatgaaagaccaactggataacctg
ctgctgaaggagagcctgctggaggacttcaaaggttacctgggctgccaggcgctgagcgaaatgatccaattttatctggaggaagtgatgccgcaggcgga
gaaccaagacccggatattaaggcgcacgttaacagcctgggcgagaacctgaaaaccctgcgtctgcgtctgcgtcgttgccaccgtttcctgccgtgcgaga
acaaaagcaaggcggtggaacaggttaagaacgcgtttaacaaactgcaagagaagggcatctataaagcgatgagcgaattcgatatctttattaactacatcg
aggcgtatatgaccatgaaaattcgtaactagtaacgagctcgatagtgctagtgtagatcgctactagagccaggcatcaaataaaacgaaaggctcagtcgaa
agactgggcctttcgttttatctgttgtttgtcggtgaacgctctctactagagtcacactggctcaccttcgggtgggcctttctgcgtttatatactagaagcggccgc
tgcag.

BBa_J23110_USP45_IL-10_rrnB_T1_T7Te (corresponding to the expression cassette comprised in strains CBT4005, CBT4071, CBT4072, CBT4073, CBT4074, CBT4112, CBT4020, CBT4075, CBT4076 and CBT4077 in the embodiments of the present disclosure, SEQ ID NO: 145)

tctagagcttgtcgacgtctcgagtttacggctagctcagtcctaggtacaatgctagcctcgaggaaagaggagaaagaagcttatgaagaaaa
agatcattagcgcgatcctgatgagcaccgtgattctgagcgcggcggcgccgctgagcggtgtttatgcgagcccgggtcagggtacccaaagcgaaaacag
ctgcacccacttcccgggcaacctgccgaacatgctgcgtgacctgcgtgatgcgttcagccgtgtgaaaaccttcttccagatgaaagaccaactggataacct

gctgctgaaggagagcctgctggaggacttcaaaggttacctgggctgccaggcgctgagcgaaatgatccaattttatctggaggaagtgatgccgcaggcgg
agaaccaagacccggatattaaggcgcacgttaacagcctgggcgagaacctgaaaaccctgcgtctgcgtctgcgtcgttgccaccgtttcctgccgtgcgag
aacaaaagcaaggcggtggaacaggttaagaacgcgtttaacaaactgcaagagaagggcatctataaagcgatgagcgaattcgatatctttattaactacatc
gaggcgtatatgaccatgaaaattcgtaactagtaacgagctcgatagtgctagtgtagatcgctactagagccaggcatcaaataaaacgaaaggctcagtcga
aagactgggcctttcgttttatctgttgtttgtcggtgaacgctctctactagagtcacactggctcaccttcgggtgggcctttctgcgtttatatactagaagcggccg
ctgcag.

BBa_J23108_USP45_IL-10_rrnB_T1_T7Te (corresponding to the expression cassette comprised in strain CBT4004 in an embodiment of the present disclosure, SEQ ID NO: 146)

tctagagcttgtcgacgtctcgagctgacagctagctcagtcctaggtataatgctagcctcgaggaaagaggagaaagaagcttatgaagaaaa
agatcattagcgcgatcctgatgagcaccgtgattctgagcgcggcggcgccgctgagcggtgtttatgcgagcccgggtcagggtacccaaagcgaaaacag
ctgcacccacttcccgggcaacctgccgaacatgctgcgtgacctgcgtgatgcgttcagccgtgtgaaaaccttcttccagatgaaagaccaactggataacct
gctgctgaaggagagcctgctggaggacttcaaaggttacctgggctgccaggcgctgagcgaaatgatccaattttatctggaggaagtgatgccgcaggcgg
agaaccaagacccggatattaaggcgcacgttaacagcctgggcgagaacctgaaaaccctgcgtctgcgtctgcgtcgttgccaccgtttcctgccgtgcgag
aacaaaagcaaggcggtggaacaggttaagaacgcgtttaacaaactgcaagagaagggcatctataaagcgatgagcgaattcgatatctttattaactacatc
gaggcgtatatgaccatgaaaattcgtaactagtaacgagctcgatagtgctagtgtagatcgctactagagccaggcatcaaataaaacgaaaggctcagtcga
aagactgggcctttcgttttatctgttgtttgtcggtgaacgctctctactagagtcacactggctcaccttcgggtgggcctttctgcgtttatatactagaagcggccg
ctgcag.

BBa_J23101_DsbA_IL-10_rrnB_T1_T7Te (corresponding to the expression cassette comprised in strain CBT4007 in an embodiment of the present disclosure, SEQ ID NO: 147)

tctagagcttgtcgacgtctcgagtttacagctagctcagtcctaggtattatgctagcctcgaggaaagaggagaaagaagcttatgaagaaaat
ctggctggcgctggcgggtctggtgctggcgttcagcgcgagcgcgagcccgggtcagggtacccaaagcgaaaacagctgcacccacttcccgggcaacc
tgccgaacatgctgcgtgacctgcgtgatgcgttcagccgtgtgaaaaccttcttccagatgaaagaccaactggataacctgctgctgaaggagagcctgctgg
aggacttcaaaggttacctgggctgccaggcgctgagcgaaatgatccaattttatctggaggaagtgatgccgcaggcggagaaccaagacccggatattaag
gcgcacgttaacagcctgggcgagaacctgaaaaccctgcgtctgcgtctgcgtcgttgccaccgtttcctgccgtgcgagaacaaaagcaaggcggtggaac
aggttaagaacgcgtttaacaaactgcaagagaagggcatctataaagcgatgagcgaattcgatatctttattaactacatcgaggcgtatatgaccatgaaaattc
gtaactagtaacgagctcgatagtgctagtgtagatcgctactagagccaggcatcaaataaaacgaaaggctcagtcgaaagactgggcctttcgttttatctgtt
gtttgtcggtgaacgctctctactagagtcacactggctcaccttcgggtgggcctttctgcgtttatatactagaagcggccgctgcag.

BBa_J23101_OmpA_IL-10_rrnB_T1_T7Te (corresponding to the expression cassette comprised in strain CBT4009 in an embodiment of the present disclosure, SEQ ID NO: 148)

tctagagcttgtcgacgtctcgagtttacagctagctcagtcctaggtattatgctagcctcgaggaaagaggagaaagaagcttatgaagaaaac
cgcgattgcgattgcggtggcgctggcgggtttcgcgaccgttgcgcaggcgagcccgggtcagggtacccaaagcgaaaacagctgcacccacttcccggg
caacctgccgaacatgctgcgtgacctgcgtgatgcgttcagccgtgtgaaaaccttcttccagatgaaagaccaactggataacctgctgctgaaggagagcct
gctggaggacttcaaaggttacctgggctgccaggcgctgagcgaaatgatccaattttatctggaggaagtgatgccgcaggcggagaaccaagacccggat
attaaggcgcacgttaacagcctgggcgagaacctgaaaaccctgcgtctgcgtctgcgtcgttgccaccgtttcctgccgtgcgagaacaaaagcaaggcggt
ggaacaggttaagaacgcgtttaacaaactgcaagagaagggcatctataaagcgatgagcgaattcgatatctttattaactacatcgaggcgtatatgaccatga
aaattcgtaactagtaacgagctcgatagtgctagtgtagatcgctactagagccaggcatcaaataaaacgaaaggctcagtcgaaagactgggcctttcgtttta
tctgttgtttgtcggtgaacgctctctactagagtcacactggctcaccttcgggtgggcctttctgcgtttatatactagaagcggccgctgcag.

BBa_J23101_PelB_IL-10_rrnB_T1_T7Te (corresponding to the expression cassette comprised in strain CBT4011 in an embodiment of the present disclosure, SEQ ID NO: 149)

tctagagcttgtcgacgtctcgagtttacagctagctcagtcctaggtattatgctagcctcgaggaaagaggagaaagaagcttatgaagtacctg
ctgccgaccgcggcggcgggtctgctgctgctggcggcgcagccggcgatggcgagcccgggtcagggtacccaaagcgaaaacagctgcacccacttcc
cgggcaacctgccgaacatgctgcgtgacctgcgtgatgcgttcagccgtgtgaaaaccttcttccagatgaaagaccaactggataacctgctgctgaaggaga
gcctgctggaggacttcaaaggttacctgggctgccaggcgctgagcgaaatgatccaattttatctggaggaagtgatgccgcaggcggagaaccaagaccc
ggatattaaggcgcacgttaacagcctgggcgagaacctgaaaaccctgcgtctgcgtctgcgtcgttgccaccgtttcctgccgtgcgagaacaaaagcaagg
cggtggaacaggttaagaacgcgtttaacaaactgcaagagaagggcatctataaagcgatgagcgaattcgatatctttattaactacatcgaggcgtatatgac
catgaaaattcgtaactagtaacgagctcgatagtgctagtgtagatcgctactagagccaggcatcaaataaaacgaaaggctcagtcgaaagactgggcctttc
gttttatctgttgtttgtcggtgaacgctctctactagagtcacactggctcaccttcgggtgggcctttctgcgtttatatactagaagcggccgctgcag.

BBa_J23101_YebF_IL-10_rrnB_T1_T7Te (corresponding to the expression cassette comprised in strain CBT4013 in an embodiment of the present disclosure, SEQ ID NO: 150)

tctagagcttgtcgacgtctcgagtttacagctagctcagtcctaggtattatgctagcctcgaggaaagaggagaaagaagcttatgaaaaaaag
aggggcgtttttagggctgttgttggtttctgcctgcgcatcagttttcgctgccaataatgaaaccagcaagtcggtcactttcccaaagtgtgaaggtctggatgct
gccggaattgccgcgagcgtaaaacgtgattaccaacaaaatcgcgtggcgcgctgggctgatgatcaaaaaattgtcggtcaggccgatcccgtggcatgggt
cagtttgcaggacattcaggggaaagatgataaatggtcagtaccgctaaccgtgcgtggtaaaagtgccgatattcattaccaggtcagcgtggactgcaaagc
gggaatggcggaatatcagcggcgtcaccaccaccaccaccacgacgatgacgataagagcccgggtcagggtacccaaagcgaaaacagctgcacccact
tcccgggcaacctgccgaacatgctgcgtgacctgcgtgatgcgttcagccgtgtgaaaaccttcttccagatgaaagaccaactggataacctgctgctgaagg
agagcctgctggaggacttcaaaggttacctgggctgccaggcgctgagcgaaatgatccaattttatctggaggaagtgatgccgcaggcggagaaccaaga
cccggatattaaggcgcacgttaacagcctgggcgagaacctgaaaaccctgcgtctgcgtctgcgtcgttgccaccgtttcctgccgtgcgagaacaaaagca
aggcggtggaacaggttaagaacgcgtttaacaaactgcaagagaagggcatctataaagcgatgagcgaattcgatatctttattaactacatcgaggcgtatat
gaccatgaaaattcgtaactagtaacgagctcgatagtgctagtgtagatcgctactagagccaggcatcaaataaaacgaaaggctcagtcgaaagactgggcc
tttcgttttatctgttgtttgtcggtgaacgctctctactagagtcacactggctcaccttcgggtgggcctttctgcgtttatatactagaagcggccgctgcag.

BBa_J23110_T7g10_USP45_IL-10_rrnB_T1_T7Te (corresponding to the expression cassette comprised in strain CBT4026 in an embodiment of the present disclosure, SEQ ID NO: 151)

tctagagcttgtcgacgtctcgagtttacggctagctcagtcctaggtacaatgctagcctcgagttaagtttagaaagaggagaaagaagcttatg
aagaaaaagatcattagcgcgatcctgatgagcaccgtgattctgagcgcggcggcgccgctgagcggtgtttatgcgagcccgggtcagggtacccaaagcg
aaaacagctgcacccacttcccgggcaacctgccgaacatgctgcgtgacctgcgtgatgcgttcagccgtgtgaaaaccttcttccagatgaaagaccaactgg
ataacctgctgctgaaggagagcctgctggaggacttcaaaggttacctgggctgccaggcgctgagcgaaatgatccaattttatctggaggaagtgatgccgc
aggcggagaaccaagacccggatattaaggcgcacgttaacagcctgggcgagaacctgaaaaccctgcgtctgcgtctgcgtcgttgccaccgtttcctgccg
tgcgagaacaaaagcaaggcggtggaacaggttaagaacgcgtttaacaaactgcaagagaagggcatctataaagcgatgagcgaattcgatatctttattaac
tacatcgaggcgtatatgaccatgaaaattcgtaactagtaacgagctcgatagtgctagtgtagatcgctactagagccaggcatcaaataaaacgaaaggctca
gtcgaaagactgggcctttcgttttatctgttgtttgtcggtgaacgctctctactagagtcacactggctcaccttcgggtgggcctttctgcgtttatatactagaagc
ggccgctgcag.

BBa_J23110_BCD2_USP45_IL-10_rrnB_T1_T7Te (corresponding to the expression cassette comprised in strains CBT4028, CBT4067, CBT4068, CBT4063 and CBT4062 in the embodiments of the present disclosure, SEQ ID NO: 152)

tctagagcttgtcgacgtctcgagtttacggctagctcagtcctaggtacaatgctagcctcgaggaaagaggagaaagaagcttatgaaagcaat
tttcgtactgaaacatcttaatcatgctaaggaggttttctaatgaagaaaaagatcattagcgcgatcctgatgagcaccgtgattctgagcgcggcggcgccgct
gagcggtgtttatgcgagcccgggtcagggtacccaaagcgaaaacagctgcacccacttcccgggcaacctgccgaacatgctgcgtgacctgcgtgatgcg
ttcagccgtgtgaaaaccttcttccagatgaaagaccaactggataacctgctgctgaaggagagcctgctggaggacttcaaaggttacctgggctgccaggcg
ctgagcgaaatgatccaattttatctggaggaagtgatgccgcaggcggagaaccaagacccggatattaaggcgcacgttaacagcctgggcgagaacctga
aaaccctgcgtctgcgtctgcgtcgttgccaccgtttcctgccgtgcgagaacaaaagcaaggcggtggaacaggttaagaacgcgtttaacaaactgcaagag
aagggcatctataaagcgatgagcgaattcgatatctttattaactacatcgaggcgtatatgaccatgaaaattcgtaactagtaacgagctcgatagtgctagtgta
gatcgctactagagccaggcatcaaataaaacgaaaggctcagtcgaaagactgggcctttcgttttatctgttgtttgtcggtgaacgctctctactagagtcacac
tggctcaccttcgggtgggcctttctgcgtttatatactagaagcggccgctgcag.

BBa_J23110_GFP_USP45_IL-10_rrnB_T1_T7Te (corresponding to the expression cassette comprised in strain CBT4029 in an embodiment of the present disclosure, SEQ ID NO: 153)

tctagagcttgtcgacgtctcgagtttacggctagctcagtcctaggtacaatgctagcctcgaggaaagaggagaaagaagcttatgcgtaaag
gcgaagagaaggaggttaactgatgaagaaaaagatcattagcgcgatcctgatgagcaccgtgattctgagcgcggcggcgccgctgagcggtgtttatgcg
agcccgggtcagggtacccaaagcgaaaacagctgcacccacttcccgggcaacctgccgaacatgctgcgtgacctgcgtgatgcgttcagccgtgtgaaaa
ccttcttccagatgaaagaccaactggataacctgctgctgaaggagagcctgctggaggacttcaaaggttacctgggctgccaggcgctgagcgaaatgatc
caattttatctggaggaagtgatgccgcaggcggagaaccaagacccggatattaaggcgcacgttaacagcctgggcgagaacctgaaaaccctgcgtctgc
gtctgcgtcgttgccaccgtttcctgccgtgcgagaacaaaagcaaggcggtggaacaggttaagaacgcgtttaacaaactgcaagagaagggcatctataaa
gcgatgagcgaattcgatatctttattaactacatcgaggcgtatatgaccatgaaaattcgtaactagtaacgagctcgatagtgctagtgtagatcgctactagag
ccaggcatcaaataaaacgaaaggctcagtcgaaagactgggcctttcgttttatctgttgtttgtcggtgaacgctctctactagagtcacactggctcaccttcgg
gtgggcctttctgcgtttatatactagaagcggccgctgcag.

BBa_J23110_Luciferase_USP45_IL-10_rrnB_T1_T7Te (corresponding to the expression cassette comprised in strain CBT4030 in an embodiment of the present disclosure, SEQ ID NO: 154)

tctagagcttgtcgacgtctcgagtttacggctagctcagtcctaggtacaatgctagcctcgaggaaagaggagaaagaagcttatgattatgtcc
ggttataaggaggttaactgatgaagaaaaagatcattagcgcgatcctgatgagcaccgtgattctgagcgcggcggcgccgctgagcggtgtttatgcgagcc
cgggtcagggtacccaaagcgaaaacagctgcacccacttcccgggcaacctgccgaacatgctgcgtgacctgcgtgatgcgttcagccgtgtgaaaaccttc
ttccagatgaaagaccaactggataacctgctgctgaaggagagcctgctggaggacttcaaaggttacctgggctgccaggcgctgagcgaaatgatccaattt
tatctggaggaagtgatgccgcaggcggagaaccaagacccggatattaaggcgcacgttaacagcctgggcgagaacctgaaaaccctgcgtctgcgtctgc
gtcgttgccaccgtttcctgccgtgcgagaacaaaagcaaggcggtggaacaggttaagaacgcgtttaacaaactgcaagagaagggcatctataaagcgatg
agcgaattcgatatctttattaactacatcgaggcgtatatgaccatgaaaattcgtaactagtaacgagctcgatagtgctagtgtagatcgctactagagccaggc
atcaaataaaacgaaaggctcagtcgaaagactgggcctttcgttttatctgttgtttgtcggtgaacgctctctactagagtcacactggctcaccttcgggtgggcc
tttctgcgtttatatactagaagcggccgctgcag.

PfnrS_BCD2_USP45_IL-I0_rrnB_T1_T7Te (corresponding to the expression cassette comprised in strain CBT4078 in an embodiment of the present disclosure, SEQ ID NO: 155)

tctagagcttgtcgacgtctcgagaaaaacgccgcaaagtttgagcgaagtcaataaactctctacccattcagggcaatatctctcttctcgagga
aagaggagaaagaagcttatgaaagcaattttcgtactgaaacatcttaatcatgctaaggaggttttctaatgaagaaaaagatcattagcgcgatcctgatgagc
accgtgattctgagcgcggcggcgccgctgagcggtgtttatgcgagcccgggtcagggtacccaaagcgaaaacagctgcacccacttcccgggcaacctg
ccgaacatgctgcgtgacctgcgtgatgcgttcagccgtgtgaaaaccttcttccagatgaaagaccaactggataacctgctgctgaaggagagcctgctggag
gacttcaaaggttacctgggctgccaggcgctgagcgaaatgatccaattttatctggaggaagtgatgccgcaggcggagaaccaagacccggatattaaggc
gcacgttaacagcctgggcgagaacctgaaaaccctgcgtctgcgtctgcgtcgttgccaccgtttcctgccgtgcgagaacaaaagcaaggcggtggaacag
gttaagaacgcgtttaacaaactgcaagagaagggcatctataaagcgatgagcgaattcgatatctttattaactacatcgaggcgtatatgaccatgaaaattcgt
aactagtaacgagctcgatagtgctagtgtagatcgctactagagccaggcatcaaataaaacgaaaggctcagtcgaaagactgggcctttcgttttatctgttgt
ttgtcggtgaacgctctctactagagtcacactggctcaccttcgggtgggcctttctgcgtttatatactagaagcggccgctgcag.

PfnrS_USP45_IL-10_rrnB_T1_T7Te (corresponding to the expression cassette comprised in strains CBT4084, CBT4088, CBT4110 and CBT4111 in the embodiments of the present disclosure, SEQ ID NO: 156)

tctagagcttgtcgacgtctcgagaaaaacgccgcaaagtttgagcgaagtcaataaactctctacccattcagggcaatatctctcttctcgagga
aagaggagaaagaagcttatgaagaaaaagatcattagcgcgatcctgatgagcaccgtgattctgagcgcggcggcgccgctgagcggtgtttatgcgagcc
cgggtcagggtacccaaagcgaaaacagctgcacccacttcccgggcaacctgccgaacatgctgcgtgacctgcgtgatgcgttcagccgtgtgaaaaccttc
ttccagatgaaagaccaactggataacctgctgctgaaggagagcctgctggaggacttcaaaggttacctgggctgccaggcgctgagcgaaatgatccaattt
tatctggaggaagtgatgccgcaggcggagaaccaagacccggatattaaggcgcacgttaacagcctgggcgagaacctgaaaaccctgcgtctgcgtctgc
gtcgttgccaccgtttcctgccgtgcgagaacaaaagcaaggcggtggaacaggttaagaacgcgtttaacaaactgcaagagaagggcatctataaagcgatg
agcgaattcgatatctttattaactacatcgaggcgtatatgaccatgaaaattcgtaactagtaacgagctcgatagtgctagtgtagatcgctactagagccaggc
atcaaataaaacgaaaggctcagtcgaaagactgggcctttcgttttatctgttgtttgtcggtgaacgctctctactagagtcacactggctcaccttcgggtgggcc
tttctgcgtttatatactagaagcggccgctgcag.

Psal_sTRSV-HHRz_USP45_IL-10_rrnB_T1_T7Te (corresponding to the expression cassette comprised in strain CBT4113 in an embodiment of the present disclosure, SEQ ID NO: 235)

ggggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcgagcccgggtcagggta
cccaaagcgaaaacagctgcacccacttcccgggcaacctgccgaacatgctgcgtgacctgcgtgatgcgttcagccgtgtgaaaaccttcttccagatgaaa
gaccaactggataacctgctgctgaaggagagcctgctggaggacttcaaaggttacctgggctgccaggcgctgagcgaaatgatccaattttatctggaggaa
gtgatgccgcaggcggagaaccaagacccggatattaaggcgcacgttaacagcctgggcgagaacctgaaaaccctgcgtctgcgtctgcgtcgttgccacc
gtttcctgccgtgcgagaacaaaagcaaggcggtggaacaggttaagaacgcgtttaacaaactgcaagagaagggcatctataaagcgatgagcgaattcgat
atctttattaactacatcgaggcgtatatgaccatgaaaattcgtaactaggagctcgatagtgctagtgtagatcgctactagagccaggcatcaaataaaacgaaa
ggctcagtcgaaagactgggcctttcgttttatctgttgtttgtcggtgaacgctctctactagagtcacactggctcaccttcgggtgggcctttctgcgtttatatact
agaagcggccgctgcag.

### Examples of IL-22 sequences comprising different regulatory elements

In some embodiments, the present application provides a variety of specific IL-22 expression cassette sequences comprising different regulatory elements (such as different promoters), as shown below. In some embodiments, the present application further provides a genetically modified microorganism (e.g., EcN) comprising any of the specific IL-22 expression cassette sequences. Examples of the specific IL-22 expression cassette sequence provided in the present application are as follows:

BBa_J23119_USP45_IL-22_rrnB_T1_T7Te (corresponding to the expression cassette comprised in strain CBT4038 in an embodiment of the present disclosure, SEQ ID NO: 157)

tctagagtctcgagttgacagctagctcagtcctaggtataatgctagcctcgaggaaagaggagaaagaagcttatgaagaaaaagatcattag
cgcgatcctgatgagcaccgtgattctgagcgcggcggcgccgctgagcggtgtttatgcggcgccgatcagcagccactgccgtctggacaaaagcaacttc
cagcaaccgtatattaccaaccgtacctttatgctggcgaaagaggcgagcctggcggacaacaacaccgatgtgcgtctgatcggcgagaaactgttccacgg
cgttagcatgagcgaacgttgctacctgatgaagcaggtgctgaactttaccctggaggaagttctgttcccgcaaagcgatcgttttcagccgtatatgcaagagg
tggttccgttcctggcgcgtctgagcaaccgtctgagcacctgccacatcgaaggtgacgatctgcacattcagcgtaacgtgcaaaaactgaaggacaccgtta
aaaagctgggcgagagcggcgaaatcaaagcgattggcgaactggatctgctgtttatgagcctgcgtaacgcgtgcatttagtaacgagctcgatagtgctagt
gtagatcgctactagagccaggcatcaaataaaacgaaaggctcagtcgaaagactgggcctttcgttttatctgttgtttgtcggtgaacgctctctactagagtca
cactggctcaccttcgggtgggcctttctgcgtttatatactagaagcggccgctgcag.

BBa_J23101_USP45_IL-22_rrnB_T1_T7Te (corresponding to the expression cassette comprised in strain CBT4041 in an embodiment of the present disclosure, SEQ ID NO: 158)

tctagagcttgtcgacgtctcgagtttacagctagctcagtcctaggtattatgctagcctcgaggaaagaggagaaagaagcttatgaagaaaaa
gatcattagcgcgatcctgatgagcaccgtgattctgagcgcggcggcgccgctgagcggtgtttatgcggcgccgatcagcagccactgccgtctggacaaaa
gcaacttccagcaaccgtatattaccaaccgtacctttatgctggcgaaagaggcgagcctggcggacaacaacaccgatgtgcgtctgatcggcgagaaactg
ttccacggcgttagcatgagcgaacgttgctacctgatgaagcaggtgctgaactttaccctggaggaagttctgttcccgcaaagcgatcgttttcagccgtatatg
caagaggtggttccgttcctggcgcgtctgagcaaccgtctgagcacctgccacatcgaaggtgacgatctgcacattcagcgtaacgtgcaaaaactgaagga
caccgttaaaaagctgggcgagagcggcgaaatcaaagcgattggcgaactggatctgctgtttatgagcctgcgtaacgcgtgcatttagtaacgagctcgata
gtgctagtgtagatcgctactagagccaggcatcaaataaaacgaaaggctcagtcgaaagactgggcctttcgttttatctgttgtttgtcggtgaacgctctctact
agagtcacactggctcaccttcgggtgggcctttctgcgtttatatactagaagcggccgctgcag.

BBa_J23102_USP45_IL-22_rrnB_T1_T7Te (corresponding to the expression cassette comprised in strain CBT4042 in an embodiment of the present disclosure, SEQ ID NO: 159)

tctagagcttgtcgacgtctcgagttgacagctagctcagtcctaggtactgtgctagcctcgaggaaagaggagaaagaagcttatgaagaaaa
agatcattagcgcgatcctgatgagcaccgtgattctgagcgcggcggcgccgctgagcggtgtttatgcggcgccgatcagcagccactgccgtctggacaaa
agcaacttccagcaaccgtatattaccaaccgtacctttatgctggcgaaagaggcgagcctggcggacaacaacaccgatgtgcgtctgatcggcgagaaact
gttccacggcgttagcatgagcgaacgttgctacctgatgaagcaggtgctgaactttaccctggaggaagttctgttcccgcaaagcgatcgttttcagccgtatat
gcaagaggtggttccgttcctggcgcgtctgagcaaccgtctgagcacctgccacatcgaaggtgacgatctgcacattcagcgtaacgtgcaaaaactgaagg
acaccgttaaaaagctgggcgagagcggcgaaatcaaagcgattggcgaactggatctgctgtttatgagcctgcgtaacgcgtgcatttagtaacgagctcgat
agtgctagtgtagatcgctactagagccaggcatcaaataaaacgaaaggctcagtcgaaagactgggcctttcgttttatctgttgtttgtcggtgaacgctctctac
tagagtcacactggctcaccttcgggtgggcctttctgcgtttatatactagaagcggccgctgcag.

BBa_J23108_USP45_IL-22_rrnB_T1_T7Te (corresponding to the expression cassette comprised in strain CBT4043 in an embodiment of the present disclosure, SEQ ID NO: 160)

tctagagcttgtcgacgtctcgagctgacagctagctcagtcctaggtataatgctagcctcgaggaaagaggagaaagaagcttatgaagaaaa
agatcattagcgcgatcctgatgagcaccgtgattctgagcgcggcggcgccgctgagcggtgtttatgcggcgccgatcagcagccactgccgtctggacaaa
agcaacttccagcaaccgtatattaccaaccgtacctttatgctggcgaaagaggcgagcctggcggacaacaacaccgatgtgcgtctgatcggcgagaaact
gttccacggcgttagcatgagcgaacgttgctacctgatgaagcaggtgctgaactttaccctggaggaagttctgttcccgcaaagcgatcgttttcagccgtatat
gcaagaggtggttccgttcctggcgcgtctgagcaaccgtctgagcacctgccacatcgaaggtgacgatctgcacattcagcgtaacgtgcaaaaactgaagg
acaccgttaaaaagctgggcgagagcggcgaaatcaaagcgattggcgaactggatctgctgtttatgagcctgcgtaacgcgtgcatttagtaacgagctcgat
agtgctagtgtagatcgctactagagccaggcatcaaataaaacgaaaggctcagtcgaaagactgggcctttcgttttatctgttgtttgtcggtgaacgctctctac
tagagtcacactggctcaccttcgggtgggcctttctgcgtttatatactagaagcggccgctgcag.

BBa_J23110_USP45_IL-22_rrnB_T1_T7Te (corresponding to the expression cassette comprised in strains CBT4039, CBT4016, CBT4110 and CBT4111 in the embodiments of the present disclosure, SEQ ID NO: 161)

tctagagcttgtcgacgtctcgagtttacggctagctcagtcctaggtacaatgctagcctcgaggaaagaggagaaagaagcttatgaagaaaa
agatcattagcgcgatcctgatgagcaccgtgattctgagcgcggcggcgccgctgagcggtgtttatgcggcgccgatcagcagccactgccgtctggacaaa
agcaacttccagcaaccgtatattaccaaccgtacctttatgctggcgaaagaggcgagcctggcggacaacaacaccgatgtgcgtctgatcggcgagaaact
gttccacggcgttagcatgagcgaacgttgctacctgatgaagcaggtgctgaactttaccctggaggaagttctgttcccgcaaagcgatcgttttcagccgtatat
gcaagaggtggttccgttcctggcgcgtctgagcaaccgtctgagcacctgccacatcgaaggtgacgatctgcacattcagcgtaacgtgcaaaaactgaagg
acaccgttaaaaagctgggcgagagcggcgaaatcaaagcgattggcgaactggatctgctgtttatgagcctgcgtaacgcgtgcatttagtaacgagctcgat
agtgctagtgtagatcgctactagagccaggcatcaaataaaacgaaaggctcagtcgaaagactgggcctttcgttttatctgttgtttgtcggtgaacgctctctac
tagagtcacactggctcaccttcgggtgggcctttctgcgtttatatactagaagcggccgctgcag.

BBa_J23114_USP45_IL-22_rrnB_T1_T7Te (corresponding to the expression cassette comprised in strains CBT4040, CBT4066, CBT4069, CBT4063 and CBT4067 in the embodiments of the present disclosure, SEQ ID NO: 162)

tctagagcttgtcgacgtctcgagtttatggctagctcagtcctaggtacaatgctagcctcgaggaaagaggagaaagaagcttatgaagaaaaa
gatcattagcgcgatcctgatgagcaccgtgattctgagcgcggcggcgccgctgagcggtgtttatgcggcgccgatcagcagccactgccgtctggacaaaa
gcaacttccagcaaccgtatattaccaaccgtacctttatgctggcgaaagaggcgagcctggcggacaacaacaccgatgtgcgtctgatcggcgagaaactg
ttccacggcgttagcatgagcgaacgttgctacctgatgaagcaggtgctgaactttaccctggaggaagttctgttcccgcaaagcgatcgttttcagccgtatatg
caagaggtggttccgttcctggcgcgtctgagcaaccgtctgagcacctgccacatcgaaggtgacgatctgcacattcagcgtaacgtgcaaaaactgaagga
caccgttaaaaagctgggcgagagcggcgaaatcaaagcgattggcgaactggatctgctgtttatgagcctgcgtaacgcgtgcatttagtaacgagctcgata
gtgctagtgtagatcgctactagagccaggcatcaaataaaacgaaaggctcagtcgaaagactgggcctttcgttttatctgttgtttgtcggtgaacgctctctact
agagtcacactggctcaccttcgggtgggcctttctgcgtttatatactagaagcggccgctgcag.

PfnrS_USP45_IL-22_rrnB_T1_T7Te (corresponding to the expression cassette comprised in strains CBT4095 and CBT4098 in the embodiments of the present disclosure, SEQ ID NO: 163)

tctagagcttgtcgacgtctcgagaaaaacgccgcaaagtttgagcgaagtcaataaactctctacccattcagggcaatatctctcttctcgagga
aagaggagaaagaagcttatgaagaaaaagatcattagcgcgatcctgatgagcaccgtgattctgagcgcggcggcgccgctgagcggtgtttatgcggcgc
cgatcagcagccactgccgtctggacaaaagcaacttccagcaaccgtatattaccaaccgtacctttatgctggcgaaagaggcgagcctggcggacaacaac
accgatgtgcgtctgatcggcgagaaactgttccacggcgttagcatgagcgaacgttgctacctgatgaagcaggtgctgaactttaccctggaggaagttctgt
tcccgcaaagcgatcgttttcagccgtatatgcaagaggtggttccgttcctggcgcgtctgagcaaccgtctgagcacctgccacatcgaaggtgacgatctgca
cattcagcgtaacgtgcaaaaactgaaggacaccgttaaaaagctgggcgagagcggcgaaatcaaagcgattggcgaactggatctgctgtttatgagcctgc
gtaacgcgtgcatttagtaacgagctcgatagtgctagtgtagatcgctactagagccaggcatcaaataaaacgaaaggctcagtcgaaagactgggcctttcgt
tttatctgttgtttgtcggtgaacgctctctactagagtcacactggctcaccttcgggtgggcctttctgcgtttatatactagaagcggccgctgcag.

### Examples of IL-17A sequences comprising different regulatory elements

An example of the specific IL-17A expression cassette sequence provided in the present application is as follows:
Psal_USP45_IL-17A_rrnB_T1_T7Te (corresponding to the expression cassette comprised in strain CBT4114 in an embodiment of the present disclosure, SEQ ID NO: 236)

gggggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcgggtattacaatcccgcg
taatccgggttgtccgaatagtgaagataaaaattttccgcgtacagttatggttaatctgaatattcataaccgtaacacaaacaccaatccgaaacgtagtagcgat
tattataatcgtagcacaagcccgtggaatctgcatcgtaacgaagatccggaacgttatccaagcgttatctgggaagcaaaatgccgccatctgggttgtatcaa
tgcagatggtaacgttgattatcatatgaatagcgtgccgattcagcaggaaattctggttctgcgtcgtgaaccgccgcattgtccgaatagctttcgtctggaaaa
aattctggttagcgttggttgtacctgtgttaccccgattgttcatcatgttgcacatcatcatcatcatcattaagagctcgatagtgctagtgtagatcgctactagagc
caggcatcaaataaaacgaaaggctcagtcgaaagactgggcctttcgttttatctgttgtttgtcggtgaacgctctctactagagtcacactggctcaccttcgggt
gggcctttctgcgtttatatactagaagcggccgctgcag.

### Examples of IL-19 sequences comprising different regulatory elements

An example of the specific IL-19 expression cassette sequence provided in the present application is as follows:
Psal_USP45_IL-19_rrnB_T1_T7Te (corresponding to the expression cassette comprised in strain CBT4114 in an embodiment of the present disclosure, SEQ ID NO: 237)

gggggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcgctgcgtcgttgtctgatt
agcaccgatatgcatcatattgaagaaagctttcaggaaattaaacgtgccattcaggcaaaagatacctttccgaatgttaccattctgagcaccctggaaaccctg
cagattatcaaaccactggatgtttgttgtgttaccaaaaatctgctggccttttatgttgatcgtgtttttaaagatcatcaggaaccgaatccgaaaattctgcgtaaaa
ttagtagcattgccaatagctttctgtatatgcagaaaaccctgcgccagtgtcaggaacagcgtcagtgtcattgtcgtcaggaagcaaccaatgcaacccgtgtg
attcatgataactatgatcagctggaagttcatgcagcagcgattaaaagcctgggtgaactggatgtgtttctggcatggattaataaaaatcatgaagtgatgttta
gcgcacatcatcatcatcatcattaagagctcgatagtgctagtgtagatcgctactagagccaggcatcaaataaaacgaaaggctcagtcgaaagactgggcct
ttcgttttatctgttgtttgtcggtgaacgctctctactagagtcacactggctcaccttcgggtgggcctttctgcgtttatatactagaagcggccgctgcag.

### Examples of IL-23 sequences comprising different regulatory elements

An example of the specific IL-23 expression cassette sequence provided In the present application is as follows:
Psal_USP45_IL-12p40_linkerl_IL-23p19_rrnB_T1_T7Te (corresponding to the expression cassette comprised in strain CBT4114 in an embodiment of the present disclosure, SEQ ID NO: 238)

gggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcgatctgggaactgaaaaa
ggatgtttatgtggttgaactggattggtatccggatgcaccgggcgaaatggtggttctgacgtgtgatacgccggaagaagatggtattacctggacactggatc
agagcagcgaagttctgggcagtggtaaaaccctgacgattcaggttaaagaatttggtgatgcaggtcagtatacctgtcataaaggtggtgaagttctgagcca
ttccctgctgctgctgcataaaaaggaagatggtatctggagcaccgatattctgaaagatcagaaagaaccgaaaaacaaaacctttctgcgttgcgaagcgaaa
aattattcaggccgttttacctgttggtggctgacgaccattagcacagatctgacctttagcgtgaaaagcagccgtggtagcagcgatccgcagggtgttacttgt
ggtgcagcaacgctgtctgcagaacgtgttcgtggtgataataaagaatatgaatatagcgtggaatgccaggaagattcagcatgtccggcagcagaagaaag
cctgccgattgaagtgatggttgatgcagttcataaactgaaatatgaaaactataccagcagcttttttatccgtgatattattaaaccggaccctccgaaaaatctgc
agctgaaacctctgaaaaatagtcgtcaggtggaagtgtcctgggaatatccggatacctggagcaccccacatagctattttagtctgaccttttgtgttcaggttca
gggcaaatcaaaacgcgaaaaaaaagatcgtgtttttaccgataaaaccagcgcaaccgttatttgtcgtaaaaatgcaagcattagcgttcgtgcacaggatcgtt
attatagcagcagctggagcgaatgggcaagcgttccgtgtagcggtagcaccagcggtagcggtaaaccgggtagcggtgaaggtagcaccaaaggtcgtg
cagttccgggtggtagcagcccggcatggacccagtgtcagcagctgagccagaaactgtgtaccctggcatggagcgcacatccgctggttggtcatatggat
ctgcgtgaagaaggtgatgaagaaaccaccaatgatgttccgcatattcagtgtggtgatggttgtgatccgcagggtctgcgtgataatagccagttttgtctgca
gcgtattcatcagggtctgattttttatgaaaaactgctgggtagcgatatttttaccggtgaaccgagcctgctgccggatagcccggttggtcagctgcatgcaag
cctgctgggtctgagccagctgctgcagccggaaggtcatcattgggaaacccagcagattccgagcctgagcccgagccagccgtggcagcgtctgctgctg
cgttttaaaattctgcgtagcctgcaggcatttgttgcagttgcagcacgtgtttttgcacatggtgcagcaaccctgagcccgcatcatcatcatcatcattaagagct
cgatagtgctagtgtagatcgctactagagccaggcatcaaataaaacgaaaggctcagtcgaaagactgggcctttcgttttatctgttgtttgtcggtgaacgctc
tctactagagtcacactggctcaccttcgggtgggcctttctgcgtttatatactagaagcggccgctgcag.

### Examples of IL-35 sequences comprising different regulatory elements

An example of the specific IL-35 expression cassette sequence provided In the present application is as follows:
Psal_USP45_EBI3_linker2_IL-12p35_rmB_T1_T7Te (corresponding to the expression cassette comprised in strain CBT4114 in an embodiment of the present disclosure, SEQ ID NO: 239)

ggggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcgcgcaaaggtccgccgg
cagcactgaccctgccgcgtgttcagtgtcgtgcatcccgctatccgattgcagttgattgtagttggaccctgccgccggcaccgaattccacctcaccggttagc
tttattgctacctatcgtctgggtatggccgcacgtggtcattcatggccttgtctgcagcagaccccgaccagcacgagctgtaccatcaccgatgttcagctgtttt
ctatggcaccgtatgttctgaatgttacggcagttcatccgtggggttcaagcagctcttttgttccatttattaccgaacatatcatcaaaccggaccctccggaaggt
gttcgtctgagcccgctggcagaacgtcagctgcaggttcagtgggaaccgccaggttcatggccgtttcctgaaatttttagcctgaaatattggattcgttataaa

cgtcagggtgcggcccgttttcatcgtgttggtcctattgaagcaacctcatttattctgcgtgcggtccgtcctcgtgcccgttattatgttcaggtcgccgcacagg
atctgaccgattatggtgaactgagcgattggtcactgccggcaaccgcgaccatgtccctgggtaaaggtggcggcggtagcggcggcggtggtagtggtgg
cggcggctctggcggcggcggttcccgtaacctgccggttgcgaccccggaccctggcatgtttccgtgtctgcatcattctcagaatctgctgcgtgcagtctct
aatatgctgcagaaagcacgtcagaccctggaattttatccgtgtaccagtgaagaaatcgatcatgaagatattaccaaagataaaaccagcaccgtggaagcat
gtctgccgctggaactgaccaaaaacgaaagctgtctgaacagccgcgaaaccagttttattacaaatggtagttgtctggcaagccgcaaaaccagctttatgat
ggcgctgtgtctgagctcaatttatgaagatctgaaaatgtatcaggtggaatttaaaaccatgaatgccaaactgctgatggaccctaaacgtcagatttttctggat
cagaatatgctggcagttatcgatgaactgatgcaggcactgaactttaatagcgaaaccgttccgcagaaaagctccctggaagaaccggatttttataaaacca
aaatcaaactgtgcatcctgctgcatgcatttcgtattcgtgcagttaccattgatcgtgtgatgagctatctgaatgcaagccatcatcatcatcatcattaagagctc
gatagtgctagtgtagatcgctactagagccaggcatcaaataaaacgaaaggctcagtcgaaagactgggcctttcgttttatctgttgtttgtcggtgaacgctct
ctactagagtcacactggctcaccttcgggtgggcctttctgcgtttatatactagaagcggccgctgcag.

### Examples of IL-37 sequences comprising different regulatory elements

An example of the specific IL-37 expression cassette sequence provided in the present application is as follows:
Psal_USP45_IL-37_rrnB_T1_T7Te (corresponding to the expression cassette comprised in strain CBT4114 in an embodiment of the present disclosure, SEQ ID NO: 240)

ggggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcggttcataccagtccgaa
agtgaaaaatctgaatccgaaaaagtttagcatccatgatcaggatcataaagttctggtactggatagcggtaatctgattgcagtgccggataaaaattatattcgt
ccggaaattttttttgccctggcaagcagcctgagttcagcctcagcggaaaaaggtagcccgattctgctgggtgttagtaaaggtgaattttgtctgtattgtgata
aagataaaggccagagccatccgagtctgcagctgaaaaaggaaaaactgatgaaactggcagcacagaaagaaagcgctcgtcgtccgtttattttttatcgtg
cacaggtgggtagctggaatatgctggaaagcgcagcccatccaggttggtttatttgcaccagctgcaattgtaatgaaccggtgggtgttaccgataaatttgaa
aatcgtaaacatatcgaatttagctttcagccggtttgtaaagcagaaatgagcccgagcgaagttagtgatcatcatcatcatcatcattaagagctcgatagtgcta
gtgtagatcgctactagagccaggcatcaaataaaacgaaaggctcagtcgaaagactgggcctttcgttttatctgttgtttgtcggtgaacgctctctactagagtc
acactggctcaccttcgggtgggcctttctgcgtttatatactagaagcggccgctgcag.

### Examples of TGF-β sequences comprising different regulatory elements

An example of the specific TGF-β expression cassette sequence provided In the present application is as follows:
Psal_sTRSV-HHRz_USP45_TGF-β_rrnB_T1_T7Te (corresponding to the expression cassette comprised in strain CBT4114 in an embodiment of the present disclosure, SEQ ID NO: 241)

ggggcctcgcttgggttattgctggtgcccggccgggcgcaatattcatgttgatgatttattatatatcgagtggtgtatttatttatattgtttgctccg
ttaccgttattaacagctgtcaccggatgtgctttccggtctgatgagtccgtgaggacgaaacagcctctacaaataattttgtttaatactagagaaagaggggaa
atactagatgaagaaaaagatcattagcgcgatcctgatgagcaccgtgattctgagcgcggcggcgccgctgagcggtgtttatgcggcactggataccaatta
ttgtttttcaagcaccgaaaaaaattgttgtgttcgtcagctgtatattgattttcgtaaagatctgggttggaaatggattcatgaaccgaaaggttatcatgcaaattttt
gtctgggtccgtgtccgtatatttggagcctggatacccagtatagcaaagttctggcactgtataatcagcataatccgggtgcaagcgcagcaccgtgttgtgttc
cgcaggcactggaaccgctgccgattgtttattatgttggtcgtaaaccgaaagttgaacagctgagcaatatgattgttcgtagctgtaaatgtagccatcatcatca
tcatcattaagagctcgatagtgctagtgtagatcgctactagagccaggcatcaaataaaacgaaaggctcagtcgaaagactgggcctttcgttttatctgttgttt
gtcggtgaacgctctctactagagtcacactggctcaccttcgggtgggcctttctgcgtttatatactagaagcggccgctgcag.

In some embodiments, the present application further provides a variety of genetically modified EcN strains having genotypes as shown in Table 10 below. As shown in Table 10, each strain is named by its genotype name, wherein EcN represents *E. coli Nissle 1917,* ΔmaeB represents an insertion at the maeB site of the strain, and the underlined part represents the name of the specific expression cassette described in the present application. For example, BBa_J23101_USP45_IL-10, indicates that the expression cassette comprises the BBa_J23101 promoter, which is operably linked to the coding sequence of the USP45 signal peptide and the coding sequence of the IL-10 polypeptide. The nucleic acid sequences of the specific expression cassettes are set forth in SEQ ID NO: 139 to SEQ ID NO: 163 of the present application. Some strains also lack one or more outer membrane proteins, which are reflected in their genotype names, for example, ΔLPP represents the missing of outer membrane protein LPP. Some strains also overexpress one or more molecular chaperones, as reflected in their genotype names, for example, ΔyicS/nepI::BBa_J23114_dsbA_dsbC_dnaK_dnaJ_grpE represents that an expression cassette expressing the molecular chaperone_dsbA_dsbC_dnaK_dnaJ_grpE is inserted into the yicS/nepI site, wherein the expression cassette has the BBa_J23114 promoter sequence which may be operably connected. Based on the description of the present application and the genotype names listed in Table 10, those skilled in the art can know the specific genetic modifications in each strain, and determine the above-mentioned specific genetic modifications, for example, performing detection and identification by designing primers or probes targeting specific insertion sequences or sequences at insertion sites.

**Table 10 Genotypes of strains of the present invention**

| Strains | Genotypes |
|---|---|
| CBT4003 | EcNΔmaeB::BBa_J23101_USP45_IL-10 |
| CBT4004 | EcNΔmaeB::BBa_J23108_USP45_IL-10 |
| CBT4005 | EeNΔmaeB::BBa_J23110_USP45_IL-10 |
| CBT4007 | EeNΔmaeB::BBa_J23101_DsbA_IL-10 |
| CBT4009 | EcNΔmaeB::BBa_J23101_OmpA_IL-10 |
| CBT4011 | EcNΔmaeB::BBa_J23101_PelB_IL-10 |
| CBT4013 | EeNΔmaeB::BBa_J23101_YebF_IL-10 |
| CBT4016 | EcNΔkefB::BBa_J23110_USP45_IL-22ΔLPP |
| CBT4020 | EcNΔmaeB::BBa_J23110_USP45_IL-10ΔLPP |
| CBT4026 | EcNΔmaeB::BBa_J23110_T7g10_USP45IL-10 |
| CBT4028 | EcNΔmaeB::BBa_J23110_BCD2_USP45_IL-10 |
| CBT4029 | EcNΔmaeB::BBa_J23110_GFP_USP45_IL-10 |
| CBT4030 | EcNΔmaeB::BBa_J23110_luciferase_USP45_IL-10 |
| CBT4038 | EcNΔmaeB::BBa_J23119_USP45_IL-22 |
| CBT4039 | EcNΔkefB::BBa_J23110_USP45_IL-22 |
| CBT4040 | EcNΔkefB::BBa_J23114_USP45_IL-22 |
| CBT4041 | EcNΔkefB::BBa_J23101_USP45_IL-22 |
| CBT4042 | EcNΔkefB::BBa_J23102_USP45_IL-22 |
| CBT4043 | EcNΔkefB::BBa_J23108_USP45_IL-22 |
| CBT4062 | EcNAmaeB::BBa_J23110_BCD2_USP45_IL-10ALPPAyicS/nepI::BBa_J23114_dsbA_dsb C_dnaK_dnaJ_grpEΔyieN::BBa_J23114_groES_groEL_tig_fkpA_surA |
| CBT4063 | EeNΔkefB::BBa_J23114_USP45_IL-22ΔmaeB::BBa_J23110_BCD2_USP45_IL-10ΔLPP ΔyicS/nepI::BBa_J23114_dsbA_dsbC_dnaK_dnaJ_grpEΔyieN::BBa_J23114_groES_groE L_tig_fkpA_surA |
| CBT4066 | EcNAkefB::BBa_J23114_USP45_IL-22ΔLPPΔyicS/nepI::BBa_J23114_dsbA_dsbC_dnaK _dnaJ_grpEΔyieN::BBa_J23114_groES_groEL_tig_fkpA_surA |
| CBT4067 | EcNΔmaeB::BBa_J23110_BCD2_USP45_IL-10ΔkefB::BBa_J23114_USP45_IL-22ΔagaI/ rsmI::BBa_J23101_USP45_Amuc_1100ΔmalP/T::BBa_J23110_USP45_Amuc_1100ΔLPP ΔyicS/nepI::BBa_J23114_dsbA_dsbC_dnaK_dnaJ_grpEΔyieN::BBa_J23114_groES_groE L_tig_fkpA_surA |
| CBT4068 | EcNΔmaeB::BBa_J23110_BCD2_USP45_IL-10ΔagaI/rsmI::BBa_J23101_USP45_Amuc_ 1100ΔmalP/T::BBa_J23110_USP45_Amuc_1100ΔLPPΔyicS/nepI::BBa_J23114_dsbA_ds bC_dnaK_dnaJ_grpEΔyieN::BBa_J23114_groES_groEL_tig_fkpA_surA |
| CBT4069 | EeNΔkefB::BBa_J23114_USP45_IL-22Δagal/rsmI::BBa_J23101_USP45_Amuc_1100Δm alP/T::BBa_J23110_USP45_Amuc_1100ΔLPPAyicS/nepI::BBa_J23114_ dsbA dsbC dnaK dnaJ grpEΔyieN::BBa_J23114_groES_groE_tig_fkpAsurA |
| CBT4070 | EcNΔagaI/rsmI::BBa_J23101_USP45_Amuc_1100ΔmalP/T::BBa_J23110_USP45_Amuc_ 1100ΔLPPΔyicS/nepI::BBa_J23114_dsbA_dsbC_dnaK_dnaJ_grpEΔyieN::BBa_J23114_groES_groEL_tig_fkpA_surA |
| CBT4071 | EcNΔmaeB::BBa_J23110_USP45_IL-10AtolQ |
| CBT4072 | EcNΔmaeB::BBa_J23110_USP45_IL-10ΔtolR |
| CBT4073 | EcNΔmaeB::BBa_J23110_USP45_IL-10ΔtolA |
| CBT4074 | EcNΔmaeB::BBa_J23110_USP45_IL-10Δpal |
| CBT4075 | EeNAmaeB::BBa_J23110_BCD2_USP45_IL-10ΔLPPΔmrcAΔyicS/nepI::BBa_J23114_ds bA_dsbC_dnaK_dnaJ_grpEΔyieN::BBa_J23114_groES_groEL_tig_fkpA_surA |
| CBT4076 | EcNΔmaeB::BBa_J23110_BCD2_USP45_IL-10ΔLPPΔompTΔyicS/nepI::BBa_J23114_ds bA_dsbC_dnaK_dnaJ_grpEΔyieN::BBa_J23114_groES_groEL_tig_fkpA_surA |
| CBT4077 | EeNΔmaeB::BBa_J23110_BCD2_USP45_IL-10ΔLPPΔompTΔmrcAΔyicS/nepI::BBa_J23 114_dsbA_dsbC_dnaK_dnaJ_grpEΔyieN::BBa_J23114_groES_groEL_tig_fkpA_surA |
| CBT4078 | EcNΔmaeB::PfnrS_BCD2_USP45_IL-10ΔLPPΔyicS/nepI::BBa_J23114_dsbA_dsbC_dna K_dnaJ_grpEΔyieN::BBa_J23114_groES_groEL_tig_fkpA_surA |
| CBT4080 | EcNΔlldD::PfnrS_USP45_Amuc_1100ΔmaeA::PfnrS_USP45_Amuc_1100ΔLPP |
| CBT4084 | EeNΔmaeB::PfnrS_USP45_IL-10ΔyicS/nepI::BBa_J23114_dsbA_dsbC_dnaK_dnaJ_grpE ΔyieN/trkD::BBa_J23114_groES_groEL_tig_fkpA_surAΔLPPΔmrcA |
| CBT4088 | EcNΔmaeB::PfnrS_USP45_IL-10ΔagaI/rsmI::PfnrS_USP45_Amue_1100ΔyicS/nepI::BBa _J23114_dsbA_dsbC_dnaK_dnaJ_grpEΔyieN/trkD::BBa_J23114_groES_groEL_tig_ fkpA_surAΔLPPΔmrcA |
| CBT4095 | EcNΔkefB::PfnrS_USP45_IL-22ΔyicS/nepI::BBa_J23114_dsbA_dsbC_dnaK_dnaJ_grpEΔ yieN::BBa_J23114_groES_groEL_tig_fkpAsurAΔLPPΔmrcA |
| CBT4096 | EcNΔagaI/rsmI::PfnrS_USP45_Amuc_1100ΔyicS/nepI::BBa_J23114_dsbA_dsbC_dnaK_ dnaJ_grpEΔyieN/trkD::BBa_J23114_groES_groEL_tig_fkpAsurAΔLPPΔmrcA |
| CBT4098 | EcNΔkefB::PfnrS_USP45_IL-22ΔagaI/rsmI::PfnrS_USP45_Amuc_1100ΔyicS/nepI::BBa_ J23114_dsbA_dsbC_dnaK_dnaJ_grpEΔyieN/trkD::BBa_J23114_groES_groEL_tig_ fkpAsurAΔLPPΔmrcA |
| CBT4101 | EeNΔagaI/rsmI::BBa_J23101_USP45_Amuc_1100(Y259A) |
| CBT4102 | EcNΔagaI/rsmI::BBa_J23110_USP45_Amuc_1100(Y259A)ΔmalP/T::BBa_J23110_USP4 5_Amuc_1100(Y259A) |
| CBT4103 | EcNΔagaI/rsmI::BBa_J23101_USP45_Amuc_1100(Y259A)ΔmalP/T::BBa_J23101_USP4 5_Amuc_1100(Y259A)ΔpflB::BBa_J23101_USP45_Amuc_1100(Y259A) |
| CBT4105 | EcNΔagaI/rsmI::BBa_J23110_USP45_Amuc_1100ΔmalP/T::BBa_J23110_USP45_Amuc_ 1100ΔLPP |
| CBT4106 | EcNΔagaI/rsmI::BBa_J23110_USP45_Amuc_1100AmalP/T::BBa_J23110_USP45_Amuc_ 1100ΔLPPΔompT |
| CBT4107 | EeNΔagaI/rsmI::BBa_J23110_USP45_Amuc_1100(Y259A) |
| CBT4108 | EcNΔagaI/rsmI::BBa_J23110_USP45_Amuc_1100(Y259A)ΔmalP/T::BBa_J23110_USP4 5_Amuc_1100(Y259A)ΔpflB::BBa_J23101_USP45_Amuc_1100(Y259A) |
| CBT4109 | EcNΔagaI/rsmI::BBa_J23110_USP45_Amuc_1100(Y259A)ΔmalP/T::BBa_J23110_USP4 5_Amuc_1100(Y259A)ΔpflB::BBa_J23110_USP45_Amuc_1100(Y259A) |
| CBT4110 | EcNΔmaeB::PfnrS_USP45_IL-10ΔkefB::BBa_J23110_USP45_IL-22ΔyicS/nepI::BBa_J2 3114_dsbA_dsbC_dnaK_dnaJ_grpEΔyieN/trkD::BBa_J23114_groES_groEL_tig_ fkpAsurAΔLPPΔmrcA |
| CBT4111 | EeNΔmaeB::PfnrS_USP45_IL-10ΔkefB::BBa_J23110_USP45_IL-22ΔagaI/rsmI::PfnrS_U SP45_Amuc_110ΔyicS/nepI::BBa_J23114_ dsbA_dsbC_dnaK_dnaJ_grpEΔyieN/trkD::BBa_J23114_groES_groEL_tig_ fkpAsurAΔLPPΔmrcA |
| CBT4112 | EcNΔmaeB::BBa_J23110 _USP45_IL-10ΔpalΔmrcA |
| CBT4113 | EeNΔmaeB::Psal-sTRSV-HHRz_USP45_IL-10-rrnB_T1_T7Te_PlacIQ_NahRAM_terΔyi cS/nepI::BBa_J23114_dsbA_dsbC_dnaK_dnaJ_grpEΔyieN/trkD::BBa_J23114_groES_groEL_tig_fkpAsurAΔLPPΔmrcA |
| CBT4114 | EcNΔyicS/nepI::BBa_J23114_dsbA_dsbC_dnaK_dnaJ_grpEΔyieN/trkD::BBa_J23114_gr oES_groEL_tig_fkpA_surAΔLPPΔmrcA |

### III. Compositions and kits

In another aspect, the present invention provides a composition comprising: (a) the genetically modified microorganism of the present invention; and (b) a physiologically acceptable carrier.

As used herein, "composition" refers to a preparation of the genetically engineered bacterium of the present invention with other components such as a physiologically suitable carrier. The expression "physiologically acceptable carrier" is used interchangeably to refer to a carrier or diluent that does not cause significant irritation to the organism and does not eliminate the biological activity and properties of the genetically modified microorganism to which it is administered.

The bacterium may be present in the composition in an amount ranging from about 10⁴ to about 10¹³ colony forming units (CFU). For example, an effective amount of the microorganism may be an amount of about 10⁵ CFU to about 10¹³ CFU, preferably about 10⁶ CFU to about 10¹³ CFU, preferably about 10⁷ CFU to about 10¹² CFU, more preferably about 10⁸ CFU to about 10¹² CFU. The microorganism may be living cells or dead cells. The effectiveness of the microorganism correlates with the presence of Amuc_1100, IL-10 and/or IL-22 provided herein.

In some embodiments, the composition disclosed herein may be formulated for oral administration and may be a nutritional or nourishing composition, such as a food, a food supplement, a feed or a feed supplement, for example a dairy product, such as a fermented dairy product including yogurt or yogurt drinks. In this case, the composition may comprise a nutritionally acceptable carrier, which may be a suitable food base. Those skilled in the art are aware of a variety of formulations that may encompass live or dead microorganisms and may be presented as food supplements (e.g., pills, tablets, etc.) or functional foods (e.g., beverages, fermented yogurt, etc.). The composition disclosed herein may further be formulated as a medicament in capsules, pills, or liquid solutions, for example, being formulated as encapsulated freeze-dried bacteria. In some embodiments, the composition is a probiotic composition.

The composition disclosed herein may be formulated for a single administration or for multiple administrations to be effective for a given subject. For example, a single administration is effective to substantially reduce monitored symptoms of the targeted disease condition in a mammalian subject administered with the composition.

In some embodiments, the composition is formulated such that a single oral dose contains at least or at least about 1×10⁴ CFU of bacterial entities and/or fungal entities. A single oral dose typically contains about or at least 1×10⁴, 1×10⁵, 1×10⁶, 1×10⁷, 1×10⁸, 1×10⁹, 1×10¹⁰, 1×10¹¹, 1×10¹², or 1×10¹³ of bacterial entities and/or fungal entities. If it is known that, for example, the concentration of cells of a given strain or the total concentration of all strains is, for example, 1×10⁴, 1×10⁵, 1×10⁶, 1×10⁷, 1×10⁸, 1×10⁹, 1×10¹⁰, 1×10¹¹, 1×10¹², or 1×10¹³ viable bacterial entities (e.g. CFU) per gram of composition or per dose administered. In some embodiments, the composition comprises 1×10⁸-1×10¹² CFU of the genetically modified microorganism of the present invention.

In some formulations, by mass, the composition comprises at least about 0.5%, 1%, 2%, 5%, 10%, 20%, 30%, 40%, 50%, 60%, 70%, 80%, 90% or greater than 90% of the microorganism of the present invention. In some formulations, by mass, the administration amount of the microorganism provided herein does not exceed 200, 300, 400, 500, 600, 700, 800, 900 mg or 1, 1.1, 1.2, 1.3, 1.4, 1.5, 1.6, 1.7, 1.8 or 1.9 grams.

The physiologically acceptable carrier for the composition disclosed herein may include, for example, a physiologically acceptable liquid, gel or solid carrier, an aqueous vehicle, a non-aqueous vehicle, an antimicrobial agent, an isotonic agent, a buffer, an antioxidant, a suspending/dispersing agent, a sequestering/chelating agent, a diluent, an adjuvant, an excipient or a non-toxic auxiliary substance, other components known in the art, or various combinations thereof. In some embodiments, the composition described in the present disclosure is a liquid formulation, a solid formulation, or a semi-solid formulation. In some embodiments, the liquid formulation is selected from the group consisting of a solution product and a suspension product.

To further illustrate, the aqueous vehicle may include, for example, sodium chloride injection, Ringer's injection, isotonic dextrose injection, sterile water injection, or dextrose and lactated Ringer's injection. The non-aqueous vehicle may include, for example, non-volatile oil derived from plant, cottonseed oil, corn oil, sesame oil, or peanut oil. The antimicrobial agent may be in a bacteriostatic or fungistatic concentration, and/or may be added to a composition in a multi-dose container containing phenol or cresol, mercury, benzyl alcohol, chlorobutanol, methyl paraben and propyl paraben, thimerosal, benzalkonium chloride and benzethonium chloride. The isotonic agent may include sodium chloride or dextrose. The buffer may include phosphate or citrate buffers. The antioxidant may include sodium bisulfate. The suspending/dispersing agent may include sodium carboxymethylcellulose, hydroxypropyl methylcellulose or polyvinylpyrrolidone. The chelating agent may include ethylenediaminetetraacetic acid (EDTA) or ethylene glycol tetraacetic acid (EGTA), ethanol, polyethylene glycol, propylene glycol, sodium hydroxide, hydrochloric acid, citric acid or lactic acid. A suitable excipient may include, for example, water, physiological saline, dextrose, glycerol or ethanol. A suitable non-toxic auxiliary substance may include, for example, a wetting or emulsifying agent, a pH buffer, a stabilizer, a solubility enhancer or an agent such as sodium acetate, anhydrous sorbitan monolaurate, triethanolamine oleate or cyclodextrin.

In some embodiments, the composition provided herein may be a pharmaceutical composition. In some embodiments, the composition provided herein may be a food supplement. The composition provided herein may include the genetically modified microorganism provided herein and a pharmaceutically, nutritionally/alimentarily, or physiologically acceptable carrier. The preferred dosage form will depend on the intended mode of administration and (therapeutic) application. The carrier may be any compatible physiologically acceptable non-toxic substance suitable for delivering the genetically modified microorganism provided herein to the gastrointestinal tract of a mammal (e.g., a human), preferably the intestinal mucosal barrier of the mammal (more preferably the colonic mucosal barrier). In some embodiments, the dosage form of the pharmaceutical composition is selected from the group consisting of dustpowder, powder, tablet, sugar-coated form, capsule, granule, suspension, solution, syrup, drop, sublingual tablet and a combination thereof.

The composition may be a liquid solution, suspension, emulsion, pill, capsule, tablet, sustained release preparation or powder. The oral preparation may comprise a standard carrier such as pharmaceutical grades of mannitol, lactose, starch, magnesium stearate, polyvinylpyrrolidone, sodium saccharin, cellulose, and magnesium carbonate. In another aspect, the present invention provides a kit comprising the composition of the present invention.

If necessary, the kit may further comprise one or more of various conventional pharmaceutical kit components, for example, a container or an additional container containing one or more pharmaceutically acceptable carriers, etc., such that it will be obvious to those skilled in the art. The kit may further comprise an instruction as an insert page or a label indicating the amounts of components to be administered, instructions for administration, and/or instructions for mixing the components.

### IV. Indications and treatment methods

In another aspect, the present invention provides a method for treating or preventing an inflammatory disease or an autoimmune disease in a subject in need thereof, comprising: administering to the subject an effective amount of the genetically modified microorganism or the composition of the present invention. In some embodiments, the genetically modified microorganism expresses at least one exogenous gene selected from the group consisting of: an exogenous gene encoding Amuc_1100 polypeptide, an exogenous gene encoding IL-10 polypeptide, an exogenous gene encoding IL-22 polypeptide, and a combination thereof.

In an aspect, the present invention further provides a method for improving the therapeutic effect of a drug in a subject, comprising administering to the subject an effective amount of the genetically modified microorganism or the composition of the present invention, wherein the drug is a drug for treating an inflammatory disease or an autoimmune disease. In some embodiments, the genetically modified microorganism expresses at least one exogenous gene selected from the group consisting of: an exogenous gene encoding Amuc_1100 polypeptide, an exogenous gene encoding IL-10 polypeptide, an exogenous gene encoding IL-22 polypeptide, and a combination thereof.

In another aspect, the present invention further provides use of the genetically modified microorganism or the composition of the present invention in the manufacture of a medicament for treating or preventing an inflammatory disease or an autoimmune disease.

In some embodiments, the inflammatory or autoimmune disease is inflammatory bowel disease (IBD). In some embodiments, the genetically modified microorganism comprises an exogenous gene encoding Amuc_1100 polypeptide and an exogenous gene encoding IL-10 polypeptide, respectively. In some embodiments, the genetically modified microorganism comprises an exogenous gene encoding IL-10 polypeptide and an exogenous gene encoding IL-22 polypeptide, respectively. In some embodiments, the genetically modified microorganism comprises an exogenous gene encoding Amuc_1100 polypeptide and an exogenous gene encoding IL-22 polypeptide, respectively. In some embodiments, the genetically modified microorganism comprises an exogenous gene encoding IL-10 polypeptide. In some embodiments, the genetically modified microorganism comprises an exogenous gene encoding Amuc_1100 polypeptide. In some embodiments, the genetically modified microorganism comprises an exogenous gene encoding IL-22 polypeptide. In some embodiments, the genetically modified microorganism comprises exogenous genes encoding Amuc_1100 polypeptide, IL-10 polypeptide and IL-22 polypeptide respectively.

In some embodiments, the inflammatory or autoimmune disease is systemic lupus erythematosus (SLE). In some embodiments, the genetically modified microorganism comprises an exogenous gene encoding Amuc_1100 polypeptide and an exogenous gene encoding IL-10 polypeptide, respectively. In some embodiments, the genetically modified microorganism comprises an exogenous gene encoding encoding IL-10 polypeptide and an exogenous gene encoding IL-22 polypeptide, respectively. In some embodiments, the genetically modified microorganism comprises an exogenous gene encoding Amuc_1100 polypeptide and an exogenous gene encoding IL-22 polypeptide, respectively. In some embodiments, the genetically modified microorganism comprises an exogenous gene encoding IL-10 polypeptide. In some embodiments, the genetically modified microorganism comprises an exogenous gene encoding Amuc_1100 polypeptide. In some embodiments, the genetically modified microorganism comprises an exogenous gene encoding IL-22 polypeptide. In some embodiments, the genetically modified microorganism comprises exogenous genes encoding Amuc_1100 polypeptide, IL-10 polypeptide and IL-22 polypeptide respectively.

In some embodiments, the inflammatory or autoimmune disease is arthritis. In some embodiments, the genetically modified microorganism comprises an exogenous gene encoding Amuc_1100 polypeptide and an exogenous gene encoding IL-10 polypeptide, respectively. In some embodiments, the genetically modified microorganism comprises an exogenous gene encoding IL-10 polypeptide and an exogenous gene encoding IL-22 polypeptide, respectively. In some embodiments, the genetically modified microorganism comprises an exogenous gene encoding Amuc_1100 polypeptide and an exogenous gene encoding IL-22 polypeptide, respectively. In some embodiments, the genetically modified microorganism comprises an exogenous gene encoding IL-10 polypeptide. In some embodiments, the genetically modified microorganism comprises an exogenous gene encoding Amuc_1100 polypeptide. In some embodiments, the genetically modified microorganism comprises an exogenous gene encoding IL-22 polypeptide. In some embodiments, the genetically modified microorganism comprises exogenous genes encoding Amuc_1100 polypeptide, IL-10 polypeptide and IL-22 polypeptide respectively.

In some embodiments, the inflammatory or autoimmune disease is asthma. In some embodiments, the genetically modified microorganism comprises an exogenous gene encoding Amuc_1100 polypeptide and an exogenous gene encoding IL-10 polypeptide, respectively. In some embodiments, the genetically modified microorganism comprises an exogenous gene encoding IL-10 polypeptide and an exogenous gene encoding IL-22 polypeptide, respectively. In some embodiments, the genetically modified microorganism comprises an exogenous gene encoding Amuc_1100 polypeptide and an exogenous gene encoding IL-22 polypeptide, respectively. In some embodiments, the genetically modified microorganism comprises an exogenous gene encoding IL-10 polypeptide. In some embodiments, the genetically modified microorganism comprises an exogenous gene encoding Amuc_1100 polypeptide. In some embodiments, the genetically modified microorganism comprises an exogenous gene encoding IL-22 polypeptide. In some embodiments, the genetically modified microorganism comprises exogenous genes encoding Amuc_1100 polypeptide, IL-10 polypeptide and IL-22 polypeptide respectively.

In some embodiments, the inflammatory or autoimmune disease is graft versus host disease (GvHD). In some embodiments, the genetically modified microorganism comprises an exogenous gene encoding Amuc_1100 polypeptide and an exogenous gene encoding IL-10 polypeptide, respectively. In some embodiments, the genetically modified microorganism comprises an exogenous gene encoding IL-10 polypeptide and an exogenous gene encoding IL-22 polypeptide, respectively. In some embodiments, the genetically modified microorganism comprises an exogenous gene encoding Amuc_1100 polypeptide and an exogenous gene encoding IL-22 polypeptide, respectively. In some embodiments, the genetically modified microorganism comprises an exogenous gene encoding IL-10 polypeptide. In some embodiments, the genetically modified microorganism comprises an exogenous gene encoding Amuc_1100 polypeptide. In some embodiments, the genetically modified microorganism comprises an exogenous gene encoding IL-22 polypeptide. In some embodiments, the genetically modified microorganism comprises exogenous genes encoding Amuc_1100 polypeptide, IL-10 polypeptide and IL-22 polypeptide respectively.

In some embodiments, the subject has a condition/disease or disorder that is an inflammatory disease or an autoimmune disease (e.g., those described herein), or has a predisposition to the disease/disorder. A purpose of treatment is to treat, cure, alleviate, relieve, alter, correct, compromise, ameliorate or affect the disease/disorder, or the predisposition of the disease/disorder.

As used herein, "treatment" of a disease, disorder or condition includes preventing or ameliorating the disease, disorder or condition, slowing the onset or rate of progression of the disease, disorder or condition, reducing the risk of developing the disease, disorder or condition, preventing or delaying the development of symptoms associated with the disease, disorder or condition, reducing or ending symptoms associated with the disease, disorder or condition, completely or partially eliminating the disease, disorder or condition, curing the disease, disorder or condition, or a combination thereof.

As used herein, the term "effective amount" refers to an amount and/or a dosage and/or a dosage regimen of one or more agents necessary to produce the desired result, e.g., an amount sufficient to alleviate in a subject one or more symptoms associated with a disease for which the subject is receiving therapy, an amount sufficient to reduce the severity of, or delay the progression of, a disease in a subject (e.g., a therapeutically effective amount), an amount sufficient to reduce the risk of, or delay the onset of, a disease in a subject, and/or an amount that reduces the ultimate severity (e.g., a prophylactically effective amount). Effective amounts vary depending on the severity of the condition being treated, individual patient parameters including age, medical condition, height, gender and weight, duration of treatment, and the nature, if any, of the concurrent treatment, and other factors within the knowledge and experience of the general practitioners or other medical practitioners. These factors are well known in the art and can be determined by no more than routine experimentation. It is generally preferred to use the maximum dose of the individual component or combination that is safe based on reasonable medical judgment. However, as one of ordinary skill in the art will appreciate, a subject may insist on a low or tolerable dose for medical reasons, psychological reasons, or virtually any other reason.

As used herein, the term "subject" includes humans and non-human animals. Non-human animals include all vertebrates, such as mammals, and non-mammals, such as non-human primates, mice, rats, cats, rabbits, sheep, dogs, cattle, chickens, amphibians, and reptiles. Unless otherwise indicated, the terms "patient" or "subject" are used interchangeably herein.

As used herein, the term "inflammatory disease or autoimmune disease" includes, but is not limited to, inflammatory diseases and autoimmune diseases. The inflammatory disease may include autoimmune diseases such as inflammatory bowel disease. "Inflammatory bowel disease" and "IBD" are used interchangeably herein to refer to a disease associated with inflammation of the digestive tract, characterized by significant local inflammation and impaired epithelial barrier function in the gastrointestinal tract, which is typically driven by T cells and activated macrophages (Ghishan et al., 2014), including, but not limited to, Crohn's disease, ulcerative colitis, Behcet's disease, lymphocytic colitis, collagenous colitis, metastatic colitis and indeterminate colitis.

As used herein, "autoimmune disease" also includes graft-versus-host disease (GvHD), systemic lupus erythematosus, arthritis (such as rheumatoid arthritis, osteoarthritis, psoriatic arthritis, or juvenile idiopathic arthritis), asthma (such as allergic asthma or neutrophilic asthma).

As used herein, the term "graft versus host" or "GVH" refers to an immune response of transplanted (donor) cells to the microorganisms and tissues of the host. As used herein, the term "graft versus host disease" or "GvHD" refers to an abnormal condition (both acute and chronic) on microorganisms and tissues caused by the effects of transplanted (graft) cells by GVH. For example, a patient who receives a blood or bone marrow transplant from another person is at risk for acute GvHD. Acute graft-versus-host disease (GvHD) is a condition caused by donor immune cells in a patient transplanted with allogeneic bone marrow or blood cells. The intestinal epithelium and liver are frequently affected tissues, and in severe cases, GvHD can cause blistering of the skin or excessive diarrhea and weight loss. Inflammation in the liver caused by donor immune cells can lead to blockages that cause jaundice. Other tissues such as the lungs and thymus may also be affected. The diagnosis of GvHD is usually confirmed by looking at a small area of skin, liver, stomach, or intestine using a microscope to observe specific inflammatory features.

Systemic lupus erythematosus (SLE) is a highly heterogeneous autoimmune disease (AID), with attacks and remissions throughout the disease and complex and diverse clinical manifestations, and has main clinical features of the presence of multiple autoantibodies in the serum, represented by antinuclear antibodies, and multisystem involvement. The symptoms of SLE vary from person to person and can range from mild to severe. Common symptoms include joint pain and swelling, fever, chest pain, hair loss, mouth sores, swollen lymph nodes, feeling tired, and red rashes most commonly on the face. There is usually a period of flare-ups of the disease and a period of remission with fewer symptoms. The cause of SLE is unknown. It is thought to be related to genetic and environmental factors. Among identical twins, if one is affected, there is a 24% chance that the other will also be affected. Female sex hormones, sunlight, smoking, vitamin D deficiency and certain infections are also thought to increase risk. The diagnosis of SLE is difficult and is currently based on a combination of symptoms and laboratory tests.

Rheumatoid arthritis (RA) is a long-term autoimmune disease that primarily affects the joints. RA usually causes heat, swelling and pain in the joints, and the pain and stiffness tend to worsen after rest. The wrists and hands are most commonly affected, and the joints on both sides of the body are usually the same. RA may also affect other parts of the body, including the skin, eyes, lungs, heart, nerves, and blood. This can lead to a low red blood cell count, inflammation around the lungs and around the heart, and may also cause fever and low energy. Typically, the symptoms appear gradually over weeks to months. Although the cause of rheumatoid arthritis is unknown, it is believed to involve a combination of genetic and environmental factors. The underlying pathogenesis involves the body's immune system attacking the joints, which causes inflammation and thickening of the joint capsule and also affects the underlying bone and cartilage. The diagnosis of RA is primarily based on the patient's signs and symptoms. In a specific diagnosis, X-rays and laboratory tests may also be combined to support the diagnosis or rule out other diseases with similar symptoms.

In some embodiments, the autoimmune disease is selected from the group consisting of inflammatory bowel disease (e.g., Crohn's disease or ulcerative colitis), graft versus host disease (GvHD), systemic lupus erythematosus, arthritis (such as rheumatoid arthritis, osteoarthritis, psoriatic arthritis, or juvenile idiopathic arthritis), asthma (such as allergic asthma or neutrophilic asthma), and a combination thereof. In some embodiments, the autoimmune disease includes graft versus host disease (GvHD). In some embodiments, the autoimmune disease includes inflammatory bowel disease (such as Crohn's disease or ulcerative colitis), systemic lupus erythematosus, asthma, multiple sclerosis, and/or rheumatoid arthritis.

About 70% of the human immune system is in the gastrointestinal tract. There are many correlations and common disease signals between intestinal autoimmune diseases (such as inflammatory bowel disease) and non-intestinal autoimmune diseases (such as multiple sclerosis), including 1) altered cytokine signaling, 2) altered immune cell activity, 3) dysbiosis of intestinal flora. Intestinal dysbiosis is closely related to the pathogenesis of autoimmune diseases, both in animal models and humans. For example, changes in the microbial composition are found in the early stages of rheumatoid arthritis and inflammatory bowel disease. Some independent studies have pointed out that dysbiosis of intestinal flora exists in SLE patients in Spain and China. The balance of intestinal bacteria is critical to the regulation and development of the immune system. Moreover, intestinal barrier function is impaired in inflammatory states, which will prompt the leakage of bacteria from the mucosal layer into blood vessels, thereby stimulating local and systemic autoimmune pathways.

Multiple targets in intestinal inflammatory signaling pathways, the innate immune system and the adaptive immune system have become research hotspots for the development of new drugs using small molecules, antibodies, gene therapy, or intestinal microbial therapies. Among them, the potential of bacteria as vector for gene therapy using genetically modified bacteria as raw materials has recently been recognized by the industry. However, although these genetically modified microorganisms have shown efficacy in some preclinical models, efficacy in patients has not yet been observed.

Without wishing to be bound by any existing theory, in order to solve the above-mentioned unmet clinical needs, the inventors of the present invention constructed engineered microorganisms (such as engineered *Escherichia coli* EcN) as living drugs and administered them to the intestine to introduce exogenous peptide factors or a combination thereof. The engineered bacteria can continuously express and secrete exogenous peptide factors in the intestine, which can continuously act on intestinal cells over a period of time, thereby enhancing the intestinal barrier and/or improving intestinal immunity, thereby ameliorating the symptoms of immune diseases in the body. If the lesion is located in the intestine, the genetically modified microorganisms of the present invention are expected to be distributed near the lesion, and the polypeptide factors secreted by the microorganisms can even directly act on cells at the lesion site, thereby exerting a therapeutic effect. Such a mechanism of action cannot be achieved by peptide drugs, because if peptide drugs are taken orally, the peptides are easily degraded in the intestines, resulting in poor efficacy. Even if the effect of oral peptides can be improved to a certain extent by using better delivery vehicles, it is still difficult to meet the treatment needs and leads to problem of high cost. For serum injection, patient compliance problems are serious, and it may cause more side effects due to cytokine drugs directly entering the blood circulation.

The inventors first screened for exogenous gene combinations that are suitable for microbial expression and secretion and can exert therapeutic effects in local delivery to the intestine. It should be noted that living drugs need to maintain the viability of microorganisms so that they can grow normally and exert therapeutic effects in the body. However, the continued expression of exogenous peptides by microorganisms, especially two or more types of exogenous peptides, will inevitably bring certain pressure on cell stability. Therefore, it is first required that the exogenous peptides expressed by the microorganisms would not have an excessive adverse impact on the viability of the microorganisms themselves. Otherwise, the poor state of the microorganisms will affect their ability to express and secrete therapeutic exogenous peptides, and the microorganisms may reduce or even shut down the expression of exogenous genes through internal stress mechanisms, leading to failure in the construction of engineered microorganisms. However, which exogenous genes are suitable for expression in engineered microorganisms for therapy is still unknown and difficult to predict. In the field of non-living drugs, many exogenous genes that appear to be suitable for expression in microorganisms turn out unable to succeed when are constructed in engineered bacteria for therapeutic purposes. During the screening process, the inventors did find that some exogenous factors were not suitable to be expressed and secreted by the engineered microorganisms of the present invention, which were thus finally given up (such as IL-19, IL-23, IL-37, etc.). After the inventors determined the Amuc_1100, IL-10 and/or IL-22 polypeptides as the basis of the combination, they further comprehensively optimized the signal peptides of these polypeptides (replacing their own signal peptides), the expression control elements in the expression cassette (screening appropriate promoters, cistrons, ribosome binding sites, etc.), and the structure of the chassis bacteria (selectively knocking out specific membrane proteins to improve membrane permeability), so that the engineered bacteria can efficiently and stably express the above-mentioned new exogenous gene combinations. It has also been confirmed in multiple animal disease models that all or part of the engineered bacteria expressing the exogenous factor combinations have unexpected effects on a variety of inflammatory diseases or autoimmune diseases, and even exhibit synergistic effect, showing a broad clinical application prospect.

The following examples are provided to better illustrate the present invention and should not be construed to limit the scope of the present invention. All specific compositions, materials and methods described in whole or in part below are within the scope of the present invention. These specific compositions, materials and methods are not intended to limit the present invention but are merely intended to illustrate specific examples that fall within the scope of the present invention. Equivalent compositions, materials and methods can be developed by those skilled in the art without making inventive effort and without departing from the scope of the present invention. It will be understood that many changes may be made in the processes described herein while remaining within the scope of the present invention. The inventors intend that such variations be included within the scope of the present invention.

### Example

### Example 1: Protocol of editing bacterial genome

### 1.1 Design of sgRNA

A 20 bp sequence (N20NGG) connecting the NGG PAM sequence was searched on both strands of the target integration site sequence and blasted against the EnN genome. A unique 20 bp sequence was selected as the sgRNA targeting the integration site. The 300-500 bp sequences upstream and downstream of the sgRNA were selected as the left homology arm (LHA) and right homology arm (RHA) respectively.

### 1.2 Construction of sgRNA plasmid

The sgRNA sequence was added to the 5' end of the gRNA backbone reverse primer, amplified by PCR and digested with restriction endonucleases PstI and SpeI. The digested PCR fragment was ligated with plasmid pCBT003 (SEQ ID NO: 137) upon similar digestion to form pCBT003_sgRNA plasmid.

### 1.3 Preparation of donor gene fragments

The exogenous genes to be integrated into the EcN genome were synthesized in cloning plasmids (such as pUC57) by GeneScript. The exogenous genes were amplified using the synthesized plasmid as a template, and the LHA and RHA of the selected integration site were amplified using the EcN genome as a template. The PCR primers used to amplify these fragments carried 15-20 bp of homologous sequences with each other, so they can be connected by overlapping PCR, and the resulting PCR product comprising LHA-exogenous gene-RHA was used as the donor gene fragment.

### Example 2: Protocol of plasmid transformation

### 2.1 Preparation of EcN/pCBT001 competent cells for electroporation

100-200 ng of pCBT001 plasmid (a plasmid expressing Cas9 protein, SEQ ID NO: 136) was added to 100 µL of EcN electroporation competent cells. The mixture was transferred to a 2 mm electroporation cuvette, and the electroporation was performed under conditions of 2.5 kV, 25 µF, and 200Ω. After the electroporation was completed, the cells were suspended in 1 mL of SOC and cultured with shaking (220 rpm) at 30°C for two hours. All cells were then spread on a LB agar plate supplemented with 50 µg/mL spectinomycin and streptomycin, and cultured at 30°C overnight. The colonies growing on the plate were EcN with pCBT001 plasmid (EcN/pCBT001).

A single EcN/pCBT001 colony on the LB agar plate with resistance was inoculated into 3 mL of LB liquid medium supplemented with 50 µg/mL spectinomycin and streptomycin, and cultured overnight at 30°C with shaking (220 rpm). Then 300 µL of this overnight culture was inoculated into 30 mL of LB liquid medium supplemented with 50 µg/mL spectinomycin and streptomycin, and cultured at 30°C with shaking (220 rpm). IPTG was added to the culture after 1 h at a final concentration of 1 mM. When the OD₆₀₀ reached approximately 0.6, cells were washed three times with 20 mL, 10 mL and 5 mL of 10% glycerol (4°C) respectively, and finally resuspended in 300 µL of 10% glycerol (4°C). The cell suspension was aliquoted into a 1.5 mL centrifuge tube at 100 µL per tube.

### 2.2 Transformation of pCBT003-sgRNA plasmid and donor gene fragment

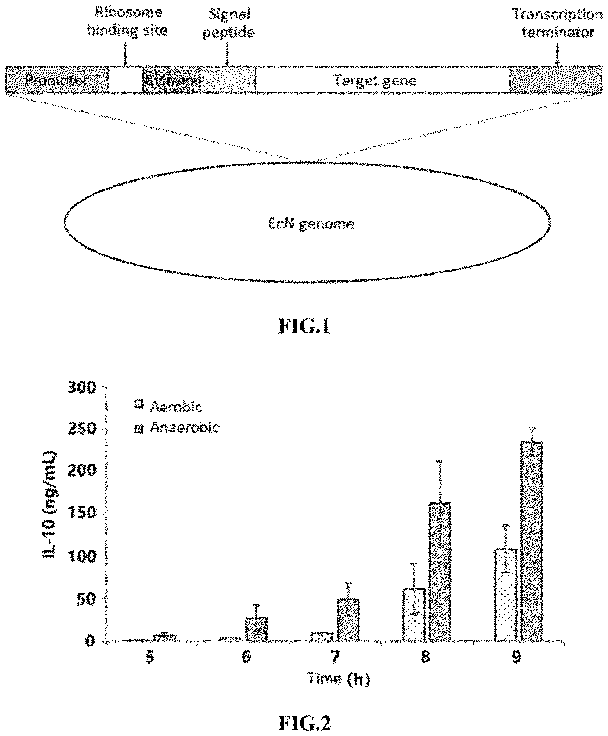

Approximately 2 µg of PCR product containing the donor gene fragment (LHA-exogenous gene-RHA) and 100-200 ng of pCBT003_sgRNA expressing the sgRNA targeting the integration site were transformed into EcN/pCBT001 competent cells for electroporation. After the electroporation was completed, the cells were suspended in 1 mL SOC and cultured with shaking (220 rpm) at 30°C for two hours. All cells were then spread on a LB agar plate supplemented with 50 µg/mL spectinomycin, streptomycin and 100 µg/mL ampicillin. The schematic diagram of the insertion of the exogenous gene expression cassette in the EcN genome is shown in FIG. 1.

### Example 3: Protocol of removing extra plasmids

### 3.1 Verification of integration of exogenous gene

The primers used for verification were designed upstream of LHA and downstream of RHA. A single colony growing on the transformation plate was selected and subjected colony PCR using the primers for verification. Clones with exogenous genes correctly integrated into the genome were screened based on the size of the PCR products.

### 3.2 Removal of pCBT003_sgRNA and pCBT001 plasmids

The selected correct clones were inoculated into 3 mL of LB liquid medium supplemented with 50 µg/mL spectinomycin, streptomycin and 10 mM arabinose, and cultured overnight at 30°C with shaking (220 rpm). Then the culture was diluted 10⁶ times. 100 µL of the diluted culture was spread on an LB agar plate supplemented with 50 µg/mL spectinomycin and streptomycin, and incubated overnight at 30°C. Single colonies were then picked and added on two LB agar plates one by one, wherein one LB agar plate was supplemented with 50 µg/mL spectinomycin, streptomycin and 100 µg/mL ampicillin, and the other one LB agar plate was supplemented with 50 µg/mL spectinomycin and streptomycin. The colonies that only grew on the LB agar plate supplemented with 50 µg/mL spectinomycin and streptomycin were the colonies without pCBT003_sgRNA plasmid.

The clone without pCBT003_sgRNA was inoculated into LB liquid medium and cultured overnight at 42°C with shaking (220 rpm). The culture was then diluted 10⁶ times. 100 µL of the diluted culture was spread on an LB agar plate. A single colony was picked and added on two LB agar plates one by one, wherein one was regular LB agar plate and one LB agar plate was supplemented with 50 µg/mL spectinomycin and streptomycin. The colonies that only grew on the LB agar plate were the colonies without pCBT001.

### 3.3 Removal of endogenous plasmids

### 3.3.1 Removal of pMUT1

The plasmid pCBT003_pMUT1_sgRNA expressing the sgRNA of pMUT1 was transformed into EcN/pCBT001. The primers for the verification of the removal of pMUT1 were specifically homologous to pMUT1 and not homologous to the genome sequence of EcN. PCR was performed with the primers for verification of pMUT1. The colonies in which desired PCR product cannot be obtained were the colonies without pMUT1.

### 3.3.2 Removal of pMUT2

The kanamycin resistance gene was connected to pMUT2 to obtain a plasmid pMUT2-kana, which comprises a nucleotide sequence set forth in SEQ ID NO: 138. The plasmid pMUT2-kana was transformed into EcNΔpMUT1. The transformed cells were spread on an LB agar plate supplemented with 10 µg/mL kanamycin. The colonies grown were EcNΔpMUT1 cells containing the plasmid pMUT2-kana. After EcNΔpMUT1/pMUT2-kana was passaged several generations in LB liquid medium supplemented with 10 µg/mL kanamycin, the plasmid was extracted. It was determined whether pMUT2 was completely replaced by pMUT2-kana detecting the electrophoresis bands. Then, pCBT001 was transferred into EcNΔpMUT1/pMUT2-kana in which pMUT2 was completely replaced, and then the plasmid pCBT003_Kana-sgRNA expressing the kanamycin resistance gene sgRNA was transferred into EcNΔpMUT1/pMUT2-kana/pCBT001. The obtained colonies were subjected to PCR using primers specific to the kanamycin resistance gene. The colonies in which desired PCR product could not be obtained were the colonies without pMUT2-kana.

In this example, the sequence of the sgRNA targeting pMUT1 is set forth in SEQ ID NO: 164; the sequence of the sgRNA targeting the kanamycin resistance gene is set forth in SEQ ID NO: 165.
Sequence of sgRNA targeting pMUT1 (SEQ ID NO: 164):
   gctgccgctattctgccgct.
Sequence of sgRNA targeting kanamycin resistance gene (SEQ ID NO: 165):
   atgaaggagaaaactcaccg.

### Example 4: Protocol of knocking out membrane proteins

The design method of sgRNA of the target membrane protein to be knocked out and the construction method of the sgRNA plasmid were the same as those described in 1.1 and 1.2 in Example 1. The 300-500 bp sequences upstream and downstream of the target membrane protein coding gene were selected as the left homology arm (LHA) and right homology arm (RHA). LHA and RHA of the selected knockout sites were amplified using the genome of EcN as a template. The PCR primers used to amplify these fragments had 15-20 bp homologous sequences with each other, such that they can be connected by overlapping PCR, and the resulting PCR product comprising LHA-RHA was used as the donor fragment. The obtained PCR product of the donor fragment and the pCBT003_sgRNA plasmid expressing the knockout site sgRNA were simultaneously transformed into competent cells comprising pCBT001. The obtained single colony was amplified with the primers for verification of the knockout site. Clones with the target protein successfully knocked out from the genome were screened based on the size of the amplification bands.

The membrane proteins knocked out in this example include tolQ, tolR, tolA, pal, lpp, mrcA and ompT. The sequence of the sgRNA targeting the tolQ knockout site is set forth in SEQ ID NO: 166, and the sequences of the homology arms on both sides of the tolQ knockout site are set forth in SEQ ID NO: 167 and 168 respectively. The sequence of the sgRNA targeting the tolR knockout site is set forth in SEQ ID NO: 169, and the sequences of the homology arms on both sides of the tolR knockout site are set forth in SEQ ID NO: 170 and 171 respectively. The sequence of the sgRNA targeting the tolA knockout site is set forth in SEQ ID NO: 172, and the sequences of the homology arms on both sides of the tolA knockout site are set forth in SEQ ID NO: 173 and 174 respectively. The sequence of the sgRNA targeting the pal knockout site is set forth in SEQ ID NO: 175, and the sequences of the homology arms on both sides of the pal knockout site are set forth in SEQ ID NO: 176 and 177 respectively. The sequence of the sgRNA targeting the lpp knockout site is set forth in SEQ ID NO: 178, and the sequences of the homology arms on both sides of the lpp knockout site are set forth in SEQ ID NO: 179 and 180 respectively. The sequence of the sgRNA targeting the mrcA knockout site is set forth in SEQ ID NO: 181, and the sequences of the homology arms on both sides of the mrcA knockout site are set forth in SEQ ID NO: 182 and 183. The sequence of the sgRNA targeting the ompT knockout site is set forth in SEQ ID NO: 184, and the sequences of the homology arms on both sides of the ompT knockout site are set forth in SEQ ID NO: 185 and 186 respectively.
Sequence of sgRNA targeting tolQ site (SEQ ID NO: 166):
   gggaaacgggataatctgag.
Sequence of the left homology arm (LHA) of tolQ site (SEQ ID NO: 167): 
gtgaatacaacgctgtttcgatggccggttcgtgtctactatgaagataccgatgccggtggtgtggtgtaccacgccagttacgtcgctttttatga
aagagcacgcacagagatgctgcgtcatcatcatttcagtcaacaggcgctgatggctgaacgcgttgcctttgtggtacgtaaaatgacggtggaatattacgca
cctgcgcggctcgacgatatgctcgaaatacagactgaaataacatcaatgcgtggcacctctttggttttcacgcaacgtattgtcaacgccgagaatactttgttg
aatgaagcagaggttctggttgtttgcgttgacccactcaaaatgaagcctcgtgcgcttcccaagtctattgtcgcggagtttaagcagtga.
Sequence of the right homology arm (RHA) of tolQ site (SEQ ID NO: 168): 
gccatggccagagcgcgtggacgaggtcgtcgcgatctcaagtccgaaatcaacattgtaccgttgctggacgtactgctggtgctgttgctgat
ctttatggcgacagcgcccatcatcacccagagcgtggaggtcgatctgccagacgctactgaatcacaggcggtgagcagtaacgataatccgccagtgattgt
tgaagtgtctggtattggtcagtacaccgtggtggttgagaaagatcgtctggagcgtttaccaccagagcaggtggtggcggaagtgtccagccgtttcaaggc
caacccgaaaacggtctttctgatcggtggcgcaaaagatgtgccttacgatgaaataattaaagcactgaacttgttacatagtgcgggtgtgaaatcggttggttt
aatgacgcagcctatctaaacatctgcgtttcccttgcttgaaagagagcgggtaacaggcgaacagtttttggaaaccgaga.
Sequence of the sgRNA targeting tolR site (SEQ ID NO: 169):
   tggtattggtcagtacaccg.
Sequence of the left homology arm (LHA) of tolR site (SEQ ID NO: 170): 
gcatcgtgccaatagccatgcgccggaagccgtagtggaaggggcgtcgcgtgcgatgcgtatttccatgaatcgtgaacttgaaaatctggaa
acgcacattcctttcctcggtacggttggctccatcagcccgtatattggtctgtttggtacggtctgggggatcatgcacgcctttatcgccctcggggcggtaaaa
caagcaacactgcaaatggttgcgcccggtatcgcagaagcgttgattgcgacggcaattggtttgttcgccgcaattccggcggtaatggcttataaccgcctca
accagcgcgtaaacaaactggaactgaattacgacaactttatggaagagtttaccgcgattctgcaccgccaggcgtttaccgttagcgagagcaacaaggggt
aa.
Sequence of the right homology arm (RHA) of tolR site (SEQ ID NO: 171): 
acatctgcgtttcccttgcttgaaagagagcgggtaacaggcgaacagtttttggaaaccgagagtgtcaaaggcaaccgaacaaaacgacaag
ctcaagcgggcgataattatttcagcagtgctgcatgtcatcttatttgcggcgctgatctggagttcgttcgatgagaatatagaagcttcagccggaggcggcgg
tggttcgtccatcgacgctgtcatggttgattcaggtgcggtagttgaacagtacaaacgtatgcaaagccaggaatcaagcgcgaagcgttctgatgagcagcg
caagatgaaggagcagcaggctgctgaagaactgcgtgagaaacaagcggctgaacaggaacgcctgaagcaacttgagaaagagcggttagcggctcag
gagcagaaaaagcaggctgaagaagccgcaaaacaggccgagttaaagcagaagcaagcggaagaggc.
Sequence of the sgRNA targeting tolA site (SEQ ID NO: 172):
   Agaactgcgtgagaaacaag.
Sequence of the left homology arm (LHA) of tolA point (SEQ ID NO: 173): 
gccatggccagagcgcgtggacgaggtcgtcgcgatctcaagtccgaaatcaacattgtaccgttgctggacgtactgctggtgctgttgctgat
ctttatggcgacagcgcccatcatcacccagagcgtggaggtcgatctgccagacgctactgaatcacaggcggtgagcagtaacgataatccgccagtgattgt
tgaagtgtctggtattggtcagtacaccgtggtggttgagaaagatcgtctggagcgtttaccaccagagcaggtggtggcggaagtgtccagccgtttcaaggc
caacccgaaaacggtctttctgatcggtggcgcaaaagatgtgccttacgatgaaataattaaagcactgaacttgttacatagtgcgggtgtgaaatcggttggttt
aatgacgcagcctatctaaacatctgcgtttcccttgcttgaaagagagcgggtaacaggcgaacagtttttggaaaccgaga.
Sequence of the right homology arm (RHA) of tolA site (SEQ ID NO: 174): 
tcgcgatgttgactgttccgacggtcaacatcaggcaccggttgccacggggttctggtagttttgtgtattttagtttgttaacattctgctaaattatc
gtgggccatcggtccagataagggagatatgatgaagcaggcattacgagtagcatttggttttctcatactgtgggcatcagttctgcatgctgaagtccgcattgt
gatcgacagcggtgtagattccggtcgtcctattggtgttgttcctttccagtgggcggggcctggtgcggcacctgaagatattggcggcatcgttgctgctgactt
gcgtaacagcggtaaatttaatccgttagatcgtgctcgtttgccacagcagccgggtagtgcgcaggaagtacaaccagctgcatggtccgcgctgggcattga
cgctgtggttgtcggtcaggtcactccgaatccggatggttcttacaatgttgcttatcaacttgttgacactggcgg.
Sequence of the sgRNA targeting pal site (SEQ ID NO: 175):
   agtcaccgtagaaggtcacg.
Sequence of the left homology arm (LHA) of pal site (SEQ ID NO: 176): 
tggtcgcagtaacaataccgaaccgacctggttcccggacagccagaacctggcatttacttctgaccaggccggtcgtccgcaggtttataaagt
gaatatcaacggcggtgcgccacaacgtattacctgggaaggttcgcagaaccaggatgcggatgtcagcagcgacggtaaatttatggtaatggtcagctcca
acggtgggcagcagcacattgccaaacaagatctggcaacgggaggcgtacaagttctgtcgtccacgttcctggatgaaacgccaagtctggcacctaacgg
cactatggtaatctacagctcttctcaggggatgggatccgtgctgaatttggtttctacagatgggcgtttcaaagcgcgtcttccggcaactgatggacaggtcaa
attccctgcctggtcgccgtatctgtgataataattaattgaatagtaaaggaatcattgaa.
Sequence of the right homology arm (RHA) of pal site (SEQ ID NO: 177): 
gagaattgcatgagcagtaacttcagacatcaactattgagtctgtcgttactggttggtatagcggccccctgggccgcttttgctcaggcaccaat
cagtagtgtcggctcaggctcggtcgaagaccgcgtcactcaacttgagcgtatttctaatgctcacagccagcttttaacccaactccagcaacaactctctgata
atcaatccgatattgattccctgcgtggtcagattcaggaaaatcagtatcaactgaatcaggtcgtggagcggcaaaagcagatcctgttgcagatcgacagcct
cagcagcggtggtgcagcggcgcaatcaaccagcggcgatcaaagcggtgtggcggcatcaacgacgccgacagctgatgctggtactgcgaatgctggcg
cgccggtgaaaagcggtgatgcaaacacggattacaatgcagctattgcgctggtgcag.
Sequence of the sgRNA targeting lpp site (SEQ ID NO: 178):
   acgttcaggctgctaaagat.
Sequence of the left homology arm (LHA) of lpp site (SEQ ID NO: 179): 
aatacttcccggatgccaccatcctggcactgaccaccaacgaaaaaacggctcaccagttagtactgagcaaaggtgttgtgccgcagctggtt
aaagagatcacatctactgatgacttctaccgtctgggtaaagaactggctctgcagagcggtctggcacacaaaggtgatgtcgtagttatggtttctggtgcact
ggtaccgagcggcactactaacaccgcatctgttcacgtcctgtaatattgcttttgtgaattaatttgtatatcgaagcgccctgatgggcgctttttttatttaatcgat
aaccagaagcaataaaaaatcaaatcggatttcactatataatctcactttatctaagatgaatccgatggaagcatcctgttttctctcaatttttttatctaaaacccag
cgttcgatgcttctttgagcgaacgatcaaaaataagtgccttcccatcaaaaaaa.
Sequence of the right homology arm (RHA) of lpp site (SEQ ID NO: 180): 
cctgtgaagtgaaaaatggcgcacattgtgcgccatttttttgcctgctatttaccgctactgcgtcgcgcgtaacatattcccttgctctggttcccca
ttctgcgctgactctactgaaggcgcattgctggctgcgggagttgctccactgctcaccgcaaccggataccctgcccgacgatacaacgctttatcgactaactt
ctgatctacagccttattgtctttaaattgcgtaaagcctgctggcagcgtgtacggcattgtctgaacgttctgctgttcttctgccgatagtggtcgatgtacttcaac
ataacgcatcccgttaggttccacggaatatttcaccggttcgttgatcactttcaccggtgttcccgtccgcacgctggagaataaagctttaatatccggtgcattc
atgcgaataca.
Sequence of the sgRNA sequence targeting mrcA site (SEQ ID NO: 181):
   caaaccgttcctctacaccg.
Sequence of the left homology arm (LHA) of mrcA site (SEQ ID NO: 182): 
gacagccaggccatttgctcccgctcaccgagggacatcgacgggcgtggaaatgaccgctgtaatgtgcgactggcgggaaacgccaacat
aatgtgatgacgctgcggcagttcacgactccacggtaacaacgtgttagccagccgctgcacatcaacaatccgcccatctttgataatgtcgttctccagtggca
accgccaccagcggtgcaataagcattcttttgcgctccgcacgatagcaaccgctaccgcttcttgctgttgtaaatgcaaaccaatttgccagttcttaaatgccat
tgtgatgatctccttatcacccgtcactctgacgggtatatcaatgcgtctggcttgcctttatactaccgcgcgtttgtttataaactgcccaaatgaaactaaatgg.
Sequence of the right homology arm (RHA) of mrcA site (SEQ ID NO: 183): 
ttaaaaaggcgcttcggcgcctttttcagtttgctgacaaagtgcacttgtttatgccagatgcgggtgaacgcgttatccggccaacaaaaaattga
aaattcaataaattgcaggaacttcgtaagcctgataagcgttgtgcatcaggcaaacttcacgcatttacactcgcccctgccctttcaaccattcgcgcacgagga
 
acagcgcgctgacattacgcgcttcgttgaagtcaggggcttccagcaaatccatcatatgcgccagcggccagcgcacctgtggtagcggctctggctcatcg
ccttccagtgattccgggtagagatcttgcgctaccacgatattcattttgctggaaaagtaagacggtgccatgctgagcttcttcaaaaaagtcagatcgttcgca
ccaaatccaacctcttcttttagctcgcggttagcggcttca.
Sequence of the sgRNA sequence targeting ompT site (SEQ ID NO: 184):
   cctgggactcatggccggat.
Sequence of the left homology arm (LHA) of ompT site (SEQ ID NO: 185): 
cccagcccttgtcgatgccgcgttcaggctagcttcgttcaaatccattaaagatatcagactactctcaggtacgtgagtaaggtaaaacccagttc
caacgccaatatccagatggttgttacctaaatgttccagaaagtgtggaagaaggtgttcctttgtaggacatccccatgcaagccgatttgatactcccaaaaccc
accagtcataaagctttagggtaagtggtgtgtaaattttagccccatcatctgtgttttttttcattagtttcaccgtgttatagttttatttgtgaattaaatcaattatagag
atgaattacaaggggttaaatgatgccacggcataacgaatttgaatatattgggtcttgtgttgcggtaagaggttacacgccaggacgcaggttaaatgcttacaa
tattaggggatattgtttgcttttaacggtaaacaaattccccggggctatacaataccaccggggagaaaatctatttaacgttg.
Sequence of the right homology arm (RHA) of ompT site (SEQ ID NO: 186):

aaaaggtctccattcaatcgttttaatgattgagtatgtattttttatatctaacttaatgagtcaattgtatattgccccactgtttatattttgtttaacattga
atttttgtcacaatgcgctgtcagttttttagtttaattgtctgtttgtgttttgtattgtggttttatgggctaattatttttttctttgtggagtttttatctattcaagtgccatgcct
ttagatgcatattagcgataatagcatgaggtttatcctcaattgctgtgttttttagtataaaaaagaggaacaaaactgagacacataaggcctcgcaatggcttgc
aaggttttacatattttgaggtggtggaacgtgtgaacgcagagatggcgcggtaatttgttgatttaaaatgtcgttcttggagtttctaagtcgtgggctacaggttc
gaatcctgcagggcgagccatttcctcgccatttatttgttcccttttacaaattacaccatcactttgttgtcgcggttttgctaaataattggaacacgtcagacgaata
aaatgaaaacagaaattctgctaaacaattcatcttgccaggattttgattaggaaagtgaaa.

### Example 5: Protocol for overexpressing molecular chaperones

### 5.1 Selection of molecular chaperones

Various molecular chaperone combinations were expressed in the engineered bacterium CBT4020 using plasmids. The production of IL-10 expressed in the culture supernatant and intracellular cells after the engineered bacterium was cultured in LB for 24 hours is shown in Table 11. The results show that the production of IL-10 can be increased by expressing molecular chaperones, and the best effect was achieved by expressing molecular chaperones dsbA, dsbC, dnaK, dnaJ, grpE, groES, groEL, tig, fkpA and surA simultaneously.

**Table 11 Various combinations of molecular chaperones and IL-10 production**

| Overexpressed molecular chaperones | IL-10 production (ng/mL/OD₆₀₀) | |
|---|---|---|
| | Supernatant | Cell lysate |
| dnaK, dnaJ, grpE | 24.92±0.75 | 8.39±1.86 |
| groES, groEL | 21.93±1.72 | 7.13±0.08 |
| Tig | 23.26±1.01 | 8.3±2.46 |
| dsbA, dsbC, fkpA, surA | 19±1.88 | 7.41±1.93 |
| dsbA, dsbC, dnaK, dnaJ, grpE, groES, groEL, tig, fkpA, surA | 28.97±4.75 | 17.04±1.02 |
| None | 14.88±0.21 | 6.52±0.06 |

### 5.2 Construction of plasmid of molecular chaperone

### 5.2.1 Construction of plasmid pCBT012

The promoter BBa_J23114 and the ribosome binding site (RBS) were amplified using the synthetic plasmid as a template. The groES, groEL and tig coding sequences were amplified using the plasmid pG-TF2 as a template. The fkpA and surA coding sequences and the LHA and RHA of the insertion site yieN were amplified using the EcN genome as a template. The transcription terminator was amplified using the plasmid pCBT003 as a template. The sgRNA expression sequence targeting the yieN site was amplified using the sgRNA expression plasmid pCBT003_yieN_sgRNA as a template. The PCR primers used to amplify these fragments had 15-20 bp homologous sequences with each other, such that they can be connected by overlapping PCR to obtain a donor gene fragment expression cassette connected with LHA and RHA-yieN_sgRNA sequences on both sides respectively. The elements on the molecular chaperone expression cassette were arranged in the following order from 5' to 3' ends as: 5'-promoter-ribosome binding site (RBS)-groES-groEL-tig-fkpA-surA coding sequence-terminator. The pCBT012 plasmid backbone was amplified using the plasmid pCBT010 as a template. The PCR primers used to amplify the pCBT012 plasmid backbone had a 15-20 bp homologous sequence with the molecular chaperone expression cassette, such that they could be connected with the molecular chaperone expression cassette using the kit ClonExpress Ultra One Step Cloning Kit (Vazyme ) to generate plasmid pCBT012 (SEQ ID NO: 215).

Sequence of the plasmid pCBT010 (SEQ ID NO: 187):

gaattccggatgagcattcatcaggcgggcaagaatgtgaataaaggccggataaaacttgtgcttatttttctttacggtctttaaaaaggccgtaa
tatccagctgaacggtctggttataggtacattgagcaactgactgaaatgcctcaaaatgttctttacgatgccattgggatatatcaacggtggtatatccagtgatt
tttttctccattttagcttccttagctcctgaaaatctcgataactcaaaaaatacgcccggtagtgatcttatttcattatggtgaaagttggaacctcttacgtgccgatc
aacgtctcattttcgccaaaagttggcccagggcttcccggtatcaacagggacaccaggatttatttattctgcgaagtgatcttccgtcacaggtatttattcgggat
cctcgctcactgactcgctacgctcggtcgttcgactgcggcgagcggaaatggcttacgaacggggcggagatttcctggaagatgccaggaagatacttaac
agggaagtgagagggccgcggcaaagccgtttttccataggctccgcccccctgacaagcatcacgaaatctgacgctcaaatcagtggtggcgaaacccgac
aggactataaagataccaggcgtttccccctggcggctccctcgtgcgctctcctgttcctgcctttcggtttaccggtgtcattccgctgttatggccgcgtttgtctc
attccacgcctgacactcagttccgggtaggcagttcgctccaagctggactgtatgcacgaaccccccgttcagtccgaccgctgcgccttatccggtaactatc
gtcttgagtccaacccggaaagacatgcaaaagcaccactggcagcagccactggtaattgatttagaggagttagtcttgaagtcatgcgccggttaaggctaa
actgaaaggacaagttttggtgactgcgctcctccaagccagttacctcggttcaaagagttggtagctcagagaaccttcgaaaaaccgccctgcaaggcggttt
tttcgttttcagagcaagagattacgcgcagaccaaaacgatctcaagaagatcatctgtaggtcgttcgctccaagctggaaggatcttcacctagatccttttaaat
taaaaatgaagttttaaatcaatctaaagtatatatgagtaaacttggtctgacagaatttctgccattcatccgcttattatcacttattcaggcgtagcaaccaggcgtt
taagggcaccaataactgccttaaaaaaattacgccccgccctgccactcatcgcagtactgttgtaattcattaagcattctgccgacatggaagccatcacaaac
ggcatgatgaacctgaatcgccagcggcatcagcaccttgtcgccttgcgtataatatttgcccatggtgaaaacgggggcgaagaagttgtccatattggccacg
tttaaatcaaaactggtgaaactcacccagggattggctgagacgaaaaacatattctcaataaaccctttagggaaataggccaggttttcaccgtaacacgccac
atcttgcgaatatatgtgtagaaactgccggaaatcgtcgtggtattcactccagagcgatgaaaacgtttcagtttgctcatggaaaacggtgtaacaagggtgaa
cactatcccatatcaccagctcaccgtctttcattgccatacg.

### 5.2.2 Construction of plasmid pCBT013

The promoter BBa_J23114 and the ribosome binding site (RBS) were amplified using the synthetic plasmid as a template. The dnaK, dnaJ and grpE coding sequences were amplified using the plasmid pKJE7 as a template. The dsbA and dsbC coding sequences and the LHA and RHA of the insertion site yicS/nepI were amplified using the EcN genome as a template. The transcription terminator was amplified using the plasmid pCBT003 as a template. The sgRNA expression sequence targeting the yicS/nepI site was amplified using the sgRNA expression plasmid pCBT003_yicS/nepI_sgRNAas a template. The PCR primers used to amplify these fragments had 15-20 bp homologous sequences with each other, such that they can be connected by overlapping PCR to obtain a donor gene fragment expression cassette connected with LHA and RHA-yicS/nepI_sgRNA sequences on both sides respectively. The elements on the molecular chaperone expression cassette were arranged in the following order from 5' to 3' ends as: 5'-promoter-ribosome binding site (RBS)-dsbA-dsbC-dnaK-dnaJ-grpE coding sequence-terminator. The pCBT013 plasmid backbone was amplified using the plasmid pCBT010 as a template. The PCR primers used to amplify the pCBT013 plasmid backbone had a 15-20 bp homologous sequence with the molecular chaperone expression cassette, such that they could be connected with the molecular chaperone expression cassette using the kit ClonExpress Ultra One Step Cloning Kit (Vazyme) to generate the plasmid pCBT013 (SEQ ID NO: 216).

### 5.3 Integration of molecular chaperones into genome

The molecular chaperone-expressing plasmids pCBT012 and pCBT013 were respectively transformed into competent cells comprising pCBT001. The obtained single colony was amplified with the primers for verification of the insertion site. Clones with the molecular chaperone expression cassette successfully inserted into the genome were screened based on the size of the amplification bands. The sequence accession numbers of each molecular chaperone are shown in Table 12.

**Table 12 Molecular chaperones**

| Molecular chaperone | Uniprot ID |
|---|---|
| dsbA | P0AEG4 |
| dsbC | P0AEG6 |
| dnaK | P0A6Y8 |
| dnaJ | P08622 |
| grpE | P09372 |
| groES | P0A6F9 |
| groEL | P0A6F5 |
| Tig | P0A850 |
| fkpA | P45523 |
| surA | P0ABZ6 |

The sequence of the sgRNA targeting the yieN insertion site is set forth in SEQ ID NO: 188, and the sequences of the homology arms on both sides of the yieN insertion site are set forth in SEQ ID NO: 189 and 190 respectively. The sequence of the sgRNA targeting the yicS/nepI insertion site is set forth in SEQ ID NO: 191, and the sequences of the homology arms on both sides of the yicS/nepI insertion site are set forth in SEQ ID NO: 192 and 193 respectively.
Sequence of the sgRNA targeting the yieN site (SEQ ID NO: 188):
   gagggtgagccataatgaag.
Sequence of the left homology arm (LHA) of the yieN site (SEQ ID NO: 189): 
tccgagacataaggcgcatccggtaatttatccagttgctggcgcagcataaaaatcagctcaaatacatgatcgggcagcgtaatttcaccaatct
ctttctgccagcgttcatattcttcatctgtgatctgcaaggaggcaggaaccggattgtcattttcatcctgttgactggtcagcatggagcggaaattcgctttatcct
gcactttatctaaccacagacgaatcagcatgcgatcatataacgcttccagactgctgtctgcttccggcagctcgttggaggccgccaccagcagacgcatcg
ggattttttctacgagtgcgccgttgcggaactggcgctcgttaatggcggtgagcagggtattaagaattgccggacccgctttccagatctcatccagaaagac
gatttcagcttccggcaggtaaccgctggttaaacgttcatagcgcccttcatcttttagcgcctgaatagaaaggggaccaaaaacttcttccggcgtggagaagc
gggtcatcagatattcaaacgcgcggg.
Sequence of the right homology arm (RHA) of the yieN site (SEQ ID NO: 190): 
aatgtggccgcatttaggagtacgattttgccgttaatcgtgcatactgtgcgcttttttgtgggccaagggactaagcacacatttcatatttcaacga
aagactagtctatgagcactgataataagcaatcattgcccgcgattaccctcgcggcgattggagttgtctacggcgatattggtaccagcccgttatatacacttc
gtgaatgtttgtccggccagtttggttttggcgttgaacgcgatgccgtgtttggctttttatcgctgatcttctggctgctaatctttgtggtttccattaaatatctcacctt
cgtgatgcgggcagata.
Sequence of the sgRNA targeting the yicS/nepI site (SEQ ID NO: 191):
   ctgaccaacgcttctttacc.
Sequence of the left homology arm (LHA) of the yicS/NepI site (SEQ ID NO: 192): 
aacgaaacgctatcgataagaataaaaaggaataaaagtggaatataaagtctggcacttcttactgaccacgcaaacacctttcgtacagcatga
tgagagcggcaaaagcaaactacacttatgcttcattcgatgcacatttgcagaaggttgccgctatgaagccaacgatgctactcatgattaccgtattcctgattttt
ccagccatttctcaggccgagtcgccattcagttctctacagtcggcaaaagagaaaaccacggtattgcaagatttacgtaaaatttgcacgccacaggcgtcatt
atcagatgaagcgtgggaaaagttaatgttgtcagatgaaaacaataaacaacatatacgcgaggccatcgtggcgatggaacgtaataatcagagcaattattgg
gaagcgttgggtaaggtagaatgcccggatatgtaagaacggcaacaaccactg.
Sequence of the right homology arm (RHA) of the yicS/NepI site (SEQ ID NO: 193):

accaacgcttctttacctgatatcaggatttcttcattttcacctttgcagtcaccaacaatgcagtcagcaacatcaatgtgccggacaacatcagcg

gcgaagtcagaccaatgttatccaacgcataaccgccgattgccgcaccgcaggtgttagcaagctgaataacggccacctgaatagatccagccttttctgcctg
atcggccaatgagcgagtgatccacgttgaccaaccaacgggaattaaagcaaaggtcagcccccagataatcgcaacaccagtagcaacaattttatagcttcc
ccacagcgtcagtaccagcgcgctcagggcgagcacaaacggtgcgcccgctaaggccagtttcaccgaacgtttgagaataaacgacgaaagcgatgtccc
gacaaagctggcgataccaaaactcagcagcaccagggttaagccatcaaca.

### Example 6: Construction of strains

### 6.1 Construction of strains expressing IL-10

### 6.1.1 Construction of donor expression cassette containing IL-10 gene fragment

The IL-10 coding sequence, signal peptide, promoter, ribosome binding site (RBS), cistron, etc. were synthesized by GeneScript in a cloning plasmid (such as pUC57). The IL-10 coding sequence, signal peptide, promoter, ribosome binding site (RBS) and cistron were amplified using the synthetic plasmid as a template, and the LHA and RHA of the insertion site were amplified using the EcN genome as a template. The transcription terminator was amplified using the plasmid pCBT003 as a template. The PCR primers used to amplify these fragments had 15-20 bp homologous sequences with each other, such that they can be connected by overlapping PCR to obtain a donor gene fragment expression cassette connected with LHA and RHA sequences on both sides respectively. The elements on the expression cassette were arranged in the following order from 5' to 3' ends as: 5'-promoter-ribosome binding site (RBS)-cistron-signal peptide-IL-10 coding sequence-terminator (the sequences of each donor gene fragment expression cassette constructed are set forth in SEQ ID NO: 144-156 and 235). The obtained PCR product of the donor gene fragment expression cassette with LHA and RHA sequences on both sides and the pCBT003_maeB_sgRNA plasmid expressing sgRNA were simultaneously transformed into competent cells containing pCBT001. Clones with the IL-10 successfully inserted into the genome were screened based on the size of the bands amplified using the verification primers of the integration sites. The production of IL-10 was measured using ELISA kit (Sino Biological, KIT10947A).

The IL-10 coding sequence in this example was the sequence encoding wild-type human IL-10 (SEQ ID NO: 11). The insertion site was maeB in the EcN genome. The sequence of the sgRNA targeting the maeB site is set forth in SEQ ID NO: 194, and the sequences of the homology arms on both sides of the maeB site are set forth in SEQ ID NO: 195 and 196 respectively.
Sequence of the sgRNA targeting maeB site (SEQ ID NO: 194):
   gaaggggaagaggcgcgcgt.
Sequence of the left homology arm (LHA) of the maeB site (SEQ ID NO: 195): 
gtggtggatgacggtaaacgtaccctcgatgatgtgattgaaggcgcggatattttcctcggctgttccggcccgaaagtgctgacccaggaaat
ggtgaagaaaatggcccgtgcgccaatgatcctggcgctggcgaacccggaaccggaaattctgccgccgctggcgaaagaagtgcgtccggatgccatcat
ctgtaccggccgttccgactacccgaaccaggtgaacaacgtcctttgcttcccgtttatcttccgtggcgcgctggacgttggcgcaaccgccatcaacgaagag
atgaaactggcggcagtacgcgcgattgcagaactggcccatgcggaacagagtgaagtggtggcttcagcgtatggcgatcaggatctgagctttggtccgga
atacatcattcc.
Sequence of the right homology arm (RHA) of the maeB site (SEQ ID NO: 196):

gcgaaaccgatccttattggtcgtccgaacgtgatcgaaatgcgcattcagaaactgggcttgcagatcaagtcgggcgttgattttgagatcgtca
ataacgaatccgatccgcgctttaaagagtactggaccgaatacttccagatcatgaagcgtcgcggtgtcactcaggagcaggcgcagcgtgcgctgatcagta
acccgacagtgatcggcgcgatcatggttcagcgtggcgaagccgatgcaatgatttgcggtacggtgggcgattatcatgaacactttagcgtggtgaaaaatg
tctttggttatcgcgatggcgttcacaccgcaggtgcaat.

The information of each strain expressing IL-10 obtained in this example and the insertion elements and knockout elements therein are shown in Table 13 below. The sequences of the relevant elements are shown in Tables 2 to 9.

**Table 13 Information of elements in strains expressing IL-10**

| Name of strain | Promoter | RBS | Cistr on | Signal peptide | Terminator | Knock out protein | Molecular chaperone overexpre ssion | SEQ ID NO of expression cassette |
|---|---|---|---|---|---|---|---|---|
| CBT4007 | BBa_J231 01 | Synthesi zed | None | DsbA | rrnB_T1_T 7Te_termina tor | None | No | 147 |
| CBT4009 | BBa_J231 01 | Synthesi zed | None | OmpA | rrnB_T1_T 7Te_termina tor | None | No | 148 |
| CBT4011 | BBa_J231 01 | Synthesi zed | None | PelB | rrnB_T1_T 7Te_termina tor | None | No | 149 |
| CBT4013 | BBa_J231 01 | Synthesi zed | None | YebF | rrnB_T1_T 7Te_termina tor | None | No | 150 |
| CBT4003 | BBa_J231 01 | Synthesi zed | None | USP45 | rrnB_T1_T 7Te_termina tor | None | No | 144 |
| CBT4004 | BBa_J231 08 | Synthesi zed | None | USP45 | rrnB_T1_T 7Te_termina tor | None | No | 146 |
| CBT4005 | BBa_J231 10 | Synthesi zed | None | USP45 | rrnB_T1_T 7Te_termina tor | None | No | 145 |
| CBT4026 | BBa_J231 10 | Synthesi zed | T7g1 0 | USP45 | rrnB_T1_T 7Te_termina tor | None | No | 151 |
| CBT4028 | BBa_J231 10 | Synthesi zed | BCD 2 | USP45 | rrnB_T1_T 7Te_termina tor | None | No | 152 |
| CBT4029 | BBa_J231 10 | Synthesi zed | GFP | USP45 | rrnB_T1_T 7Te_termina tor | None | No | 153 |
| CBT4030 | BBa_J231 10 | Synthesi zed | Lucif erase | USP45 | rrnB_T1_T 7Te_termina tor | None | No | 154 |
| CBT4071 | BBa_J231 10 | Synthesi zed | None | USP45 | rrnB_T1_T 7Te_termina tor | tolQ | No | 145 |
| CBT4072 | BBa_J231 10 | Synthesi zed | None | USP45 | rrnB_T1_T 7Te_termina tor | tolR | No | 145 |
| CBT4073 | BBa_J231 10 | Synthesi zed | None | USP45 | rrnB_T1_T 7Te_termina tor | tolA | No | 145 |
| CBT4074 | BBa_J231 10 | Synthesi zed | None | USP45 | rrnB_T1_T 7Te_termina tor | pal | No | 145 |
| CBT4112 | BBa J231 10 | Synthesized | None | USP45 | rrnB_T1_T 7Te_termina tor | pal, mrcA | No | 145 |
| CBT4020 | BBa_J231 10 | Synthesi zed | None | USP45 | rrnB_T1_T 7Te_termina tor | LPP | No | 145 |
| CBT4062 | BBa_J231 10 | Synthesi zed | BCD 2 | USP45 | rrnB_T1_T 7Te_termina tor | LPP | Yes | 152 |
| CBT4075 | BBa_J231 10 | Synthesi zed | BCD 2 | USP45 | rrnB_T1_T 7Te_termina tor | LPP, mrcA | Yes | 145 |
| CBT4076 | BBa_J231 10 | Synthesi zed | BCD 2 | USP45 | rrnB_T1_T 7Te_termina tor | LPP, ompT | Yes | 145 |
| CBT4077 | BBa_J231 10 | Synthesi zed | BCD 2 | USP45 | rrnB_T1_T 7Te_termina tor | LPP, ompT, mrcA | Yes | 145 |
| CBT4078 | PfnrS | Synthesi zed | BCD 2 | USP45 | rrnB_T1_T 7Te_termina tor | LPP | Yes | 155 |

### 6.1.2 Optimization for IL-10 expression

The production of IL-10 using different signal peptides expressed in the culture supernatant and in the cells after culturing the engineered bacteria in LB for 24 hours is shown in Table 14. Among them, the dsbA signal peptide was used in strain CBT4007; the ompA signal peptide was used in strain CBT4009; the pelB signal peptide was used in strain CBT4011; the yebF signal peptide was used in strain CBT4013, and the USP45 signal peptide was used in strain CBT4003. At the same dilution, the concentrations of IL-10 in the engineered bacterial samples using the OmpA, PelB and YebF signal peptides were lower than the detection limit. When the USP45 signal peptide was used, the IL-10 production was significantly higher than that of the DsbA signal peptide.

**Table 14 Genotypes of the engineered bacteria using different signal peptides and IL-10 production thereof**

| Strain | Signal peptide in expression cassette | IL-10 production (ng/mL) | |
|---|---|---|---|
| | | Supernatant | Cell lysate |
| CBT4007 | DsbA | 1.02±0.73 | 1.23±0.05 |
| CBT4009 | OmpA | * | - |
| CBT4011 | PelB | - | - |
| CBT4013 | YebF | - | - |
| CBT4003 | USP45 | 6.15±4.12 | 4.47±3.39 |

| | | | |
|---|---|---|---|
| *: "-" means lower than the detection limit | | | |

The production of IL-10 using different promoters expressed in the culture supernatant and in the cells after culturing the engineered bacteria in LB for 24 hours is shown in Table 15. Among them, the BBa_J23101 promoter was used in strain CBT4003; the BBa_J23108 promoter was used in strain CBT4004; and the BBa_J23110 promoter was used in strain CBT4005. The results show that IL-10 production can be regulated by selecting an appropriate promoter (such as BBa_J23101 promoter).

**Table 15 Genotypes of the engineered bacteria with different promoters and IL-10 production thereof**

| Strain | Promoter in expression cassette | IL-10 production (ng/mL) | |
|---|---|---|---|
| | | Supernatant | Cell lysate |
| CBT4003 | J23101 | 15.92±5.75 | 49.56±3.42 |
| CBT4004 | J23108 | 3.64±0.54 | 29.03±7.31 |
| CBT4005 | J23110 | 6.99±1.49 | 35.39±1.15 |

The production of IL-10 using different cistrons expressed in the culture supernatant after culturing the engineered bacteria in LB for 24 hours is shown in Table 16. Among them, the cistron T7g10 was used in the strain CBT4026; the cistron BCD2 was used in the strain CBT4028; the cistron GFP was used in the strain CBT4029; the cistron lucifierase was used in the strain CBT4030. The results show that the IL-10 production can be modulated by selecting different cistrons.

**Table 16 Genotypes of the engineered bacteria with different cistrons and IL-10 production thereof**

| Strain | Cistron in expression cassette | IL-10 production (ng/mL) |
|---|---|---|
| CBT4005 | None | 3.07±0.49 |
| CBT4026 | T7g10 | 3.35±2.3 |
| CBT4028 | BCD2 | 6.58±2.12 |
| CBT4029 | GFP | 1.77±0.12 |
| CBT4030 | Luciferase | 1.26±0.39 |

The production of IL-10 expressed in the culture supernatant and in the cells after culturing the engineered bacteria with different membrane proteins knocked out and the engineered bacteria overexpressing molecular chaperones in LB for 12 hours is shown in Table 17. Among them, the membrane proteins tolQ, tolR, tolA, pal and lpp were knocked out in the strains CBT4071, CBT4072, CBT4073, CBT4074 and CBT4020 respectively, and the control was the strain CBT4005 whose outer membrane was not modified. The results show that individually knocking out tolQ, tolR, tolA, pal or lpp could significantly improve the production and secretion efficiency of IL-10.

CBT4062 is an engineered strain in which the membrane protein lpp was knocked out and molecular chaperones were overexpressed. Overexpression of molecular chaperones was achieved by inserting the expression cassette of dsbA, dsbC, dnaK, dnaJ and grpE which under control of the BBa _J23114 promoter into the yicS/nepI site, and inserting the expression cassette of groES, groEL, tig, fkpA and surA which under control of the BBa_J23114 promoter into the yieN site of the strain. The sequence accession numbers of each molecular chaperone are shown in Table 12. Compared with the control strain CBT4028, which only has lpp knocked out but does not express molecular chaperones, the overexpression of molecular chaperones in CBT4062 can significantly increase the production of IL-10. CBT4075 is an engineered strain in which lpp and mrcA were simultaneously knocked out and molecular chaperones were overexpressed. CBT4076 is an engineered strain in which lpp and ompT were simultaneously knocked out and molecular chaperones were overexpressed. Compared with CBT4062 in which only lpp was knocked out and molecular chaperones were overexpressed, the knockout of the combination of lpp with mrcA or ompT further significantly increased IL-10 production and secretion levels. However, the strain CBT4077, in which the three membrane proteins lpp, mrcA, and ompT were simultaneously knocked out and the molecular chaperones were overexpressed, no higher IL-10 production was shown. Compared with CBT4074 in which only pal was knocked out, CBT4112, an engineered strain in which both pal and mrcA were knocked out, showed no significant change in IL-10 production. Therefore, mrcA can only improve protein secretion efficiency when lpp was also knocked out as a combination.

**Table 17 IL-10 production and genotypes of the engineered bacteria in which different membrane proteins were knocked out and molecular chaperones were overexpressed**

| Strain | Cistron | Outer membrane protein knocked out | Overexpress of molecular chaperones | IL-10 production (ng/mL) | |
|---|---|---|---|---|---|
| | | | | Supernatant | Cell lysate |
| CBT4005 | None | None | No | 4.23±0.09 | 12.06±2.37 |
| CBT4071 | None | tolQ | No | 38.39±2.14 | 9.73±2.9 |
| CBT4072 | None | tolR | No | 44.3±13.36 | 13.02±11.03 |
| CBT4073 | None | tolA | No | 34.19±0.82 | 1.75±0.14 |
| CBT4074 | None | pal | No | 38.1±1.98 | 50.32±1.28 |
| CBT4112 | None | pal, mrcA | No | 41.69±12.07 | 49.35±5.73 |
| CBT4020 | None | LPP | No | 50.18±1.29 | 171.1±4.46 |
| CBT4028 | BCD2 | LPP | No | 69.35±11.87 | 121.53±21.11 |
| CBT4062 | BCD2 | LPP | Yes | 97.71±0.36 | 106.9±11.55 |
| CBT4075 | BCD2 | LPP, mrcA | Yes | 177.21±52.8 | 163.4±11.66 |
| CBT4076 | BCD2 | LPP, ompT | Yes | 160.85±4.75 | 179.87±14.95 |
| CBT4077 | BCD2 | LPP, ompT, mrcA | Yes | 123.38±95.77 | 105.93±49.23 |

The expression of IL-10 in strain CBT4078 (EcNΔmaeB::PfnrS_BCD2_USP45_IL-10ΔLPPΔyicS/nepI::BBa_J23114_dsbA_dsbC_dnaK_dnaJ_grpEΔyie N::BBa_J23114_groES_groEL_tig_fkpA_surA) was under control of an anaerobic inducible promoter, IL-10 expression level of this strain cultured under anaerobic and aerobic conditions in LB (phosphate citrate buffer system to adjust pH=8.0)+1% w/v glucose medium were compared, and the results are shown in FIG. 2. The results show that when using an anaerobic promoter, the IL-10 production obtained by anaerobic culture was significantly higher than that of aerobic culture.

### 6.2 Construction of strains expressing IL-22

### 6.2.1 Construction of IL-22 donor gene fragment expression cassette

The IL-22 coding sequence, signal peptide, promoter, ribosome binding site (RBS), cistron, etc. were synthesized by GeneScript in cloning plasmids (such as pUC57). The IL-22 coding sequence, signal peptide, promoter, ribosome binding site (RBS) and cistron were amplified using the synthetic plasmid as a template. The LHA and RHA at the insertion site were amplified using the EcN genome as a template. The transcription terminator was amplified using the plasmid pCBT003 as a template. The PCR primers used to amplify these fragments had 15-20 bp homologous sequences with each other, such that they can be connected by overlapping PCR to obtain a donor gene fragment expression cassette connected with LHA and RHA sequences on both sides respectively. Each element was arranged in the following order from 5' to 3' ends as: 5'-promoter-ribosome binding site (RBS)-cistron-signal peptide-IL-22 coding sequence-terminator (the sequences of each constructed donor gene fragment expression cassette are set forth in SEQ ID NO: 157-163). The obtained PCR product of the donor gene fragment expression cassette connected with LHA and RHA sequences on both sides and the pCBT003_kefB_sgRNA plasmid or pCBT003_maeB_sgRNA plasmid expressing sgRNA were simultaneously transferred into competent cells containing pCBT001. Clones with the IL-22 successfully inserted into the genome were screened based on the size of the bands amplified using the verification primers of the integration sites. The production of IL-22 was measured using ELISA kit (Sino Biological, KIT13059).

The IL-22 coding sequences in this example are all the sequence encoding wild-type human IL-22 (SEQ ID NO: 15). The inserted site is maeB (CBT4038) or kefB (CBT4041, CBT4042, CBT4043, CBT4039, CBT4040, CBT4016) of the EcN genome. The sequence of the sgRNA targeting the maeB site is set forth in SEQ ID NO: 194, and the sequences of the homology arms on both sides of the maeB site are set forth in SEQ ID NO: 195 and 196 respectively. The sequence of the sgRNA targeting the kefB site is set forth in SEQ ID NO: 197, and the sequences of the homology arms on both sides of the kefB site are set forth in SEQ ID NO: 198 and 199 respectively.
Sequence of the sgRNA targeting kefB site (SEQ ID NO: 197):
   gccggaagacactatgaagc.
Sequence of the left homology arm (LHA) of the kefB site (SEQ ID NO: 198): 
ttgtttatggatgcgctggggttgtcgatggcgctcggtacgtttattgcgggtgtgctactggcggaaagtgaatatcgccatgaactggaaacgg
ctatcgatcccttcaaaggcttgctgctcggtttgttctttatctctgtcggcatgtcgctcaacctcggggtgctttatacccatctgttgtgggtagtgataagtgtggt
tgtgctggtggcggtgaaaattctcgtgctgtatctgctggcgcgattgtatggcgtgcgcagttctgagcggatgcagtttgctggcgtgttgagtcagggcggtg
agtttgcctttgtcctcttttctaccgcttcttcacaacgcttattccagggcgaccagat.
Sequence of the right homology arm (RHA) of the kefB site (SEQ ID NO: 199):

ttgcatattcttgcgcgagcgcgcggacgtgtggaagcgcatgagttattacaggcaggggtgacgcagttttcccgtgaaacattctccagtgcg
ttagagctggggcgcaagacgctggtcacgcttggcatgcatccgcatcaggcgcagcgcgcgcaactgcattttcgccgcctggatatgcgaatgctgcggg
agttaatcccgatgcatgctgataccgtacaaatttctcgcgccagggaagcccgacgtgaactggaagagattttccagcgtgaaatgcaacaagaacgacgcc
agctggacggctgggatgaatttgagt.

The specific information of each strain expressing IL-22 constructed in this example is shown in Table 18 below, and the sequences of the relevant elements are shown in Tables 2 to 9.

**Table 18 Information of elements in strains expressing IL-22**

| Name of strain | Promote r | RBS | Cistron | Signal peptide | Terminate r | Knock out protein | Overexpre ssion of molecular chaperone | SEQ ID NO of expression cassette |
|---|---|---|---|---|---|---|---|---|
| CBT4038 | BBa_J23 119 | Synthesize d | None | USP45 | rrnB_T1_ T7Te_ter minator | None | No | 157 |
| CBT4041 | BBa_J23 101 | Synthesize d | None | USP45 | rrnB_T1_ T7Te_ter minator | None | No | 158 |
| CBT4042 | BBa_J23 102 | Synthesize d | None | USP45 | rrnB_T1_ T7Te_ter minator | None | No | 159 |
| CBT4043 | BBa_J23 108 | Synthesize d | None | USP45 | rrnB_T1_ T7Te_ter minator | None | No | 160 |
| CBT4039 | BBa_J23 110 | Synthesize d | None | USP45 | rrnB_T1_ T7Te_ter minator | None | No | 161 |
| CBT4040 | BBa_J23 114 | Synthesize d | None | USP45 | rrnB_T1_ T7Te_ter minator | None | No | 162 |
| CBT4016 | BBa_J23 110 | Synthesize d | None | USP45 | rrnB_T1_ T7Te_ter minator | LPP | No | 161 |

### 6.2.2 Optimization of IL-22 expression conditions

The production of IL-22 driven by different promoters expressed in the culture supernatant and in the cells after culturing the engineered bacteria in LB for 24 hours is shown in Table 19. Among them, the BBa_J23119 promoter (inserted into the ΔmaeB site) was used in strain CBT4038; the BBa_J23101 promoter was used in strain CBT4041; the BBa_J23102 promoter was used in strain CBT4042; the BBa_J23108 promoter was used in strain CBT4043; the BBa_J23110 promoter was used in strain CBT4039; the BBa_J23114 promoter was used in strain CBT4040. The results show that IL-22 production can be regulated by selecting an appropriate promoter (such as BBa_J23119 promoter).

**Table 19 Genotypes of the engineered bacteria with different promoters and IL-22 production thereof**

| Strain | Promoter in expression cassette | IL-22 production (ng/mL) | |
|---|---|---|---|
| | | Supernatant | Cell lysate |
| CBT4038 | BBa_J23119 | 259.09±18.23 | 90.09±23.47 |
| CBT4041 | BBa_J23101 | 145.6±50.1 | 40.91±1.62 |
| CBT4042 | BBa_J23102 | 157.7±22.56 | 30.17±3.37 |
| CBT4043 | BBa_J23108 | 58.53±2.85 | 15.18±1.89 |
| CBT4039 | BBa_J23110 | 94.5±2.65 | 18.32±4.8 |
| CBT4040 | BBa_J23114 | 4.78±0.63 | 0.21±0.23 |

The production of IL-22 expressed in the culture supernatant and in the cells after culturing the engineered bacteria in LB for 24 hours is shown in Table 20, wherein different membrane proteins were knocked out and molecular chaperones were overexpressed. The results showed that the IL-22 secretion rate in strain CBT4016 with knockout of membrane protein LPP was significantly higher than that of strain CBT4039 without knockout of LPP.

**Table 20 IL-22 production and genotypes of engineered bacteria with knockout of different membrane proteins**

| Strain | Promoter in expression cassette | Outer membrane protein knockout | IL-22 production (ng/mL) | |
|---|---|---|---|---|
| | | | Supernatant | Cell lysate |
| CBT4039 | BBa_J23110 | None | 67.36±4.03 | 36.5±2.47 |
| CBT4016 | BBa_J23110 | LPP | 377.45±22.6 | 27.12±2.72 |

### 6.3 Construction of strains expressing Amuc_1100

### 6.3.1 Construction of donor expression cassette containing Amuc_1100 gene fragment

The Amuc_1100 coding sequence, signal peptide, promoter, ribosome binding site (RBS), cistron, etc. were synthesized by GeneScript in cloning plasmids (such as pUC57). The Amuc_1100 coding sequence, signal peptide, promoter, ribosome binding site (RBS) and cistron were amplified using the synthetic plasmid as a template. The LHA and RHA at the insertion site were amplified using the EcN genome as a template. The transcription terminator was amplified using the plasmid pCBT003 as a template. The PCR primers used to amplify these fragments had 15-20 bp homologous sequences with each other, such that they can be connected by overlapping PCR to obtain a donor gene fragment expression cassette connected with LHA and RHA sequences on both sides respectively. Each element was arranged in the following order from 5' to 3' ends as: 5'-promoter-ribosome binding site (RBS)-cistron-signal peptide-Amuc_1100 coding sequence-terminator (the sequences of each constructed donor gene fragment expression cassette are set forth in SEQ ID NO: 139-143). The obtained PCR product of the donor gene fragment expression cassette with LHA and RHA sequences on both sides and the pCBT003_agaI/rsmI_sgRNA plasmid or pCBT003_malP/T_sgRNA plasmid or pCBT003_pflB_sgRNA plasmid or pCBT003_lldD_sgRNA plasmid or pCBT003_maeA_sgRNA plasmid expressing sgRNA were simultaneously transferred into competent cells containing pCBT001. Clones with the Amuc_1100 successfully inserted into the genome were screened based on the size of the bands amplified using the verification primers of the integration sites.

The Amuc_1100 coding sequences in this example are sequences encoding wild-type Amuc_1100 (SEQ ID NO: 5, shown as WT in Table 21) or Y259A mutant Amuc_1100 (the numbering at position 259 is based on the numbering of sequence set forth in SEQ ID NO: 5, shown as Y259A in Table 21). When one copy of Amuc_1100 was inserted, the insertion site was the agaI/rsmI site of the EcN genome; when two copies of Amuc_1100 were inserted, the first and second insertion sites were the agaI/rsmI site and malP/T site of the EcN genome respectively; when three copies of Amuc_1100 were inserted, the first, second and third insertion sites were the agaI/rsmI site, malP/T site and pflB site respectively.

The sequence of the sgRNA targeting the agaI/rsmI site is set forth in SEQ ID NO: 200, and the sequences of the homology arms on both sides of the agaI/rsmI site are set forth in SEQ ID NO: 201 and 202 respectively. The sequence of the sgRNA targeting the malP/T site is set forth in SEQ ID NO: 203, and the sequences of the homology arms on both sides of the malP/T site are set forth in SEQ ID NO: 204 and 205 respectively. The sequence of the sgRNA targeting the pflB site is set forth in SEQ ID NO: 206, and the sequences of the homology arms on both sides of the pflB site are set forth in SEQ ID NO: 207 and 208 respectively. The sequence of the sgRNA targeting the lldD site is set forth in SEQ ID NO: 209, and the sequences of the homology arms on both sides of the lldD site are set forth in SEQ ID NO: 210 and 211 respectively. The sequence of the sgRNA targeting the maeA site is set forth in SEQ ID NO: 212, and the sequences of the homology arms on both sides of the maeA site are set forth in SEQ ID NO: 213 and 214 respectively.
Sequence of the sgRNA targeting agaI/rsmI site (SEQ ID NO: 200):
   ggcgagttaacgacgacaca.
Sequence of the left homology arm (LHA) of the agaI/rsmI site (SEQ ID NO: 201): 
gcgctaaaagctattgaagcggagccgcgcacgctgattttttatgaatctacccaccgtctgttagatagcctggaagatatcgttgcggtattag
gcgaatcccgttacgtggttctggcgcgtgagctaaccaaaacctgggaaaccattcacggcgcgcccgttggcgagctgctggcgtgggtaaaagaagatga
aaaccgtcgcaaaggcgaaatggtgctgattgtcgaaggccataaagcgcaggaaaatgacttgcctgctgatgccctgcgcacgctggcgctgctacaggca
gaactgccgctgaaaaaagcggcggcgctggccgcagaaattcacggcgtgaagaaaaatgcgctgtataagtatgcgctggagcagcagggagagtgatta
caggcgtcgcagcaggaatagcgctac.
Sequence of the right homology arm (RHA) of agaI/rsmI site (SEQ ID NO: 202): 
tcacgcatgtgaaatggttatgcagccataaaaatgaagccgggatagcggtggagactttagccgtgagaaaacgttctgtcgcatcctgttttcc
ttcgccagtcaccagtaacaaaacttcgcgggcattgagaatatccttcaggcctaaggtgatcccacgagtcacgggacggtccgcggtttttaacatctcatgtt
gctgtgttctggcatcaagttgactgatatggcaggctggttgcaggctttctcccggttcgttcagcccaagatgaccgtttttccccaatccgagaacgcataaatc
cagaccgcctttgcgcgcaatcaggttcgttacccgttcgcactctgtctcatttatctcttcggagcgaaagctgatgagctggtcttcacgcagccccagcggct
gcacgatatgctgttgtaggaaagtttcgcaggtg.
Sequence of the sgRNA targeting malP/T site (SEQ ID NO: 203):
   ttgcgtattttcaaaaagcg.
Sequence of the left homology arm (LHA) of the malP/T site (SEQ ID NO: 204): 
accagccgagattcaacaggttgttgcccgtcaggcgaccaatcaaaaactccattgagatgtagttaacatgtcgctgattcgccactggcttgg
cgaatggctgagcacgcagcatttcggccagtgcttcgctcactgccagccaccactggcgaggagtcatttcagccgcagaatttaagccataacgctgccact
gacgtgaaagcgcttcctgaaattgcttatcgttaaaaataggttgtgacataggagttccacttttcttagattttcaacacaacgttatcgctagtttgccaggctcga
tgttgaccttcctcatcctgcgggggattaggcagggaggagttgcggggatgagcaaggaaatgtgatctcgaccacttaaagctagtgcaatccacaggatta
gcagcaaatcaatgccataccgcgcagaaaatctgtatctaagtgcaaaaaatggccgtt.
Sequence of the right homology arm (RHA) of malP/T site (SEQ ID NO: 205): 
cgtattttcaaaaagcggaaggtaactctataaattaagtaaaggagtgaaacagtttcacaagtaaaatatcaagtgtgctccatctcattcttaata
gatttattaagatcatctttttagatggcactttcatcagaaatgaagaagaaccccttgcttaaatgaaactgatgaacataaggaaaataccgtacaacccagtattc
acgctggatcagcgtcgttttaggtgagttgttaataaacatttggaattgtgacacagtgcaaattcagacacataaaaaaacgtcatcgcttgcattagaaaggttt
ctggccgaccttataaccattaattacgaagcgcaaaaaaaataatatttcctcattttccacagtgaagtgattaactatgctgattccgtcaaaattaagtcgtccggt
tcgactcgaccataccgtggttcgtga.
Sequence of the sgRNA targeting pflB site (SEQ ID NO: 206):
   ccgtgacgttatccgcacca.
Sequence of the left homology arm (LHA) of the pflB site (SEQ ID NO: 207): 
acgatgacctgatgcgtccggacttcaacaacgatgactacgctattgcttgctgcgtaagcccgatgatcgttggtaaacaaatgcagttcttcgg
tgcgcgtgcaaacctggcgaaaaccatgctgtacgcaatcaacggcggcgttgacgaaaaactgaaaatgcaggttggtccgaagtctgaaccgatcaaaggc
gatgtcctgaactatgatgaagtgatggagcgcatggatcacttcatggactggctggctaaacagtacatcactgcactgaacatcatccactacatgcacgaca
agtacagctacgaagcctctctgatggcgctgcacgaccgtgacgttatcc.
Sequence of the right homology arm (RHA) of pflB site (SEQ ID NO: 208): 
gtgacgctatcccgactcagtctgttctgaccatcacttctaacgttgtgtatggtaagaaaactggtaacaccccagacggtcgtcgtgctggcgc
gccgttcggaccgggtgctaacccgatgcacggtcgtgaccagaaaggtgctgtagcgtctctgacttccgttgctaaactgccgtttgcttacgctaaagatggta
tctcctacaccttctctatcgttccgaacgcactgggtaaagacgacgaagttcgtaagaccaacctggctggtctgatggatggttacttccaccacgaagcatcc
atcgaaggtggtcagcacctgaacgttaacgtgatgaaccgtgaaatgctgctcgacgcgatggaaaacccggaaaaatatccgcagctgaccatccgtgtatct
ggctacgcagtacgtttcaactcgct.
Sequence of the sgRNA targeting the lldD site (SEQ ID NO: 209):
   gttatcgtgatgcgcattct.
Sequence of the left homology arm (LHA) of the lldD site (SEQ ID NO: 210): 
tttccgcagccagcgattatcgcgccgcagcgcaacgcattctgccgccgttcctgttccactatatggatgggggggcatattctgaatacacgc
tgcgccgcaacgtggaagatttgtcagaagtggcgctgcgccagcgtattctgaaaaacatgtctgacttaagcctggaaacgacgctgtttaatgagaaattgtc
gatgccggtggcgctaggtccggtaggtttgtgtggcatgtatgcgcgacgcggcgaagttcaggctgccaaagcagcagatgcgcatggcattccgtttactct
ctcgacggtttccgtttgcccgattgaagaagtggctccggctatcaaacgtccgatgtggttccagctttatgtgctgcgcgatcgcggctttatgcgtaacgccct
ggagcgagcaaaagccgcgggttgttcgacgctggttttcacc.
Sequence of the right homology arm (RHA) of the lldD site (SEQ ID NO: 211): 
gatccgcgatttctgggatggcccgatggtgatcaaagggatcctcgatccggaagatgcgcgcgatgcagtacgttttggtgctgatggaattgt
ggtttctaaccacggtggccgccagttagatggcgtactctcttctgctcgtgcactgcctgctattgcggatgcggtgaaaggtgatatcgccattctggcggata
gcggaatacgtaacgggcttgatgtcgtgcgtatgattgcgctcggtgccgacaccgtactgctgggtcgtgctttcctgtatgcactggcaacagcgggccagg
cgggtgtagctaatctgctaaatctgatcgaaaaagagatgaaagtggcgatgacgctgactggcgcgaaatcgattagcgaaattacgcaagattcgctggtgc
aggggctgggtaaagagttgcctgcggca.
Sequence of the sgRNA targeting maeA site (SEQ ID NO: 212):
   taacacccagcccgatgccc.
Sequence of the left homology arm (LHA) of the maeA site (SEQ ID NO: 213): 
ttctgcatagcaggtgaggcaaatgagatttattcgccactacccagtatggatgagatctgaaaaagggagaggaaaattgcccggtagccttca
ctaccgggcgcaggcttagatggaggtacggcggtagtcgcggtattcggcgtgccagaaattatcatcaatggcctgttgcagagcttcggcagacgttttcac
cgccacgccttgctgctgcgccattttgccaaccgcaaaggcaattgcgcgggagactttctgaatatctttcagttccggcagtaccagaccttcgccgttcagga
ccagcggcgaatactgagcaagcatttcacttgccgacatcagcatctc.
Sequence of the right homology arm (RHA) of the maeA site (SEQ ID NO: 214):

gagattttcgcgcttctgcaccagtttggtctggaaaggcagcaggttcggcatcttgtcggtcagcaggccaaagcgatcgaccataaagacttt
ctgccgcgccgcttcctcgcttaatccttcgcgctgggtctgggcgatgatcatttcggcaatgccgcatcccgctgaacctgcgccaaggaagacgatttttttctc
gcttaactgaccacctgccgcgcggctggctgcgatcagtgtgccgactgttaccgccgcggtgccctgaatgtcatcgttaaaagaacaaatttcattgcgatag
cggttaagtaacggcatcgcatttttttgtgcgaagtcttcaaactgcaacagtacgtccggccagcgttgtttcacagcctggataaattcatcaacaaattcatagta
ttcgtcgtcagtgatacgcggattacgccagcccatatacagcggatcgttaagcagctgttggttgttggtgccgacatccagcaccaccggaagggtataggc
cggactgatgccgccacaggcggtatagagcgacagtttaccgatcgga.

The yield of Amuc_1100 was measured by Western Blot. The information of strains is shown in Table 21. The sequences of the relevant elements are shown in Tables 2 to 9.

**Table 21 Strains expressing Amuc1100**

| Name of strain | Promoter | RBS | Cistr on | Signal peptide | Encoded protein | Terminate r | Knoc kout protei n | Overexpre ssion of molecular chaperone | SEQ ID NO of expression cassette |
|---|---|---|---|---|---|---|---|---|---|
| CBT41 01 | BBa_J23 101 | Synthes ized | None | USP45 | Y259A | rrnB_T1_ T7Te _terminato r | None | No | 139 |
| CBT41 02 | BBa_J23 110 | Synthes ized | None | USP45 | Y259A | rrnB_T1_ T7Te_terminato r | None | No | 140 |
| | BBa_J23 110 | Synthes ized | None | USP45 | Y259A | rrnB_T1_ T7Te _terminato r | | | 140 |
| CBT41 03 | BBa_J23 101 | Synthes ized | None | USP45 | Y259A | rrnB_T1_ T7Te _terminato r | None | No | 139 |
| | BBa_J23 101 | Synthes ized | None | USP45 | Y259A | rrnB_T1_ T7Te _terminato r | | | 139 |
| | BBa_J23 101 | Synthes ized | None | USP45 | Y259A | rrnB_T1_ T7Te _terminato r | | | 139 |
| CBT41 05 | BBa_J23 110 | Synthes ized | None | USP45 | WT | rrnB_T1_ T7Te _terminato r | LPP | No | 142 |
| | BBa_J23 110 | Synthes ized | None | USP45 | WT | rrnB_T1_ T7Te _terminato r | | | 142 |
| CBT41 06 | BBa_J23 110 | Synthes ized | None | USP45 | WT | rrnB_T1_ T7Te _terminato r | LPP, omp T | No | 142 |
| | BBa_J23 110 | Synthes ized | None | USP45 | WT | rrnB_T1_ T7Te _terminato r | | | 142 |
| CBT41 07 | BBa_J23 110 | Synthes ized | None | USP45 | Y259A | rrnB_T1_ T7Te _terminato r | None | No | 140 |
| CBT41 08 | BBa_J23 110 | Synthes ized | None | USP45 | Y259A | rrnB_T1_ T7Te _terminato r | None | No | 140 |
| | BBa_J23 110 | Synthes ized | None | USP45 | Y259A | rrnB_T1_ T7Te _terminato r | | | 140 |
| | BBa_J23 101 | Synthes ized | None | USP45 | Y259A | rrnB_T1_ T7Te _terminato r | | | 139 |
| CBT41 09 | BBa_J23 110 | Synthes ized | None | USP45 | Y259A | _terminato rrrnB_T1_ T7Te | None | No | 140 |
| | BBa_J23 110 | Synthes ized | None | USP45 | Y259A | rrnB_T1_ T7Te _terminato r | | | 140 |
| | BBa_J23 110 | Synthes ized | None | USP45 | Y259A | rrnB_T1_ T7Te _terminato r | | | 140 |

### 6.3.2 Optimization of Amuc_1100 expression conditions

The expression levels of Amuc_1100 (Y259A) with different number of copies were compared, and the results are shown in FIG. 3A. Among them, lanes A and B are strain CBT4101, in which one copy of Amuc_1100 (Y259A) is expressed by the BBA_J23101 promoter; lane C is strain CBT4107, in which one copy of Amuc_1100 (Y259A) is expressed by the BBA_J23110 promoter; lane D is strain CBT4102, in which two copies of Amuc_1100 (Y259A) is expressed by the BBA_J23110 promoter; lane E is strain CBT4103, in which three copies of Amuc_1100 (Y259A) is expressed by the BBA_J23101 promoter; lane F is strain CBT4108, in which there are three copies of Amuc_1100 (Y259A), one copy is driven by the BBA_J23101 promoter and the other two copies are driven by the BBA_J23110 promoter; lane G is strain CBT4109, in which three copies of Amuc_1100 (Y259A) are expressed by the BBA_J23110 promoter. This result shows that the expression of Amuc_1100 can be increased by increasing the copy number of Amuc_1100.

The stability of Amuc_1100 was improved by modifying the outer cell membrane, which was measured and compared. The results are shown in FIG. 3B. Among them, the membrane proteins LPP and ompT were knocked out in strain CBT4106; only the membrane protein LPP was knocked out in strain CBT4105. This result shows that the knockout of ompT can reduce the degradation of Amuc_1100 in the supernatant.

### 6.4 Construction of strains expressing combinations of IL-10, IL-22 and/or Amuc_1100

The preparation methods of IL-10, IL-22, and Amuc_1100 donor gene fragments are the same as those described in 6.1, 6.2, and 6.3 of this example. A strain that simultaneously expresses IL-10 and IL-22 as a combination was constructed by introducing the IL-22 donor gene fragment into a strain that expresses IL-10. A strain that simultaneously expresses IL-10 and Amuc_1100 as a combination was constructed by introducing Amuc_1100 donor gene fragment into a strain that expresses IL-10. A strain that simultaneously expresses IL-22 and Amuc_1100 as a combination was constructed by introducing the Amuc_1100 donor gene fragment into a strain that expresses IL-22. A strain that simultaneously expresses IL-10, IL-22 and Amuc_1100 as a combination was constructed by introducing the Amuc_1100 donor gene fragment into a strain that expresses both IL-10 and IL-22. The information of each strain expressing the combinations obtained in this example and the insertion elements and knockout elements therein are shown in Table 22 below. The sequences of the related elements are shown in Tables 2 to 9.

**Table 22Information of each element in strains expressing combinations**

| Nam e of strain | Insert ion site | Promot er | RBS | Cistr on | Signal peptide | Encode d protein | Terminal or | Knock out protein | Overexpres sion of molecular chaperone | SEQ ID NO of expression cassette |
|---|---|---|---|---|---|---|---|---|---|---|
| CBT 4068 | maeB | BBa_J 23110 | Synth esize d | BCD 2 | USP45 | IL-10 | rrnB_T1 _T7Te_te rminator | None | Yes | 152 |
| | agaI/ rsmI | BBa_J 23101 | Synth esize d | None | USP45 | Amuc_ 1100 (WT) | rrnB_T1 (Mutant) _T7Te_te rminator | LPP | | 141 |
| | malP/T | BBa_J 23110 | Synthesize d | None | USP45 | Amuc_1100 (WT) | rrnB_T1_T7Te_te rminator | | | 142 |
| CBT 4066 | kefB | BBa_J 23114 | Synth esize d | None | USP45 | IL-22 | rrnB_T1 _T7Te_te rminator | LPP | Yes | 162 |
| CBT 4069 | kefB | BBa_J 23114 | Synth esize d | None | USP45 | IL-22 | rrnB_T1 _T7Te_te rminator | LPP | Yes | 162 |
| | agaI/ rsmI | BBa_J 23101 | Synth esize d | None | USP45 | Amuc_ 1100 (WT) | rrnB_T1 (Mutant) _T7Te_te rminator | | | 141 |
| | malP/ T | BBa_J 23110 | Synth esize d | None | USP45 | Amuc_ 1100 (WT) | rrnB_T1 _T7Te_te rminator | | | 142 |
| CBT 4063 | kefB | BBa_J 23114 | Synth esize d | None | USP45 | IL-22 | rrnB_T1 _T7Te_te rminator | LPP | Yes | 162 |
| | maeB | BBa_J 23110 | Synth esize d | BCD 2 | USP45 | IL-10 | rrnB_T1 _T7Te_te rminator | | | 152 |
| CBT 4067 | maeB | BBa_J 23110 | Synth esize d | BCD 2 | USP45 | IL-10 | rrnB_T1 _T7Te_te rminator | LPP | Yes | 152 |
| | kefB | BBa_J 23114 | Synth esize d | None | USP45 | IL-22 | rrnB_T1 _T7Te_te rminator | | | 162 |
| | agaI/ rsmI | BBa_J 23101 | Synth esize d | None | USP45 | Amuc_ 1100 (WT) | rrnB_T1 (Mutant) _T7Te_te rminator | | | 141 |
| | malP/ T | BBa_J 23110 | Synth esize d | None | USP45 | Amuc_ 1100 (WT) | rrnB_T1 _T7Te_te rminator | | | 142 |
| CBT 4070 | agaI/ rsmI | BBa_J 23101 | Synth esize d | None | USP45 | Amuc_ 1100 (WT) | rrnB_T1 (Mutant) _T7Te_te rminator | LPP | Yes | 141 |
| | malP/ T | BBa_J 23110 | Synth esize d | None | USP45 | Amuc_ 1100 (WT) | rrnB_T1 _T7Te_te rminator | | | 142 |
| CBT 4080 | lldD | PfnrS | Synth esize d | None | USP45 | Amuc_ 1100 (WT) | rrnB_T1 _T7Te_te rminator | LPP | No | 143 |
| | mae A | PfnrS | Synth esize d | None | USP45 | Amuc_ 1100 (WT) | rrnB_T1 _T7Te_te rminator | | | 143 |

Among them, the schematic diagram of the location of each gene on the EcN chromosome of the strain expressing the combination of IL-10, IL-22 and Amuc_1100 simultaneously is shown in FIG. 4A.

### 6.5 Determination of strain growth status

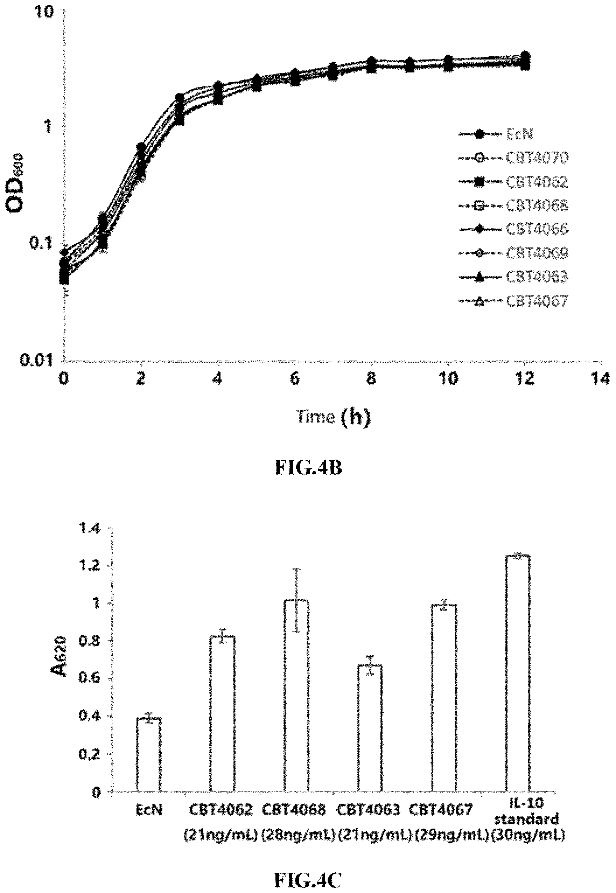

The growth curves of strains that express one gene, a combination of two genes, and a combination of three genes are shown in FIG. 4B. Among them, CBT4070 expressed Amuc_1100 alone; CBT4062 expressed IL-10 alone; CBT4068 expressed IL-10 and Amuc_1100 simultaneously; CBT4066 expressed IL-22 alone; CBT4069 expressed IL-22 and Amuc_1100 simultaneously; CBT4063 expressed IL-10 and IL-22 simultaneously; CBT4067 simultaneously expressed IL-10, IL-22 and Amuc_1100. The strain information is shown in Table 10.

The results show that the growth of the engineered bacteria expressing one gene, a combination of two genes, and a combination of three genes was not significantly affected.

### 6.6 Determination of expression levels of exogenous genes

The expression level results of IL-10 and IL-22 in the supernatant of the strains expressing one gene, a combination of two genes, and a combination of three genes are shown in Table 23. Among them, CBT4062 expressed IL-10 alone; CBT4068 expressed IL-10 and Amuc_1100 simultaneously; CBT4066 expressed IL-22 alone; CBT4069 expressed IL-22 and Amuc_1100 simultaneously; CBT4070 expressed Amuc_1100 alone; CBT4063 expressed IL-10 and IL-22 simultaneously; CBT4067 simultaneously expressed IL-10, IL-22 and Amuc_1100. This result shows that the expression of IL-10, IL-22 and Amuc_1100 by the engineered bacteria will not be reduced by the combination.

**Table 23 Genotypes of strains expressing combinations and expression levels of IL-10, IL-22 and Amuc 1100**

| Strain | Gene in expression cassette | Outer membrane protein knocked out | Overexpre ssion of molecular chaperone s | IL-10 production (ng/mL) | IL-22 production (ng/mL) | Amuc_1100 production (ng/mL) |
|---|---|---|---|---|---|---|
| CBT4062 | IL-10 | ΔLPP | Yes | 63.26±2.55 | - | |
| CBT4068 | IL-10 | ΔLPP | Yes | 81.4±1.3 | - | 506.69±39.429 |
| | Amuc_1100 (2 copies) | | | | | |
| CBT4066 | IL-22 | ΔLPP | Yes | - | 71.28±9.83 | |
| CBT4069 | IL-22 | ΔLPP | Yes | - | 103.62±8.4 | 483.12±173.9 |
| | Amuc_1100 (2 copies) | | | | | |
| CBT4063 | IL-22 | ΔLPP | Yes | 81.24±18.39 | 59.06±1.41 | |
| | IL-10 | | | | | |
| CBT4067 | IL-10 | ΔLPP | Yes | 79±9.1 | 93.59±6.12 | 571.72±29.19 |
| | IL-22 | | | | | |
| | Amuc_1100 (2 copies) | | | | | |
| CBT4070 | Amuc_1100 (2 copies) | ΔLPP | Yes | - | - | 653.71±151.95 |

### Example 7: Activity assay of expressed proteins

### 7.1 IL-10 activity detection

The samples to be detected were diluted to an appropriate concentration with complete cell culture medium. 20 µL of the diluted sample and IL-10 standard was added to a flat-bottom 96-well plate respectively, wherein the EcN sample was used as a negative control. A cell suspension of HEK-Blue^{™} IL-10 Cells (InvivoGen, Cat # hkb-il10) at about 280,000 cells/mL was prepared, and 180 µL of this suspension (about 50,000 cells) was quickly added to the wells to which the sample had been added. The 96-well plate was incubated at 5% CO₂, 37°C for 22 hours. Then 20 µL of the supernatant was added to a new flat-bottom 96-well plate, and 180 µL of QUANTI-Blue reagent was added to each well. The value of A₆₂₀ was read after an hour of incubation at 37°C.

The cellular activity of IL-10 in the culture supernatant of strains expressing one gene, a combination of two genes, and a combination of three genes is shown in FIG. 4C. Among them, the negative control is supernatant of EcN, which does not express IL-10. The IL-10 standard had a concentration of 30 ng/mL. CBT4062 expressed IL-10 alone, and the concentration of the sample was diluted to 21 ng/mL. CBT4068 expressed IL-10 and Amuc_1100 simultaneously, and the sample concentration was diluted to 28 ng/mL. CBT4063 expressed IL-10 and IL-22 simultaneously, and the concentration of the sample was diluted to 21 ng/mL. CBT4067 expressed IL-10, IL-22 and Amuc_1100 simultaneously, and the concentration of the sample was diluted to 29 ng/mL.

The results show that the IL-10 secreted by the engineered bacteria expressing combinations of genes shows a biological activity, which is comparable to that of the standard product.

### 7.2 IL-22 activity detection

The samples to be detected were diluted to an appropriate concentration with complete cell culture medium. 20 µL of the diluted sample and IL-22 standard was added to a flat-bottom 96-well plate respectively, wherein the EcN sample was used as a negative control. A cell suspension of HEK-Blue^{™} IL-22 Cells (InvivoGen, Cat # hkb-il22) at about 280,000 cells/mL was prepared, and 180 µL of this suspension (about 50,000 cells) was quickly added to the wells to which the sample had been added. The 96-well plate was incubated at 5% CO₂, 37°C for 22 hours. Then 20 µL of the supernatant was added to a new flat-bottom 96-well plate, and 180 µL of QUANTI-Blue reagent was added to each well. The value of A₆₂₀ was read after an hour of incubation at 37°C.

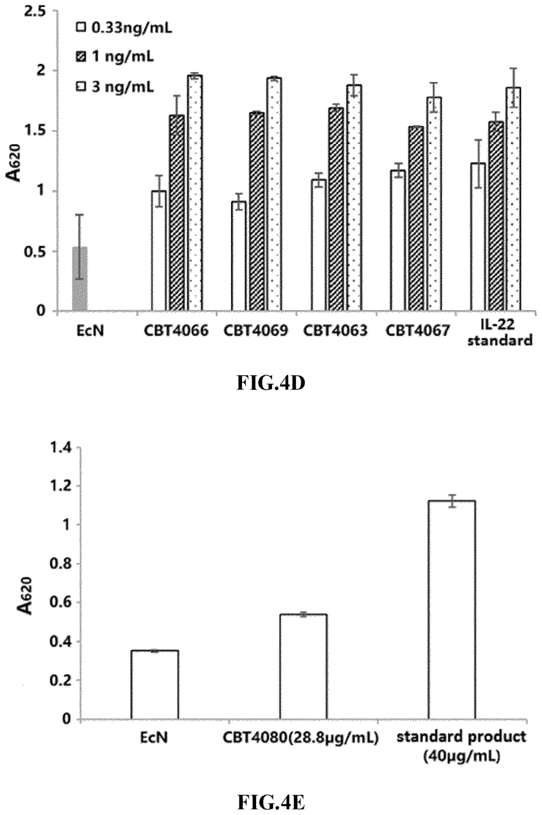

The cellular activity of IL-22 in the supernatant of strains expressing one gene, a combination of two genes, and a combination of three genes is shown in FIG. 4D. Among them, the negative control is supernatant of EcN, which does not express IL-22. CBT4066 expressed IL-22 alone. CBT4069 expressed IL-22 and Amuc_1100 simultaneously. CBT4063 expressed IL-22 and IL-10 simultaneously. CBT4067 expressed IL-10, IL-22 and Amuc_1100 simultaneously. The concentrations of IL-22 standard and each strain sample were diluted to 0.33 ng/mL, 1 ng/mL and 3 ng/mL.

The results show that the IL-22 secreted by the engineered bacteria expressing combinations of genes has biological activity, which is comparable to that of the standard product.

### 7.3 Amuc_1100 activity detection

The samples to be detected were diluted to an appropriate concentration with complete cell culture medium. 20 µL of the diluted sample and Amuc_1100 standard was added to a flat-bottom 96-well plate respectively, wherein the EcN sample was used as a negative control. A cell suspension of HEK-Blue^{™} hTLR2 Cells (InvivoGen, Cat # hkb-htlr2) at about 280,000 cells/mL was prepared, and 180 µL of this suspension (about 50,000 cells) was quickly added to the wells to which the sample had been added. The 96-well plate was incubated at 5% CO₂, 37°C for 22 hours. Then 20 µL of the supernatant was added to a new flat-bottom 96-well plate, and 180 µL of QUANTI-Blue reagent was added to each well. The value of A₆₂₀ was read after an hour of incubation at 37°C.

Strain CBT4080 was selected as the subject for determination. The activity of Amuc_1100 secreted by strain CBT4080 in the supernatant was determined. The results are shown in FIG. 4E. Among them, the negative control is the purified product from the supernatant of EcN, where Amuc_1100 was not expressed. The concentration of Amuc_1100 standard product was 40 µg/mL. The purity of CBT4080 purified sample was about 70%, and the concentration was 28.8 µg/mL. The results show that the Amuc_1100 expressed and secreted by the engineered bacteria had biological activity, which is comparable to that of the standard product.

### Example 8: Construction of strains expressing a combination of IL-10, IL-22 and/or Amuc_1100 in anaerobic induction

The preparation methods of IL-10, IL-22, and Amuc_1100 donor gene fragments are the same as those described in 6.1, 6.2, and 6.3 of Example 6. A strain that expresses a combination of IL-10 and IL-22 simultaneously was constructed by introducing IL-22 donor gene fragment into a strain that expressed IL-10. A strain that expresses a combination of IL-10 and Amuc_1100 simultaneously was constructed by introducing Amuc_1100 donor gene fragment into a strain that expressed IL-10. A strain that expresses a combination of IL-22 and Amuc_1100 simultaneously was constructed by introducing Amuc_1100 donor gene fragment into a strain that expressed IL-22. A strain that expresses a combination of IL-10, IL-22 and Amuc_1100 simultaneously was constructed by introducing Amuc_1100 donor gene fragment into a strain that expressed both IL-10 and IL-22. The information of each strain expressing combinations obtained in this example and the insertion elements and knockout elements therein is shown in Table 24 below, and the sequences of the related elements are shown in Tables 2 to 9.

**Table 24 Information of each element in the strains expressing gene combinations in Example 8**

| Name of strain | Inserti on site | Promot er | RBS | Cistr on | Signal peptide | Encode d protein | Terminal or | Knock out protein | Overexpr ession of molecular chaperone | SEQ ID NO of expressi on cassette |
|---|---|---|---|---|---|---|---|---|---|---|
| CBT40 84 | maeB | PfnrS | Synthe sized | None | USP45 | IL-10 | rrnB_T1 _T7Te_t erminato r | LPP, mrcA | Yes | 156 |
| CBT40 95 | kefB | PfnrS | Synthe sized | None | USP45 | IL-22 | rrnB_T1 _T7Te_t erminato r | LPP, mrcA | Yes | 163 |
| CBT40 96 | agaI/ rsmI | PfnrS | Synthe sized | None | USP45 | Amuc_ 1100 (WT) | rrnB_T1 _T7Te_t erminato r | LPP, mrcA | Yes | 143 |
| CBT40 88 | maeB | PfnrS | Synthe sized | None | USP45 | IL-10 | rrnB_T1 _T7Te_t erminato r | LPP, mrcA | Yes | 156 |
| | agaI/ rsmI | PfnrS | Synthe sized | None | USP45 | Amuc_ 1100 (WT) | rrnB_T1 _T7Te_t erminato r | | | 143 |
| CBT40 98 | kefB | PfnrS | Synthe sized | None | USP45 | IL-22 | rrnB_T1 _T7Te_t erminato r | LPP, mrcA | Yes | 163 |
| | agaI/ rsmI | PfnrS | Synthe sized | None | USP45 | Amuc_ 1100 (WT) | rrnB_T1 _T7Te_t erminato r | | | 143 |
| CBT41 10 | maeB | PfnrS | Synthe sized | None | USP45 | IL-10 | rrnB_T1 _T7Te_t erminato r | LPP, mrcA | Yes | 156 |
| | kefB | BBa_J 23110 | Synthe sized | None | USP45 | IL-22 | rrnB_T1 _T7Te_t erminato r | | | 161 |
| CBT41 11 | maeB | PfnrS | Synthe sized | None | USP45 | IL-10 | rrnB_T1 _T7Te_t erminato r | LPP, mrcA | Yes | 156 |
| | kefB | BBa_J 23110 | Synthe sized | None | USP45 | IL-22 | rrnB_T1 _T7Te_t erminato r | | | 161 |
| | agaI/ rsmI | PfnrS | Synthe sized | None | USP45 | Amuc_ 1100 (WT) | rrnB_T1 _T7Te_t erminato r | | | 143 |

The strain used in the experiment was cultured in LB (phosphate citric acid buffer system to adjust pH = 8.0) + 1% w/v glucose medium aerobically for 4 hours and anaerobically for 2 hours. The expression levels of IL-10, IL-22 and Amuc_1100 in the culture supernatant are shown in Table 25.

**Table 25 Expression levels of IL-10, IL-22, and Amuc_1100 in the strains used in the experiment**

| Strain | Gene in expression cassette | Outer membrane protein knocked out | Overexpres sion of molecular chaperones | IL-10 production(ng /mL/OD₆₀₀) | IL-22 production(ng/ mL/OD₆₀₀) | Amuc_1100 production(ng/ mL/OD₆₀₀) |
|---|---|---|---|---|---|---|
| CBT4084 | IL-10 | LPP, mrcA | Yes | 575.78±113.7 7 | - | |
| CBT4095 | IL-22 | LPP, mrcA | Yes | - | 4118.86±1137. 64 | - |
| CBT4096 | Amuc_11 00 | LPP, mrcA | Yes | - | - | 1367.93±382.9 1 |
| CBT4088 | IL-10 | LPP, mrcA | Yes | 394.76±51.09 | | 3574.56±876.3 5 |
| | Amuc_11 00 | | | | | |
| CBT4098 | IL-22 | LPP, mrcA | Yes | - | 4526.74±1978. 42 | 2313.41±560.9 7 |
| | Amuc_11 00 | | | | | |
| CBT4110 | IL-10 | LPP, mrcA | Yes | 932.18±206.2 5 | 3268.59±281.3 7 | - |
| | IL-22 | | | | | |
| CBT4111 | IL-10 | LPP, mrcA | Yes | 464.77±4.65 | 1904.9±361.77 | 4589.59±1359. 36 |
| | IL-22 | | | | | |
| | Amuc_11 00 | | | | | |

### Example 9: Construction of plasmid for salicylic acid-induced expression of IL-10

Salicylic acid-inducible promoter Psal, ribozyme (sTRSV-HHRz), ribosome binding site (RBS), salicylic acid-inducible promoter repressor expression cassette 5'-PlacIQ-NahRAM-ter-3', etc. were synthesized by GeneScript in cloning plasmids (e.g. pUC57). The 5'-USP45_IL-10_rrnB_T1_T7Te-3' sequence of the IL-10 expression cassette without a promoter was amplified using the CBT4084 strain genome as a template. Psal_sTRSV-HHRz_RBS and PlacIQ_NahRAM_ter were amplified using the synthetic plasmid as a template. The LHA and RHA of the maeB insertion site were amplified using the genome of EcN as a template. The sgRNA of the maeB site was amplified using the plasmid pCBT003_maeB_sgRNA as a template. The plasmid backbone was amplified using pCBT010 as a template. The PCR primers used to amplify these fragments had 15-20 bp homologous sequences with each other and can therefore be connected using the kit ClonExpress Ultra One Step Cloning Kit (Vazyme) to generated a plasmid pCBT010_maeB_Psal_IL-10 containing an expression cassette 5'-Psal-sTRSV HHRz_RBS_USP45_IL-10_rrnB_T1_T7Te-3' connected to LHA at one end, an expression cassette 5'-PlacIQ-NahRAM-ter-3' (the sequence thereof is set forth in SEQ ID NO: 243) connected to RHA at the other end, and sgRNA targeting the maeB site. The plasmid pCBT010_maeB_Psal_IL-10 was transformed into competent cells containing pCBT001. The obtained single colony was amplified with the primers for the verification of the insertion site. Clones with IL-10 successfully inserted into the genome were screened based on the size of the amplification bands. The production of IL-10 was determined using ELISA kit (Sino Biological, KIT10947A).

PlacIQ-NahRAM-ter sequence (SEQ ID NO: 243):

atggcggcgcgccatcgaatggtgcaaaacctttcgcggtatggcatgatagcgcccggaagagagtcaattcatgggggttgaatatggaact
gcgtgacctggatttaaacctgctggtggtgttcaaccagttgctggtcgacagacgcgtctctgtcactgcggagaacctgggcctgacccagcctgccgtgag
caatgcgctgaaacgcctgcgcacctcgctacaggacccactcttcgtgcgcacacatcagggaatggaacccacaccctatgccgcgcatctggccgagcac
gtcacttcggccatgcacgcactgcgcaacgccctacagcaccatgaaagcttcgatccgctgaccagcgagcgtaccttcaccctggccatgaccgacattgg
cgagatctacttcatgccgcggctgatggatgcgctggctcaccaggcccccaattgcgtgatcagtacggtgcgcgacagttcgatgagcctgatgcaggcctt
gcagaacggaaccgtggacttggccgtgggcctgcttcccaatctgcaaactggcttctttcagcgccggctgctccagaatcactacgtgtgcctatgtcgcaag
gaccatccagtcacccgcgaacccctgactctggagcgcttctgttcctacggccacgtgcgtgtcatcgccgctggcaccggccacggcgaggtggacacgt
acatgacacgggtcggcatccggcgcgacatccgtctggaagtgccgcacttcgccgccgttggccacatcctccagcgcaccgatctgctcgccactgtgcc
gatatgtttagccgactgctgcgtagagcccttcggcctaagcgccttgccgcacccagtcgtcttgcctgaaatagccatcaacatgttctggcatgcgaagtacc
acaaggacctagccaatatttggttgcggcaactgatgtttgacctgtttacggattgataaggatcctaattggtaacgaatcagacaattgacggctcgagggagt
agcatagggtttgcagaatccctgcttcgtccatttgacaggcacattatgcatcgatgataagctgtcaaacatgagcagatcctctacgccggacgcatcgtggc
cggcatcaccggcgccacaggtgcggttgctggcgcctatatcgccgacatcaccgatggggaagatcgggctcgccacttcgggctcatgagcaaatattttat
ctg.

**Table 26 Information of each element in the strain used in Example 9**

| Name of strain | Inserti on site | Promot er | RBS | Ribo zyme | Signal peptide | Encode d protein | Terminal or | Knock out protein | Overexpr ession of molecular chaperone | Expressi on cassette SEQ ID NO |
|---|---|---|---|---|---|---|---|---|---|---|
| CBT41 13 | maeB | Psal | Synthe sized | sTRS V-H HRz | USP45 | IL-10 | rrnB_T1 _T7Te_t erminato r | LPP, mrcA | Yes | 235 |

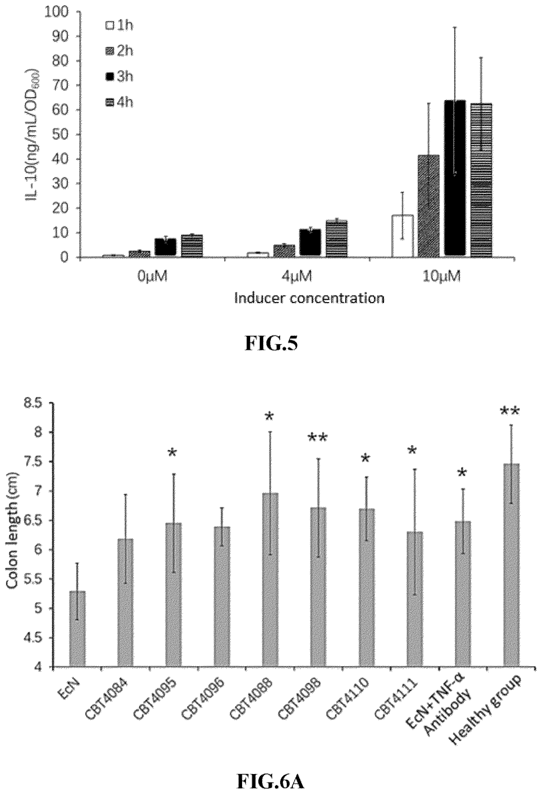

Strain CBT4113 was cultured aerobically in LB medium for 3 hours, then added with different concentrations of sodium salicylate, and then cultured continuously for 1-4 hours. The expression level of IL-10 in the culture supernatant is shown in FIG. 5. The results show that precise control of IL-10 expression level can be achieved by adjusting the concentration of the inducer and the induction duration.

### Example 10: Expression of other cytokines

In addition, the inventors further tried a variety of other cytokines that also have immunomodulatory effects, such as IL-23 (IL-12p40_linker1_IL-23p19), IL-17A, IL-19, IL-35 (EBI3_linker2_IL-12p35), IL-37 and TGF-β, and constructed engineered bacteria in an attempt to study their effects *in vivo.* The process is as follows: the coding sequences of IL-23 (IL-12p40_linker1_IL-23p19), IL-17A, IL-19, IL-35 (EBI3_linker2_IL-12p35), IL-37, and TGF-β were amplified using the synthesized plasmid as a template. The plasmid backbone sequence of 5'-rrnB_T1_T7Te_PlacIQ_NahRAM_ter-3' comprising a salicylic acid-inducible promoter Psal, ribozyme (sTRSV-HHRz), ribosome binding site (RBS), 5'-Psal_sTRSV-HHRz_RBS_USP45-3' sequence of the secretion signal peptide USP45, a transcription terminator rmB_T1_T7Te and a salicylic acid regulatory element PlacIQ-NahRAM-ter was amplified using plasmid pCBT010_maeB_Psal_IL-10 as a template. The PCR primers used to amplify these fragments had 15-20 bp homologous sequences with each other and can therefore be connected using the ClonExpress Ultra One Step Cloning Kit (Vazyme) to generate plasmids pCBT010_Psal_IL-23, pCBT010_Psal_IL-17A, pCBT010_Psal_IL-19, pCBT010_Psal_IL-35, pCBT010_Psal_IL-37 and pCBT010_Psal_TGF-β containing expression cassettes 5'-Psal-sTRSV HHRz_RBS_USP45_cytokine_rrnB_T1_T7Te-3' and 5'-PlacIQ-NahRAM-ter-3'. These plasmids were transformed into competent cells of the bacterial strain CBT4114 (genotypes are shown in Table XX). The single colony obtained was amplified with the primers for the verification of the plasmids, and the plasmids which get into the cell successfully was verified by the size of the amplified bands. The production of cytokines was determined using an ELISA kit. The kits used in this example are shown in Table 27.

**Table 27 Cytokine detection kit**

| Brand | Cat. No. | Name |
|---|---|---|
| MULTISCIENCES | EK1177-48 | Human IL-17AF ELISA Kit |
| MULTISCIENCES | EK123-48 | Human IL-23 ELISA Kit |
| Abclonal | RK00175 | Human IL-19 ELISA kit |
| MULTISCIENCES | EK135-48 | Human IL-35 ELISA kit |
| Abclonal | RK00117 | Human IL-37 ELISA kit |
| MULTISCIENCES | EK981-48 | Human/Mouse/Rat TGF-β1 ELISA Kit |

The strains carried these plasmids expressing cytokines were cultured aerobically in LB medium for 4 hours, then added with 100 µM sodium salicylate, and continuously cultured for 2 hours. The cytokine contents in the supernatant and in the cells are shown in Table 28.

**Table 28 Cytokine production of each strain induced by salicylic acid**

| Strain | Expression vector | Gene in expression cassette | Outer membrane protein knocked out | Overexpres sion of molecular chaperones | Production in supernatant (ng/mL/OD ₆₀₀) | Production in cells (ng/mL/O D₆₀₀) |
|---|---|---|---|---|---|---|
| CBT4114 | Plasmid pCBT010_Psal IL-23 | IL-23 | LPP, mrcA | Yes | 0.01±0.00 | 0.02±0.00 |
| CBT4114 | Plasmid pCBT010_Psal _IL-17A | IL-17A | LPP, mrcA | Yes | 0.14±0.02 | 1.35±0.15 |
| CBT4114 | Plasmid pCBT010_Psal _IL-19 | IL-19 | LPP, mrcA | Yes | 0.23±0.06 | 0.73±0.00 |
| CBT4114 | Plasmid pCBT010_Psal _IL-35 | IL-35 | LPP, mrcA | Yes | 0.65±0.01 | 3.89±0.19 |
| CBT4114 | Plasmid pCBT010_Psal _IL-37 | IL-37 | LPP, mrcA | Yes | 0.08±0.02 | 3.1±0.36 |
| CBT4114 | Plasmid pCBT010_Psal _TGF-β | TGF-β | LPP, mrcA | Yes | 0.09±0.01 | 0.14±0.02 |

It is unexpectedly found that the expression and secretion amounts of the above-mentioned cytokines were too low to reach the effective concentration. It is speculated that EcN may have poor tolerance to these proteins or the above-mentioned proteins were unstable after expression. Therefore, the above-mentioned factors are not suitable for use in EcN for expression and secretion.

### Example 11: Drug efficacy assay in inflammatory bowel disease (IBD) mouse model induced by T cell transplantation

The inflammatory bowel disease mice used in this experiment were induced by injecting purified CD4⁺CD45RB^{high} cells, which did not contain regulatory T cells, into severe combined immunodeficiency (SCID) mice, which lacked T cells and B cells. Balb/C mice (20 g, 8-10 weeks old) were used to prepare CD4⁺CD45RB^{low} and CD4⁺CD45RB^{high} cells, and RAG1-/- mice (20 g, 8-10 weeks old) were used to receive T cell transplantation. RAG1-/- mice were randomly divided into 11 groups, with 10 mice in each group. Group 1 was the healthy group and transplanted with CD4⁺CD45RB^{low} cells, and groups 2-11 were the disease groups and transplanted with CD4⁺CD45RB^{high} cells. The day of T cell transplantation was recorded as day 0, and administration began on day 14. The culture of EcN, CBT4084, CBT4095, CBT4096, CBT4088, CBT4098, CBT4110 and CBT4111 incubated aerobically in LB for 4 hours was concentrated and resuspended in 0.2 M sodium bicarbonate + 1% w/v glucose solution to obtain a bacterial suspension, respectively. Group 2 was the positive administration group. TNF-α antibody was injected intraperitoneally every 7 days from days 14 to 37, and 100 µL of EcN bacterial suspension was intragastrically administered every day. 100 µL of bacterial suspension of EcN, CBT4084, CBT4095, CBT4096, CBT4088, CBT4098, CBT4110 and CBT4111 was administered to groups 3-11 intragastrically, once a day, at an amount of 2.5 × 10⁹ CFU per mouse. On the 41st day, the mice were sacrificed, the length of the intestines was measured, and HE staining was performed for pathological analysis.

In each experimental group, the colon length at the efficacy endpoint in the inflammatory bowel disease mouse model induced by T cell transplantation is shown in FIG. 6A. Among them, CBT4096 expressed Amuc_1100 alone; CBT4084 expressed IL-10 alone; CBT4088 expressed IL-10 and Amuc_1100 simultaneously; CBT4095 expressed IL-22 alone; CBT4098 expressed IL-22 and Amuc_1100 simultaneously; CBT4110 expressed IL-10 and IL-22 simultaneously; CBT4111 simultaneously expressed IL-10, IL-22 and Amuc_1100. The results show that the engineered bacteria expressing single protein alone or combination of proteins were all effective in maintaining the colon length in inflammatory bowel disease model mice. The statistical analysis showed that the colon length of all engineered bacteria expressing combination of proteins was significantly longer than that of the control group (p<0.05). The effect was comparable to that of the positive control drug TNF-α antibody, indicating that the therapeutic effect of the engineered bacteria expressing protein combination in the inflammatory bowel disease mouse model induced by T cell transplantation was better than that of the engineered bacteria expressing a single protein alone.

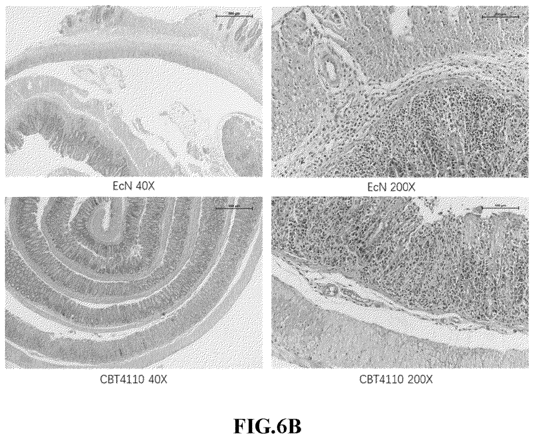

HE staining results of mice colon samples of the EcN control group and CBT4110 expressing both IL-10 and IL-22 are shown in FIG. 6B. The colon inflammation of CBT4110 mice was significantly less than that of the control group.

### Example 12: Drug efficacy assay in DSS-induced inflammatory bowel disease (IBD) mouse model

In addition to the mouse intestinal model in Example 11, the therapeutic effect of the engineered bacteria of the present invention was further verified in DSS-induced mouse model of inflammatory bowel disease. 35 C57/B6 mice (8 weeks old, 18-20 g, female) were randomly divided into 7 model groups, with 5 mice in each group. The mice started to drink 2.5% DSS on day 0 for 4 days and then switched to drink normal water for 3 days for a total of 4 rounds of DSS induction. The first group was the model control group, which was not administered with drugs, and the second group was the positive drug group, which was administered intragastrically with positive drug CsA every day from day 0 to day 27 at an amount of 25 mpk. The culture of EcN, CBT4096, CBT4088, CBT4098 and CBT4110 incubated aerobically in LB for 4 hours were concentrated and resuspended with 0.2 M sodium bicarbonate + 1% w/v glucose solution to obtain a bacterial suspension, respectively. 100 µL of bacterial suspensions of EcN, CBT4096, CBT4088, CBT4098 and CBT4110 were administered to groups 3-7 respectively, once a day, at an amount of 2.5×10⁹CFU per mouse, from day 0 to day 27 days, with day 28 as the end point of the experiment. The mice were sacrificed and the length of the intestines was measured.

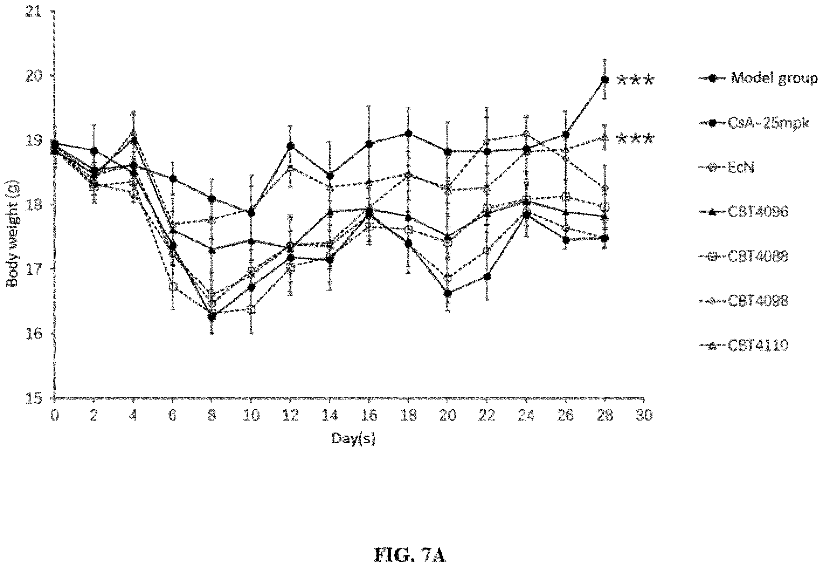

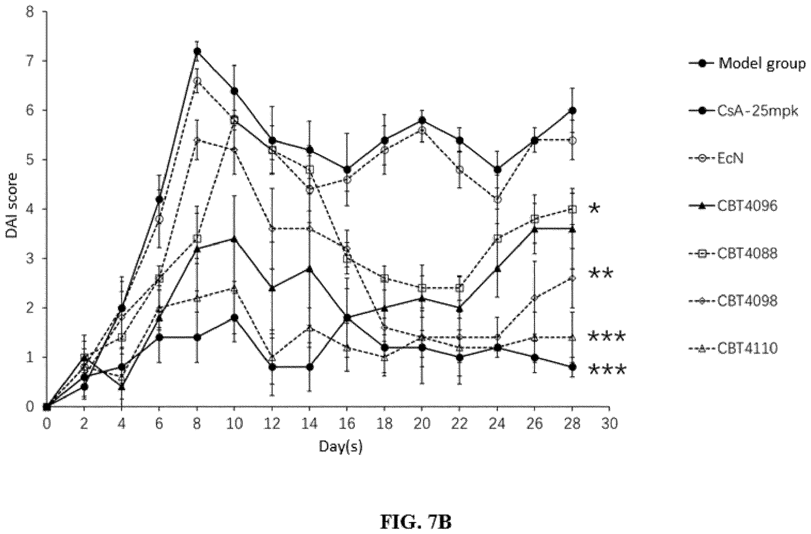

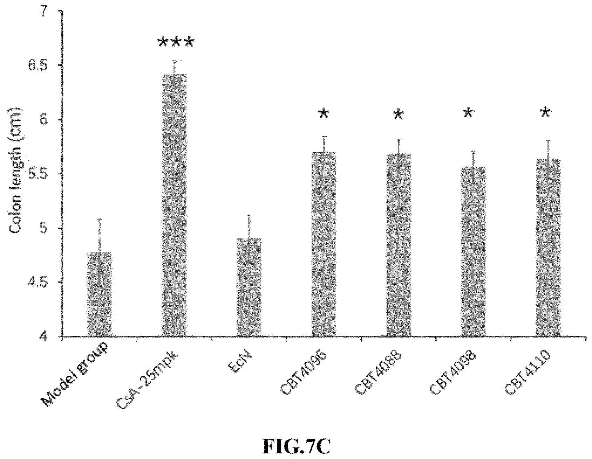

The body weight changes, DAI score (Disease activity index, also a commonly used indicator for evaluating inflammatory bowel disease) and colon length at drug efficacy endpoint in the DSS-induced inflammatory bowel disease mouse model are shown in FIG. 7. Compared with the EcN control group, the colon length in the engineered bacteria CBT4096, CBT4088, CBT4098, and CBT4110 groups all increased significantly (p<0.05) (FIG. 7C), wherein in terms of maintaining body weight (FIG. 7A) and reducing DAI score (FIG. 7B), CBT4110 had more significant advantages (p<0.001).

### Example 13: Drug efficacy assay in GvHD model

BALB/c (H-2d) mice and C57BL/6 (H-2b) mice (6-8 weeks old) were used as donors and recipients, respectively. The day of bone marrow transplantation (BMT) was recorded as day 0, and the day of radiation was day -1. There were 7 syngeneic transplanted mice in group 1, 9 allogeneic transplanted mice in groups 2-10, and 7 mice received irradiation but not transplantation in group 11. The culture of EcN, CBT4084, CBT4095, CBT4096, CBT4088, CBT4098, CBT4110 and CBT4111 incubated aerobically in LB for 4 hours were concentrated and resuspended in 0.2 M sodium bicarbonate + 1% w/v glucose solution to obtain a bacterial suspension, respectively. From day -1 to day 30, mice in groups 1, 2, 10 and 11 were administered intragastrically with 100 µL of EcN cell suspension containing 2.5 × 10⁹ CFU. Meanwhile, mice in group 10 were injected with 20 mg/kg prednisone every day. 100 µL of the bacterial suspensions of CBT4084, CBT4095, CBT4096, CBT4088, CBT4098, CBT4110 and CBT4111 was administered intragastrically to groups 3-9 respectively. The survival rate of the mice was recorded every day.

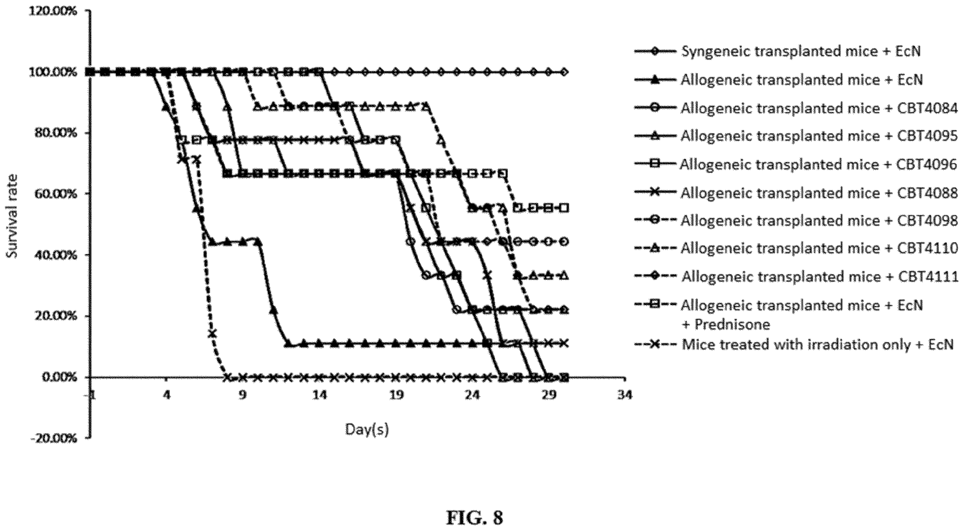

The survival rate of the GvHD animal model is shown in FIG. 8. The results show that on day 20, the mice in groups of CBT4096 expressing Amuc_1100 alone, CBT4098 expressing IL-22 and Amuc_1100 simultaneously, CBT4110 expressing IL-10 and IL-22 simultaneously, and CBT4111 expressing IL-10, IL-22 and Amuc_1100 simultaneously all had the same or even higher survival rate as the positive drug group. On day 25, the mice in groups of CBT4088 simultaneously expressing IL-10 and Amuc_1100, CBT4098 simultaneously expressing IL-22 and Amuc_1100, CBT4110 simultaneously expressing IL-10 and IL-22, and CBT4111 simultaneously expressing IL-10, IL-22 and Amuc_1100 all had a higher survival rate than that of the mice in groups of CBT4084 expressing IL-10 alone, CBT1095 expressing IL-22 alone, and CBT4096 expressing Amuc_1100 alone, indicating that the engineered bacteria expressing the combinations of proteins have better efficacy than the engineered bacteria expressing a single protein alone to varying degrees in the GvHD model. At the end of the 30th day of the experiment, the survival rate of the CBT4098 group expressing both IL-22 and Amuc_1100 was still close to that of the positive drug group. It can be seen that the engineered bacteria expressing IL-22 and Amuc_1100 had the most significant therapeutic effect on GvHD.

### Example 14: Drug efficacy assay in SLE model

First, the therapeutic effects of the engineered bacteria expressing two proteins simultaneously and the engineered bacteria expressing one protein alone were evaluated in the SLE model. The SLE model was established using MRL/MpJ-Faslpr mice, and MRL/MpJ mice were used as negative control. The mice were administered for 8 weeks. The culture of EcN, CBT4084, CBT4096 and CBT4088 incubated aerobically in LB for 4 hours were concentrated and resuspended in 0.2 M sodium bicarbonate + 1% w/v glucose solution to obtain a bacterial suspension, respectively. The experimental mice were divided into 6 groups. The first group was the negative control group, including 5 MRL/MpJ mice. The second group was the positive drug group, including 10 MRL/MpJ-Faslpr mice, wherein the mice were administered intragastrically every day with 9 mg/kg prednisone and 100 µL of EcN bacterial suspension. Groups 3-6 were drug administration groups, each including 10 MRL/MpJ-Faslpr mice, wherein the mice were administered intragastrically every day with 100 µL of the bacterial suspensions of EcN, CBT4084, CBT4096 and CBT4088 respectively at an amount of 2.5×10⁹CFU per mouse. After the experiment, the kidneys were taken and prepared into sections to evaluate the renal tubular damage.

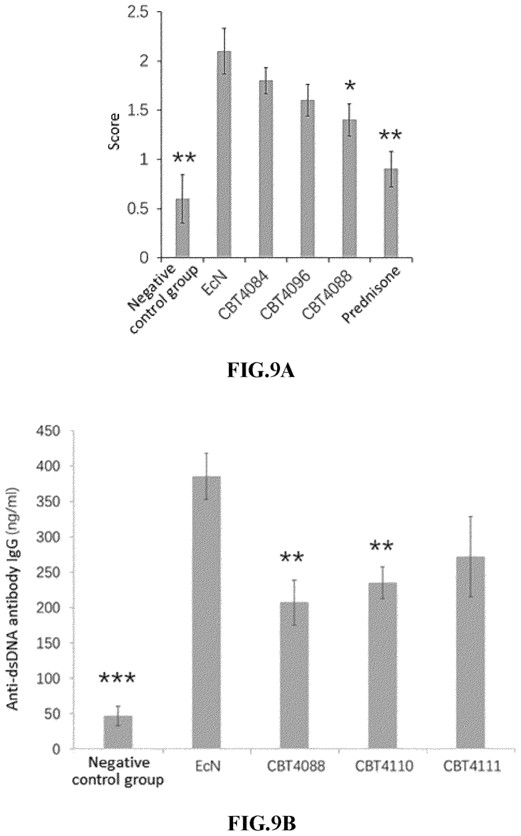

The renal tubular damage is shown in FIG. 9A. Compared with the control EcN, the engineered strain CBT4084 that expressed IL-10 alone and the engineered strain CBT4096 that expressed Amuc_1100 alone cannot significantly reduce the damage of renal tubules. However, the engineered strain CBT4088 that expressed both IL-10 and Amuc_1100 can significantly reduce renal tubular damage, indicating that the simultaneous expression of IL-10 and Amuc_1100 produced a synergistic effect on the SLE model.

Subsequently, the therapeutic effects of the engineered bacteria that simultaneously expressed IL-10 and Amuc_1100 (CBT4088), the engineered bacteria that simultaneously expressed IL-10 and IL-22 (CBT4110), and the engineered bacteria that simultaneously expressed IL-10, IL-22 and Amuc_1100 (CBT4111) were further compared. The experimental mice were divided into 5 groups. Group 1 was the negative control group, including 6 MRL/MpJ mice, and groups 2-5 were drug administration groups, each including 10 MRL/MpJ-Faslpr mice, wherein the mice were administered with 100 µL of the bacterial suspensions of EcN, CBT4088, CBT4110 and CBT4111 intragastrically every day at an amount of 1.0×10¹⁰CFU per mouse. At the end of the experiment, the concentration of anti-double stranded DNA antibody IgG in serum and the concentration of albumin in urine were measured. Kidney sections were prepared for PAS staining to evaluate glomerular damage.

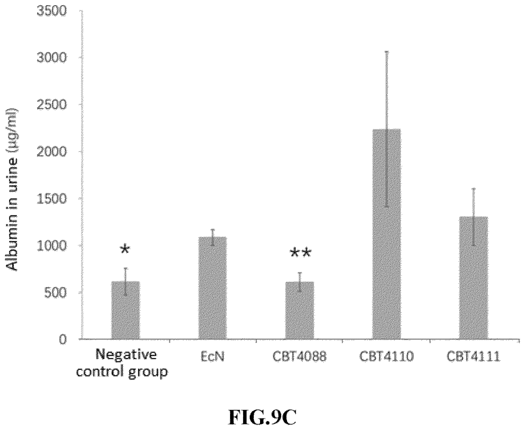

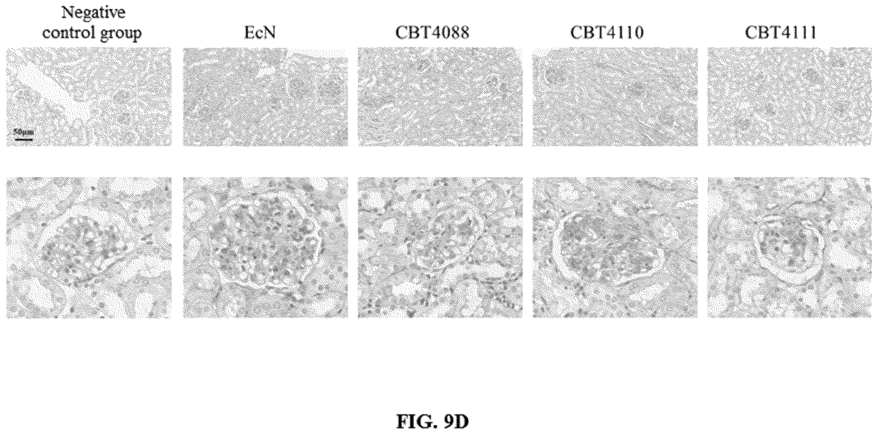

First, the concentration changes of anti-double stranded DNA antibody IgG, a serological marker of SLE, in mouse serum were measured. The results are shown in FIG. 9B. Both CBT4088 and CBT4110 can significantly reduce the concentration of the anti-double stranded DNA antibody IgG in the serum of model mice (p<0.01), with CBT4088 showing the most reduction. Kidney PAS staining showed that in the EcN group, the glomerular capillary endothelial cells and mesangial cells of the diseased mice significantly proliferated, the glomerular cavity was narrowed or occluded, and the glomerular function was lost, while the pathological sections of CBT4088 and CBT4111 showed significant improvement (FIG. 9D). However, only CBT4088 could significantly reduce the concentration of urinary albumin (an indicator of kidney damage) (p<0.01) (FIG. 9C). The above results show that the engineered bacteria CBT4088, which simultaneously expressed IL-10 and Amuc_1100, had the best effect on treating SLE.

### Example 15: Drug efficacy assay in CIA model

Collagen-induced arthritis (CIA) is an experimental spontaneous immune disease. CIA can be induced by immunizing susceptible rodent strains (rats and mice) with type II collagen. Immunized animals develop a form of spontaneous immune-mediated polyarthritis. The CIA model was established using DBA/1 mice. On day 0, all DBA/1 mice were anesthetized with 2%-5% isoflurane and then injected subcutaneously with 50 µL of collagen emulsion at the base of the tail 2-3 cm away from the body. Three weeks later, on the 21st day, the same volume of collagen emulsion was injected into the base of the tail in the same way. On the day of the second collagen emulsion challenge, the modeled animals were randomly divided into 6 groups according to body weight, with 5 or 10 mice in each group. Administration was started on the day of the grouping. The culture of EcN, CBT4088, CBT4098, CBT4110 and CBT4111 incubated aerobically in LB for 4 hours were concentrated and resuspended in 0.2 M sodium bicarbonate + 1% w/v glucose solution to obtain a bacterial suspension, respectively. Groups 1 to 5 each included 10 mice, which were given 100 µL of the bacterial suspensions of EcN, CBT4088, CBT4098, CBT4110 and CBT4111 respectively by gavage every day, at an amount of 1.5×10¹⁰ cells per mouse. Group 6 was a positive drug group, including 5 mice, which were administered with 100 µL of EcN and 0.2 mg/kg dexamethasone by gavage every day. The administration period lasted for 36 days. The arthritis incidence of the limbs of each group of animals was observed and scored twice a week, and the thickness of the front and rear foot pads was measured as well.

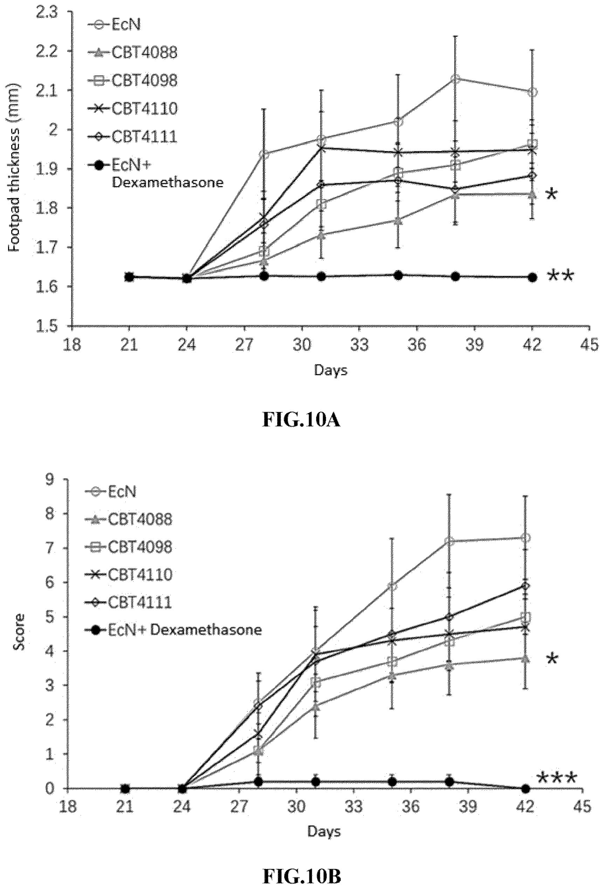

The thickness of the front and rear footpads of mice and the arthritis scores of the limbs are shown in FIG. 10. The engineered strain CBT4088 that simultaneously expressed IL-10 and Amuc_1100, the engineered strain CBT4098 that simultaneously expressed IL-22 and Amuc_1100, the engineered strain CBT4110 that simultaneously expressed IL-10 and IL-22, and the engineered strain CBT4111 that simultaneously expressed IL-10, IL-22 and Amuc_1100 can reduce the severity of the disease to a certain extent. Among them, CBT4088 that simultaneously expressed IL-10 and Amuc_1100 had the most significantly positive impact on footpad thickness (FIG. 10A) and disease score (FIG.. 10B) (p<0.05).

### Example 16: Drug efficacy assay in OVA-induced asthma model

Asthma is a chronic inflammatory disease of the airways, characterized by an increase in the number of type 2 T helper lymphocytes (T helper 2 cells, Th2) and eosinophils, and the occurrence of airway inflammation. It is associated with high levels of serum immunoglobulin E (IgE), as well as interleukin 4 (IL-4), interleukin 5 (IL-5) and interleukin 13 (IL-13) produced in the lungs by allergen-specific Th2 cells. Airway inflammation is associated with the infiltration of eosinophils, neutrophils, and T and B lymphocytes in the airways and lung tissue.

In this example, ovalbumin (OVA)-induced asthma model was established using BALB/c female mice. The mice were randomly divided into 7 groups according to body weight, and the administration period was 31 days. The culture of EcN, CBT4088, CBT4098, CBT4110 and CBT4111 incubated aerobically in LB for 4 hours were concentrated and resuspended in 0.2 M sodium bicarbonate + 1% w/v glucose solution to obtain a bacterial suspension, respectively. Groups 1 to 5 included 10 mice in each group, which were intragastrically administered with 100 µL of the bacterial suspensions of EcN, CBT4088, CBT4098, CBT4110 and CBT4111 respectively every day, at an amount of 1.5×10¹⁰ cells per mouse. Group 6 was the positive administration group, including 6 mice, which were intragastrically administered with 100 µL of EcN bacterial suspension every day, and additionally administered with the positive drug dexamethasone 1 mg/kg from days 27 to 31. Group 7 was a non-sensitized control group, including 5 mice, which were intragastrically administered with 100 µL of EcN bacterial suspension every day. The mice in groups 1-6 were intraperitoneally injected with 100 µL of sensitizing solution (containing 20 µg ovalbumin and 2 mg alum) on days 1 and 14. As a control, the mice in group 7 were injected with 100 µL of PBS solution. On days 28, 29, and 30, the mice in groups 1-6 were challenged with 1% OVA solution dissolved in PBS (pH=7.2) for 30 minutes. As a control, the mice in group 7 were challenged with PBS (pH=7.2).

On day 32, all animals were euthanized. The lungs were lavaged with PBS (pH=7.2) solution through endotracheal intubation. The total number of cells in bronchoalveolar lavage fluid (BALF) was counted by a hemocytometer. Eosinophils, macrophages, neutrophils, and lymphocytes were counted in the cytocentrifuge preparations after cytospins and Wright-Giemsa staining.

The cell count results in the bronchoalveolar lavage fluid (BALF) are shown in FIG.s 11A to 11E. Compared with the control group that was only administered with EcN in the group 1, the total number of cells, the number of eosinophils and the number of macrophages in the bronchoalveolar lavage fluid (BALF) of all administration groups decreased. Among them, CBT4098, which expressed both IL-22 and Amuc_1100, can significantly reduce the total number of cells (p<0.01), the number of eosinophils (p<0.05) and the number of macrophages (p<0.05). CBT4110 simultaneously expressing IL-10 and IL-22, and CBT4111 simultaneously expressing IL-10, IL-22 and Amuc_1100, can also significantly reduce the total number of cells and the number of macrophages.

Although the present disclosure has been specifically shown and described with reference to specific embodiments, some of which are preferred embodiments, it will be understood by those skilled in the art that various modifications may be made in form and detail without departing from the spirit and scope of the disclosure herein.

## Claims

1. A genetically modified microorganism comprising at least two exogenous genes respectively encoding a polypeptide selected from the group consisting of:
a) Amuc_1100 polypeptide,
b) IL-10 polypeptide, and
c) IL-22 polypeptide.

2. The genetically modified microorganism according to claim 1, comprising:
a) one exogenous gene encoding Amuc_1100 polypeptide and one exogenous gene encoding IL-10 polypeptide;
b) one exogenous gene encoding IL-10 polypeptide and one exogenous gene encoding IL-22 polypeptide; or
c) one exogenous gene encoding Amuc_1100 polypeptide and one exogenous gene encoding IL-22 polypeptide.

3. The genetically modified microorganism according to claim 1, comprising exogenous genes encoding Amuc_1100 polypeptide, IL-10 polypeptide and IL-22 polypeptide respectively.

4. A genetically modified microorganism comprising an exogenous gene encoding Amuc_1100 polypeptide, IL-10 polypeptide or IL-22 polypeptide.

5. The genetically modified microorganism according to any one of preceding claims, wherein the Amuc_1100 polypeptide is a wild-type Amuc_1100 polypeptide or a functional equivalent of the wild-type Amuc_1100 polypeptide.

6. The genetically modified microorganism according to any one of preceding claims, wherein the IL-10 polypeptide is a wild-type IL-10 polypeptide or a functional equivalent of the wild-type IL-10 polypeptide.

7. The genetically modified microorganism according to any one of preceding claims, wherein the IL-22 polypeptide is a wild-type IL-22 polypeptide or a functional equivalent of the wild-type IL-22 polypeptide.

8. The genetically modified microorganism according to claim 5, wherein the functional equivalent of the wild-type Amuc_1100 polypeptide is selected from the group consisting of a mutant, fragment, fusion, derivative and a combination thereof, and the functional equivalent at least partially retains one or more biological functions of the wild-type Amuc_1100, optionally, the biological function is selected from the group consisting of: a) regulating and/or promoting intestinal immune system function of mammals, b) maintaining, restoring and/or increasing physical integrity of the intestinal mucosal barrier of mammals, and c) activating TLR2.

9. The genetically modified microorganism according to claim 8, wherein the fragment of the wild-type Amuc_1100 polypeptide comprises:
(a) an amino acid sequence set forth in SEQ ID NO: 2, or an amino acid sequence having at least 80% sequence identity with the amino acid sequence set forth in SEQ ID NO: 2, or
(b) an amino acid sequence set forth in SEQ ID NO: 3, or an amino acid sequence having at least 80% sequence identity with the amino acid sequence set forth in SEQ ID NO: 3.

10. The genetically modified microorganism according to any one of the preceding claims, wherein the Amuc_1100 polypeptide comprises a sequence set forth in SEQ ID NO: 5, or an amino acid sequence having at least 80% sequence identity with the sequence set forth in SEQ ID NO: 5 and still maintaining activity of modulating intestinal immunity and/or activating toll-like receptor 2 (TLR2).

11. The genetically modified microorganism according to any one of the preceding claims, wherein the Amuc_1100 polypeptide comprises a mutation at position 259, which is Y, and the position 259 is numbered based on the numbering of the sequence set forth in SEQ ID NO: 5; preferably, the Amuc_1100 polypeptide comprises Y259A or Y259S mutation at position 259, and the position 259 is numbered based on the numbering of the sequence set forth in SEQ ID NO: 5.

12. The genetically modified microorganism according to any one of the preceding claims, wherein the IL-10 polypeptide comprises a sequence set forth in SEQ ID NO: 11, or an amino acid sequence having at least 80% sequence identity with the sequence set forth in SEQ ID NO: 11 and still maintaining activity of modulating immune cells.

13. The genetically modified microorganism according to any one of the preceding claims, wherein the IL-22 polypeptide comprises a sequence set forth in SEQ ID NO: 15, or an amino acid sequence having at least 80% sequence identity with the sequence set forth in SEQ ID NO: 15 and still maintaining activity of binding to IL-22 receptor and modulating IL-22 receptor-expressing cells.

14. The genetically modified microorganism according to any one of the preceding claims, wherein the microorganism is capable of expressing and/or secreting a polypeptide encoded by the exogenous gene; or
the microorganism is capable of expressing and/or secreting a polypeptide encoded by the exogenous gene in the intestine of a human or animal.

15. The genetically modified microorganism according to any one of preceding claims, wherein the exogenous gene is in an exogenous expression cassette.

16. The genetically modified microorganism according to claim 15, wherein the exogenous expression cassette is in a plasmid, and wherein the plasmid is introduced into the microorganism and is suitable for expression in the microorganism.

17. The genetically modified microorganism according to claim 15, wherein the exogenous expression cassette is integrated into the genome of the genetically modified microorganism.

18. The genetically modified microorganism according to claim 15, wherein the microorganism comprises:
a first exogenous expression cassette comprising a nucleotide sequence encoding Amuc_1100 polypeptide and a second exogenous expression cassette comprising a nucleotide sequence encoding IL-10 polypeptide;
a second exogenous expression cassette comprising a nucleotide sequence encoding IL-10 polypeptide and a third exogenous expression cassette comprising a nucleotide sequence encoding IL-22 polypeptide;
a first exogenous expression cassette comprising a nucleotide sequence encoding Amuc_1100 polypeptide and a third exogenous expression cassette comprising a nucleotide sequence encoding IL-22 polypeptide;
a first exogenous expression cassette comprising a nucleotide sequence encoding Amuc_1100 polypeptide, a second exogenous expression cassette comprising a nucleotide sequence encoding IL-10 polypeptide and a third exogenous expression cassette comprising a nucleotide sequence encoding IL-22 polypeptide;
a first exogenous expression cassette comprising a nucleotide sequence encoding Amuc_1100 polypeptide;
a second exogenous expression cassette comprising a nucleotide sequence encoding IL-10 polypeptide; or
a third exogenous expression cassette comprising a nucleotide sequence encoding IL-22 polypeptide.

19. The genetically modified microorganism according to any one of claims 15-18, wherein the exogenous expression cassette is present in one or more copies (e.g., two, three, four, five, six, etc.) in the genome.

20. The genetically modified microorganism according to any one of the preceding claims, wherein the Amuc_1100 polypeptide, the IL-10 polypeptide and/or the IL-22 polypeptide is a polypeptide without its own signal peptide.

21. The genetically modified microorganism according to claim 20, wherein a first signal peptide is connected to the N-terminus of the Amuc_1100 polypeptide, a second signal peptide is connected to the N-terminus of the IL-10 polypeptide, and/or a third signal peptide is connected to the N-terminus of the IL-22 polypeptide, preferably, the first signal peptide, the second signal peptide and/or the third signal peptide is capable of secreting the Amuc_1100 polypeptide, the IL-10 polypeptide and/or the IL-22 polypeptide outside the microorganism.

22. The genetically modified microorganism according to claim 20, wherein
the first signal peptide, the second signal peptide, and the third signal peptide are the same or different; and
the first signal peptide, the second signal peptide and the third signal peptide respectively comprise an amino acid sequence selected from the group consisting of SEQ ID NOs: 80-122, and a homologous sequence having at least 80% sequence identity with the amino acid sequence;
preferably, the first signal peptide, the second signal peptide, and/or the third signal peptide is a USP45 signal peptide, and the USP45 signal peptide comprises an amino acid sequence set forth in SEQ ID NO: 114, or a homologous sequence having at least 80% sequence identity with SEQ ID NO: 114.

23. The genetically modified microorganism according to claim 21, wherein the first signal peptide, second signal peptide, and/or third signal peptide are processed by a secretion system present in the microorganism;
particularly, the secretion system is a system native or non-native to the microorganism.

24. The genetically modified microorganism according to any one of claims 15 to 23, wherein the exogenous gene is operably linked to an expression control element; preferably, the expression control element comprises a promoter, more preferably, the promoter is selected from the group consisting of BBa_J23101, BBa_J23108, BBa_J23110, PfnrS, Psal, Pvan, BBa_J23119, BBa_J23102 and BBa_J23114.

25. The genetically modified microorganism according to claim 24, wherein the expression control element comprises a ribosome binding site (RBS), preferably, the ribosome binding site comprises a nucleotide sequence set forth in SEQ ID NO: 63.

26. The genetically modified microorganism according to claim 24 or 25, wherein the expression control element comprises a cistron, preferably the cistron is selected from the group consisting of T7g10, BCD2, GFP and luciferase.

27. The genetically modified microorganism according to any one of claims 24 to 26, wherein the expression control element comprises a terminator, preferably the terminator is rmB_T1_T7Te terminator, more preferably, the rmB_T1_T7Te terminator comprises a sequence set forth in SEQ ID NO: 242.

28. The genetically modified microorganism according to any one of preceding claims, wherein the genetically modified microorganism further comprises one or more genetic modifications to the genome of the microorganism.

29. The genetically modified microorganism according to claim 28, wherein the one or more genetic modifications comprise engineering modification and/or optimization of the secretion system and at least one outer membrane protein encoding gene is deleted, deactivated or inhibited.

30. The genetically modified microorganism according to claim 29, wherein the outer membrane protein is selected from the group consisting of lpp, mrcA, ompT, tolQ, tolR, tolA and pal.

31. The genetically modified microorganism according to claim 29, wherein the at least one outer membrane protein is selected from the group consisting of lpp, mrcA, ompT and a combination thereof.

32. The genetically modified microorganism according to claim 29, wherein the at least one outer membrane protein is lpp and mrcA.

33. The genetically modified microorganism according to any one of claims 29 to 32, wherein the genetically modified microorganism further comprises one or more overexpressed molecular chaperones.

34. The genetically modified microorganism according to claim 33, wherein the molecular chaperone is selected from the group consisting of: dsbA, dsbC, dnaK, dnaJ, grpE, groES, groEL, tig, fkpA, surA, and a combination of two or more (e.g., three, four, five, six, seven, eight, nine, or ten) of the molecular chaperones.

35. The genetically modified microorganism according to claim 28, wherein the one or more genetic modifications include inactivation or deletion of at least one auxotrophy-associated gene.

36. The genetically modified microorganism according to any one of preceding claims, wherein the microorganism is an auxotroph of one or more substances selected from the group consisting of: uracil, thymine, diaminoheptanyl diacid, leucine, histidine, tryptophan, lysine, methionine, adenine and non-wild-type amino acids.

37. The genetically modified microorganism according to claim 36, wherein the non-wild-type amino acid is selected from the group consisting of: 1-4,4'-biphenylalanine, p-acetyl-1-phenylalanine, p-iodo-l-phenylalanine and p-azido-l-phenylalanine.

38. The genetically modified microorganism according to any one of preceding claims, wherein the microorganism is selected from the group consisting of bacteria, archaea, fungi and algae.

39. The genetically modified microorganism according to claim 38, wherein the fungi are selected from the group consisting of yeast and filamentous fungi.

40. The genetically modified microorganism according to any one of preceding claims, wherein the microorganism is selected from the group consisting of probiotic microorganism and non-pathogenic microorganism.

41. The genetically modified microorganism according to claim 40, wherein the probiotic microorganism is a probiotic bacterium or probiotic yeast.

42. The genetically modified microorganism according to claim 41, wherein the probiotic bacterium is selected from the group consisting of: *Bacteroides, Bifidobacterium, Clostridium, Escherichia, Lactobacillus* and *Lactococcus.*

43. The genetically modified microorganism according to claim 42, wherein the probiotic bacterium is a bacterium of genus *Escherichia.*

44. The genetically modified microorganism according to claim 42, wherein the probiotic bacterium is *Escherichia coli* strain Nissle 1917 (EcN).

45. The genetically modified microorganism according to claim 41, wherein the probiotic bacterium is a bacterium normally found in the human intestinal tract.

46. The genetically modified microorganism according to claim 41, wherein the probiotic yeast is selected from the group consisting of: *Saccharomyces cerevisiae, Candida utilis, Kluyveromyces lactis* and *Saccharomyces carlsbergensis.*

47. A combination of genetically modified microorganisms, comprising at least two (e.g., two, three, four, etc.) different genetically modified microorganisms according to any one of claims 1-46.

48. A polynucleotide comprising at least one recombinant expression cassette, comprising
i) at least one or at least two exogenous genes encoding Amuc_1100 polypeptide, IL-10 polypeptide and/or IL-22 polypeptide respectively, and
ii) one or more regulatory elements operably linked to the at least one or at least two exogenous genes.

49. The polynucleotide according to claim 48, comprising exogenous genes encoding the following polypeptides respectively: a) Amuc_1100 polypeptide and IL-10 polypeptide; b) IL-10 polypeptide and IL-22 polypeptide, or c ) Amuc_1100 polypeptide and IL-22 polypeptide.

50. The polynucleotide according to claim 48, comprising exogenous genes encoding Amuc_1100 polypeptide, IL-10 polypeptide and IL-22 polypeptide respectively.

51. The polynucleotide according to claim 48, wherein the polynucleotide comprises: a first recombinant expression cassette comprising a nucleotide sequence encoding Amuc_1100 polypeptide, and one or more regulatory elements operably linked thereto; a second recombinant expression cassette comprising a nucleotide sequence encoding IL-10 polypeptide, and one or more regulatory elements operably linked thereto; and/or a third recombinant expression cassette comprising a nucleotide sequence encoding IL-22 polypeptide, and one or more regulatory elements operably linked thereto.

52. The polynucleotide according to claim 51, wherein the nucleotide sequence encodes the Amuc_1100 polypeptide encodes an Amuc_1100 polypeptide in which its own signal peptide is replaced with a first signal peptide, and the nucleotide sequence encoding IL-10 polypeptide encodes an IL-10 polypeptide in which its own signal peptide is replaced with a second signal peptide, and/or the nucleotide sequence encoding IL-22 polypeptide encodes an IL-22 polypeptide in which its own signal peptide is replaced with a third signal peptide.

53. The polynucleotide according to claim 52, wherein the first signal peptide, second signal peptide and third signal peptide are the same or different, optionally, the first signal peptide, the second signal peptide, and/or the third signal peptide is USP45 signal peptide, and the USP45 signal peptide comprises an amino acid sequence set forth in SEQ ID NO: 114, or a homologous sequence having at least 80% sequence identity with SEQ ID NO: 114.

54. The polynucleotide according to claim 48, wherein the one or more regulatory elements are selected from the group consisting of a promoter, a ribosome binding site (RBS), a cistron, a terminator, and a combination thereof.

55. The polynucleotide according to claim 54, wherein,
the promoter is selected from the group consisting of BBa_J23101, BBa_J23108, BBa_J23110, PfimS, Psal, Pvan, BBa_J23119, BBa_J23102 and BBa_J23114;
the ribosome binding site is a synthetic fragment comprising a nucleotide sequence set forth in SEQ ID NO: 63;
the cistron is selected from the group consisting of: T7g10, BCD2, GFP and luciferase; and/or
the terminator is rrnB_T1_T7Te terminator, preferably, the terminator comprises a sequence set forth in SEQ ID NO: 242.

56. The polynucleotide according to claim 48, wherein the recombinant expression cassette is suitable for expression in probiotic bacteria or probiotic yeast.

57. The polynucleotide according to claim 48, comprising a nucleotide sequence set forth in any one of SEQ ID NOs: 139-163 and 235, or a combination thereof.

58. A composition, comprising:
(a) the genetically modified microorganism according to any one of claims 1 to 46 or the combination of genetically modified microorganisms according to claim 47 as an active ingredient; and
(b) a physiologically or pharmacologically acceptable carrier.

59. The composition according to claim 58, wherein the composition is a pharmaceutical composition.

60. The composition according to claim 59, wherein the pharmaceutical composition is an oral preparation.

61. The composition according to claim 59, wherein the pharmaceutical composition is a liquid preparation, a solid preparation or a semi-solid preparation.

62. The composition according to claim 59, wherein the pharmaceutical composition is in a dosage form selected from the group consisting of: dustpowder, powder, tablet, sugar-coated form, capsule, granule, suspension, solution, syrup, drop and sublingual tablet.

63. The composition according to claim 60, wherein the liquid preparation is selected from the group consisting of solution preparation and suspension preparation.

64. The composition according to claim 59, wherein the pharmaceutical composition comprises 1×10⁸ to 1×10¹² CFU of the genetically modified microorganism according to any one of claims 1-46 or the combination of genetically modified microorganisms according to claim 47.

65. The composition according to claim 58, wherein the composition is an edible composition.

66. The composition according to claim 58, wherein the composition is a probiotic composition.

67. The composition according to claim 58, wherein the composition is a food supplement.

68. Use of the genetically modified microorganism according to any one of claims 1 to 46, the combination of genetically modified microorganisms according to claim 47, or the composition according to claim 58 in the manufacture of a medicament for treating or preventing an inflammatory disease or autoimmune disease.

69. The use according to claim 68, wherein the autoimmune disease is selected from the group consisting of inflammatory bowel disease, graft versus host disease (GvHD), systemic lupus erythematosus (SLE), arthritis, asthma, and a combination thereof.

70. The use according to claim 69, wherein the autoimmune disease is selected from inflammatory bowel disease including Crohn's disease or ulcerative colitis, and/or the arthritis is selected from the group consisting of rheumatoid arthritis, osteoarthritis, psoriatic arthritis and juvenile idiopathic arthritis, and/or the asthma is selected from the group consisting of allergic asthma and neutrophilic asthma.

71. Use of the genetically modified microorganism according to any one of claims 1-46, or the combination of genetically modified microorganisms according to claim 47, or the composition according to any one of claims 58-69 in the manufacture of a medicament for improving the therapeutic effect of a drug for treating an inflammatory disease or autoimmune disease.

72. A method for producing the genetically modified microorganism according to any one of claims 1-46, comprising the steps of:
introducing the polynucleotide according to any one of claims 48-57 into a microorganism so that the exogenous gene in the polynucleotide is expressed in the microorganism.

73. A method for treating or preventing an inflammatory disease or an autoimmune disease in a subject in need thereof, comprising:
administering to the subject an effective amount of the genetically modified microorganism according to any one of claims 1-46, or the combination of genetically modified microorganisms according to claim 47, or the composition according to claims 58-69.

74. The method according to claim 73, wherein the autoimmune disease is selected from the group consisting of inflammatory bowel disease, graft versus host disease (GvHD), systemic lupus erythematosus, arthritis, asthma, and a combination thereof.

75. The method according to claim 73, wherein the autoimmune disease is selected from inflammatory bowel disease including Crohn's disease or ulcerative colitis, and/or the arthritis is selected from the group consisting of rheumatoid arthritis, osteoarthritis, psoriatic arthritis and juvenile idiopathic arthritis, and/or the asthma is selected from the group consisting of allergic asthma and neutrophilic asthma.

76. A method for improving the efficacy of a drug in a subject, comprising:
administering to the subject an effective amount of the genetically modified microorganism according to any one of claims 1-46, or the combination of genetically modified microorganisms according to claim 47, or the composition according to claims 58-67;
wherein the drug is a drug for treating an inflammatory disease or an autoimmune disease.

77. Use of a genetically modified microorganism in the manufacture of a medicament for treating an inflammatory disease or an autoimmune disease, wherein the inflammatory disease or autoimmune disease is selected from the group consisting of: inflammatory bowel disease (IBD), graft-versus-host disease (GvHD), systemic lupus erythematosus (SLE), arthritis and asthma;
wherein the genetically modified microorganism comprises at least one, at least two or at least three exogenous genes encoding polypeptides selected from the group consisting of:
a) Amuc_1100 polypeptide;
b) IL-10 polypeptide; and
c) IL-22 polypeptide.

78. The use according to claim 77, wherein:
(1) the genetically modified microorganism comprises exogenous genes encoding Amuc_1100 polypeptide and IL-10 polypeptide respectively;
(2) the genetically modified microorganism comprises exogenous genes encoding IL-10 polypeptide and IL-22 polypeptide respectively;
(3) the genetically modified microorganism comprises exogenous genes encoding Amuc_1100 polypeptide and IL-22 polypeptide respectively;
(4) the genetically modified microorganism comprises an exogenous gene encoding IL-10 polypeptide;
(5) the genetically modified microorganism comprises an exogenous gene encoding Amuc_1100 polypeptide;
(6) the genetically modified microorganism comprises an exogenous gene encoding IL-22 polypeptide; or
(7) the genetically modified microorganism comprises exogenous genes encoding Amuc_1100 polypeptide, IL-10 polypeptide and IL-22 polypeptide respectively.
